(19) Europäisches Patentamt / European Patent Office / Office européen des brevets

(11) **EP 3 508 493 B1**

(12) **EUROPEAN PATENT SPECIFICATION**

(45) Date of publication and mention of the grant of the patent:
**02.10.2024 Bulletin 2024/40**

(21) Application number: **17846649.6**

(22) Date of filing: **31.08.2017**

(51) International Patent Classification (IPC):
**C07K 1/22** *(2006.01)* **C07K 16/00** *(2006.01)*
**C07K 17/08** *(2006.01)* **B01D 15/26** *(2006.01)*

(52) Cooperative Patent Classification (CPC):
**B01D 15/265; C07K 1/22; C07K 16/00; C07K 17/08**

(86) International application number:
**PCT/JP2017/031366**

(87) International publication number:
**WO 2018/043645 (08.03.2018 Gazette 2018/10)**

(54) **PROTEIN PURIFICATION METHOD USING ACTIVATED CARBON**

PROTEINAUFREINIGUNGSVERFAHREN MIT AKTIVKOHLE

PROCÉDÉ DE PURIFICATION DE PROTÉINES UTILISANT DU CHARBON ACTIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.08.2016 JP 2016170265**

(43) Date of publication of application:
**10.07.2019 Bulletin 2019/28**

(73) Proprietor: **Kyowa Kirin Co., Ltd.**
**Tokyo (JP)**

(72) Inventors:
• **ISHIHARA, Takashi**
  **Tokyo (JP)**
• **YAMADA, Tsuyoshi**
  **Tokyo (JP)**
• **KIKUCHI, Shinsuke**
  **Tokyo (JP)**

(74) Representative: **Hoffmann Eitle**
**Patent- und Rechtsanwälte PartmbB**
**Arabellastraße 30**
**81925 München (DE)**

(56) References cited:
EP-A2- 2 639 239     WO-A1-2014/024514
WO-A1-2015/005960    WO-A1-2015/005961
JP-A- 2013 189 427   JP-A- 2017 132 757

• **MATTHEW T. STONE ET AL: "Separating Proteins with Activated Carbon", LANGMUIR, vol. 30, no. 27, 15 July 2014 (2014-07-15), US, pages 8046 - 8055, XP055244941, ISSN: 0743-7463, DOI: 10.1021/la501005s**
• **JOHNSON M. W. ET AL.: "Comparison of Concentration Measurement Technologies in Bioprocess Solutions", BIOPROCESS INTERNATIONAL, October 2015 (2015-10-01), pages 1 - 5, XP055472285**

**Description**

Technical Field

The present invention relates to a method for purifying a protein as claimed using an activated carbon, and a method for preparing proteins in large-scale efficiently at low cost, which comprises the purification method.

Background Art

Development of genetic recombination technologies has provided drugs including a variety of proteins as an active ingredient. In particular, numerous drugs including antibodies as an active ingredient have been recently developed and commercialized. In addition, efficient and low-cost production of these proteins in large-scale has become a more important issue in biopharmaceutical industry.

Generally, such proteins are produced by culturing recombinant cells in which a vector including a gene encoding a protein of interest is inserted. The culture broth includes impurities such as a wide variety of medium-derived components, host cell-derived components, protein-derived by-products or the like, in addition to the protein of interest. Thus, it is a very difficult and challenging task to achieve both the purification of the protein of interest (hereinafter, also abbreviated as target protein) by removing impurities to meet purity requirements for protein drugs as well as the efficient and low-cost production of the protein of interest in large-scale by increasing a recovery rate.

In general, the protein purification method is carried out by a combination of different modes of chromatography. Chromatography is to separate the protein of interest from impurities, for example, based on charge, hydrophilicity, molecular size or the like.

In particular, when the protein of interest is an antibody, Protein A affinity chromatography or Protein G affinity chromatography is used as one of chromatography for purifying the antibody, by using binding property of Protein A or Protein G to the specific region of antibody such as Fc chain (Patent Document 1).

However, Protein A affinity resins generally used are very expensive in comparison to ion exchange resins or hydrophobic resins, and a vast amount of resins are needed for large-scale purification of antibodies in industrial drug productions or the like, resulting in an inevitable increase in the production costs.

In order to solve the problems described above, a purification method using an activated carbon which is used as an adsorbent or as a decolorant in the industrial fields, such as the production of chemicals and foods, sewage or waste water treatment, water filtration, and production of small-molecule drugs, as an inexpensive natural material having extensive non-specific adsorption properties and formed of natural sources, instead of protein A affinity chromatography has been known (Patent Document 2). EP 2 639 239 A2 describes the removal of protein aggregates from biopharmaceutical preparations in a flow-through mode

Related Art

Patent Documents

[Patent Document 1] Japanese Patent Publication No. Hei5-504579
[Patent Document 2] International Patent Publication No. 2014/024514

Disclosure of Invention

Problems to Be Solved by the Invention

In the purification of a protein using an activated carbon, a purification process after an activated carbon treatment process is generally carried out in an adsorption mode of specifically adsorbing the antibody of interest onto resins such as an ion exchange resin or a hydrophobic resin, washing the adsorbing resin to separate impurities, and finally eluting the antibody of interest from the resin.

In this regard, buffers used in the washing and eluting steps are different from each other, scale-up of chromatography apparatus brings out enlargement or complexity of the accompanying production facilities such as buffer tank, and moreover, manipulations become complicated. As a result, all of these factors are the cause of increasing production costs in all of the purification processes. Therefore, the removal of impurities in the activated carbon treatment process as much as possible to reduce the number of subsequent purification processes are important for the reduction of production costs.

Due to such a reason described above, it is required to provide a method for purifying a protein capable of efficiently separating impurities, reducing the number of purification process after the activated carbon treatment process by increasing a recovery rate of the protein of interest, and performing efficient, large-scale, and low-cost production of drugs, rather than the method for purifying a protein using an activated carbon of the related art.

Thus, an object of the present invention is to provide a method for purifying a protein as claimed capable of significantly reducing impurities and achieving a high recovery rate, compared to the method for purifying a protein using an activated carbon of the related art.

Means for Solving the Problems

The present inventors have made many efforts to solve the above objects. As a result, they surprisingly found that, a high recovery rate can be acquired regarding the protein of interest with a significantly decreased content of various impurities, by performing the activated carbon treatment to an activated carbon pretreatment solution having an adjusted conductivity of a protein-containing aqueous solution, by performing dilution, concentration dilution, buffer exchange, or addition of sodium chloride in advance, in a case of separating the protein from impurities using an inexpensive activated carbon in a non-adsorption mode, thereby completing the present invention.

The present invention is as set out by the claims

Effects of the Invention

In the method for purifying a protein of the present invention, the protein of interest can be acquired at a high recovery rate by efficiently separating impurities and reducing the amount of the protein of interest adsorbed to the activated carbon, compared to the method for purifying a protein using an activated carbon of the related art, by performing the activated carbon treatment to the activated carbon pretreatment solution obtained by adjusting the conductivity of the protein-containing aqueous solution, in a case of separating the protein and the impurities in a non-adsorption mode using the activated carbon.

The present invention provides a method for purifying a protein as claimed which can lower production cost or reduce labor than the conventional protein purification methods, and has impurity separation properties higher than or equivalent to the conventional protein purification methods, in particular, in the antibody purification, and a method for preparing a protein comprising the purification method. The protein prepared by the preparation method of the present invention is useful as a drug.

Brief Description of the Drawings

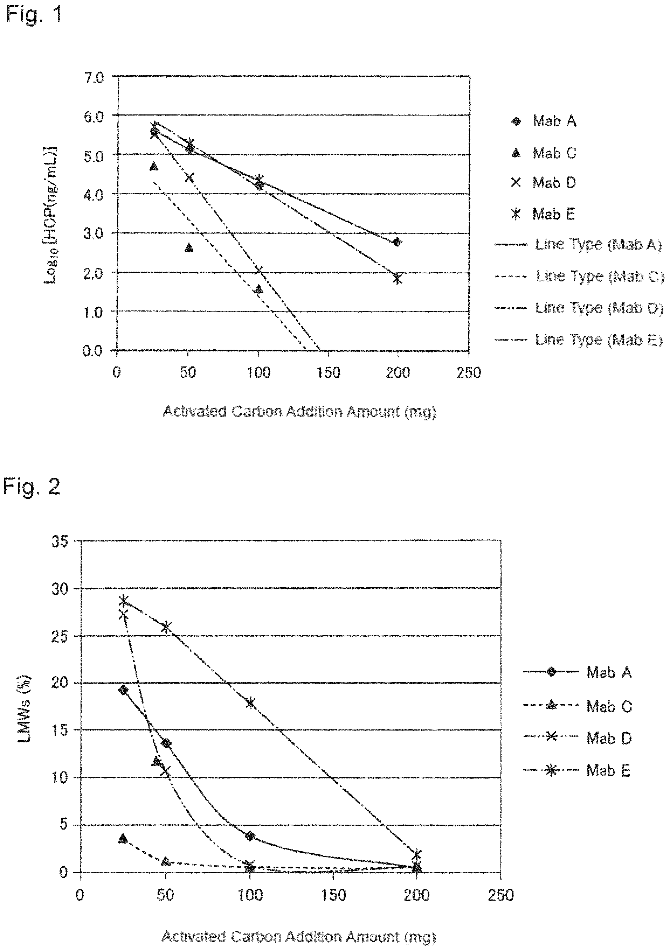

FIG. 1 shows a relationship between an activated carbon addition amount and a concentration of a host cell protein (HCP) after an activated carbon treatment. A horizontal axis indicates an activated carbon addition amount (mg) and a vertical axis indicates a logarithmic value of the HCP concentration (ng/mL). A black diamond shows a result obtained by treating a pH-adjusted clarified solution including Mab A, a black triangle shows a result obtained by treating a pH-adjusted clarified solution including Mab C, x shows a result obtained by treating a pH-adjusted clarified solution including Mab D, and * shows a result obtained by treating a pH-adjusted clarified solution including Mab E.
FIG. 2 shows a relationship between the activated carbon addition amount and contents of protein-derived degradation products (LMWs) after the activated treatment. A horizontal axis indicates an activated carbon addition amount (mg) and a vertical axis indicates a LMWs content (%). A black diamond shows a result obtained by treating a pH-adjusted clarified solution including Mab A, a black triangle shows a result obtained by treating a pH-adjusted clarified solution including Mab C, x shows a result obtained by treating a pH-adjusted clarified solution including Mab D, and * shows a result obtained by treating a pH-adjusted clarified solution including Mab E.

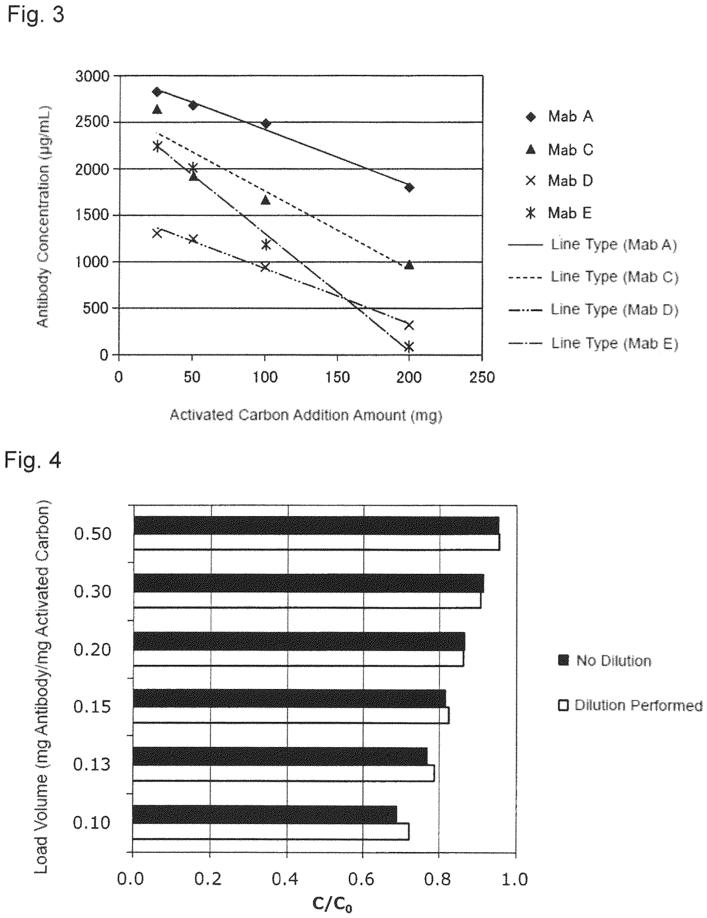

FIG. 3 shows a relationship between the activated carbon addition amount and a concentration of an antibody after the activated carbon treatment. A horizontal axis indicates an activated carbon addition amount (mg) and a vertical axis indicates the antibody concentration ($\mu$g/mL). A black diamond shows a result obtained by treating a pH-adjusted clarified solution including Mab A, a black triangle shows a result obtained by treating a pH-adjusted clarified solution including Mab C, x shows a result obtained by treating a pH-adjusted clarified solution including Mab D, and * shows a result obtained by treating a pH-adjusted clarified solution including Mab E.
FIG. 4 shows an effect of the dilution of an activated carbon pretreatment solution applied to a recovery ratio ($C/C_0$) in each load volume. A black bar shows a result obtained without dilution of the activated carbon pretreatment solution and a white bar shows a result obtained by the dilution.
FIG. 5 shows an effect of the dilution of the activated carbon pretreatment solution applied to a concentration of a host cell protein (HCP) in each load volume. A black bar shows a result obtained without dilution of the activated carbon pretreatment solution and a white bar shows a result obtained by the dilution.

FIG. 6 shows an effect of the dilution of the activated carbon pretreatment solution applied to a content of protein-derived polymers (HMWs) in each load volume. A black bar shows a result obtained without dilution of the activated carbon pretreatment solution and a white bar shows a result obtained by the dilution.

FIG. 7 shows an effect of the dilution of the activated carbon pretreatment solution applied to a content of a protein-derived degradation products (LMWs) in each load volume. A black bar shows a result obtained without dilution of the activated carbon pretreatment solution and a white bar shows a result obtained by the dilution.

FIG. 8 shows an effect of the concentration dilution of the activated carbon pretreatment solution applied to a recovery ratio ($C/C_0$).

FIG. 9 shows an effect of the concentration dilution of the activated carbon pretreatment solution applied to the concentration of the host cell protein (HCP). The HCP concentration in a case of performing the concentration dilution was corrected by considering a concentration dilution ratio.

FIG. 10 shows an effect of the concentration dilution of the activated carbon pretreatment solution applied to the content of the protein-derived polymers (HMWs).

FIG. 11 shows an effect of the concentration dilution of the activated carbon pretreatment solution applied to the content of the protein-derived degradation products (LMWs).

FIG. 12 shows an effect of the conductivity of the activated carbon pretreatment solution applied to the recovery ratio ($C/C_0$). A vertical axis indicates $C/C_0$ and a horizontal axis indicates the conductivity (mS/cm).

FIG. 13 shows an effect of the conductivity of the activated carbon pretreatment solution applied to the content of the protein-derived polymers (HMWs). A vertical axis indicates the HMWs content (%) and a horizontal axis indicates the conductivity (mS/cm).

FIG. 14 shows an effect of the conductivity of the activated carbon pretreatment solution applied to the content of the protein-derived degradation products (LMWs). A vertical axis indicates the LMWs content (%) and a horizontal axis indicates the conductivity (mS/cm).

FIG. 15 shows an effect of the conductivity of the activated carbon pretreatment solution applied to the concentration of the host cell protein (HCP). A vertical axis indicates the HCP concentration (ng/mg-P) and a horizontal axis indicates the conductivity (mS/cm).

FIG. 16 shows an effect of a concentration of sodium chloride (NaCl) added to the activated carbon pretreatment solution applied to the recovery ratio ($C/C_0$). A vertical axis indicates $C/C_0$ and a horizontal axis indicates the NaCl concentration (mmol/mL).

FIG. 17 shows an effect of the concentration of sodium chloride (NaCl) added to the activated carbon pretreatment solution applied to the content of the protein-derived polymers (HMWs). A vertical axis indicates HMWs content (%) and a horizontal axis indicates the NaCl concentration (mmol/mL).

FIG. 18 shows an effect of the concentration of sodium chloride (NaCl) added to the activated carbon pretreatment solution applied to the content of the protein-derived degradation products (LMWs). A vertical axis indicates LMWs content (%) and a horizontal axis indicates the NaCl concentration (mmol/mL).

FIG. 19 shows an effect of the concentration of sodium chloride (NaCl) added to the activated carbon pretreatment solution applied to the concentration of the host cell protein (HCP). A vertical axis indicates the HCP concentration (ng/mg-P) and a horizontal axis indicates the NaCl concentration (mmol/mL).

FIG. 20 shows an effect of the antibody concentration of the activated carbon pretreatment solution applied to the recovery ratio ($C/C_0$). A vertical axis indicates $C/C_0$ and a horizontal axis indicates the treatment antibody concentration (mg/mL).

FIG. 21 shows an effect of the antibody concentration of the activated carbon pretreatment solution applied to the content of the protein-derived polymers (HMWs). A vertical axis indicates the HMWs content (%) and a horizontal axis indicates the treatment antibody concentration (mg/mL).

FIG. 22 shows an effect of the antibody concentration of the activated carbon pretreatment solution applied to the content of the protein-derived degradation products (LMWs). A vertical axis indicates the LMWs content (%) and a horizontal axis indicates the treatment antibody concentration (mg/mL).

FIG 23 shows an effect of the antibody concentration of the activated carbon pretreatment solution applied to the concentration of the host cell protein (HCP). A vertical axis indicates the HCP concentration (ng/mg-P) and a horizontal axis indicates the treatment antibody concentration (mg/mL).

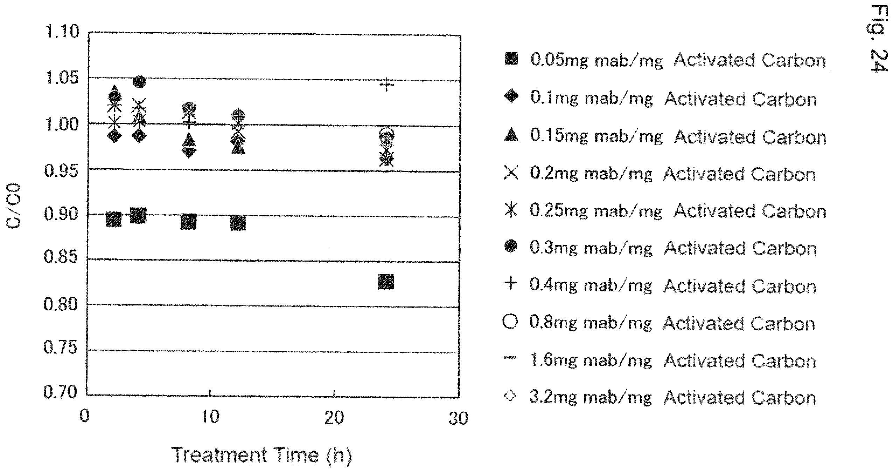

FIG. 24 shows an effect of the activated carbon treatment time applied to the recovery ratio ($C/C_0$). A vertical axis indicates $C/C_0$ and a horizontal axis indicates the treatment time (h). A black square shows a result of the load volume of 0.05 mg mab/mg activated carbon, a black diamond shows a result of the load volume of 0.1 mg mab/mg activated carbon, a black triangle shows a result of the load volume of 0.15 mg mab/mg activated carbon, x shows a result of the load volume of 0.2 mg mab/mg activated carbon, * shows a result of the load volume of 0.25 mg mab/mg activated carbon, a black circle shows a result of the load volume of 0.3 mg mab/mg activated carbon, +

shows a result of the load volume of 0.4 mg mab/mg activated carbon, a white circle shows a result of the load volume of 0.8 mg mab/mg activated carbon, - shows a result of the load volume of 1.6 mg mab/mg activated carbon, and a white diamond shows a result of the load volume of 3.2 mg mab/mg activated carbon.

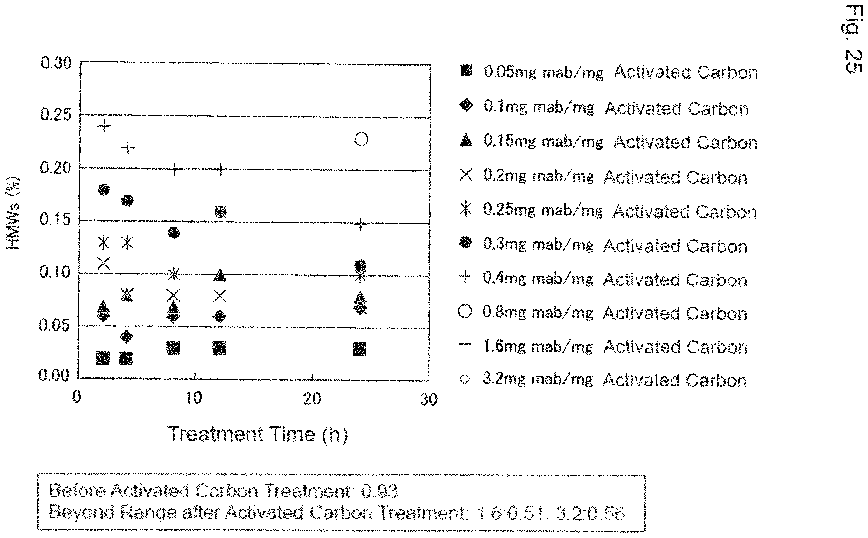

FIG. 25 shows an effect of the activated carbon treatment time applied to the content of the protein-derived polymers (HMWs). A vertical axis indicates HMWs content (%) and a horizontal axis indicates the treatment time (h). A black square shows a result of the load volume of 0.05 mg mab/mg activated carbon, a black diamond shows a result of the load volume of 0.1 mg mab/mg activated carbon, a black triangle shows a result of the load volume of 0.15 mg mab/mg activated carbon, x shows a result of the load volume of 0.2 mg mab/mg activated carbon, * shows a result of the load volume of 0.25 mg mab/mg activated carbon, a black circle shows a result of the load volume of 0.3 mg mab/mg activated carbon, + shows a result of the load volume of 0.4 mg mab/mg activated carbon, a white circle shows a result of the load volume of 0.8 mg mab/mg activated carbon, - shows a result of the load volume of 1.6 mg mab/mg activated carbon, and a white diamond shows a result of the load volume of 3.2 mg mab/mg activated carbon. The HMWs content before the activated carbon treatment is 0.93%. In addition, the HMWs content of the treatment for 24 hours with the load volume of 1.6 mg mab/mg activated carbon is 0.51%, and the HMWs content of the treatment for 24 hours with the load volume of 3.2 mg mab/mg activated carbon is 0.56%.

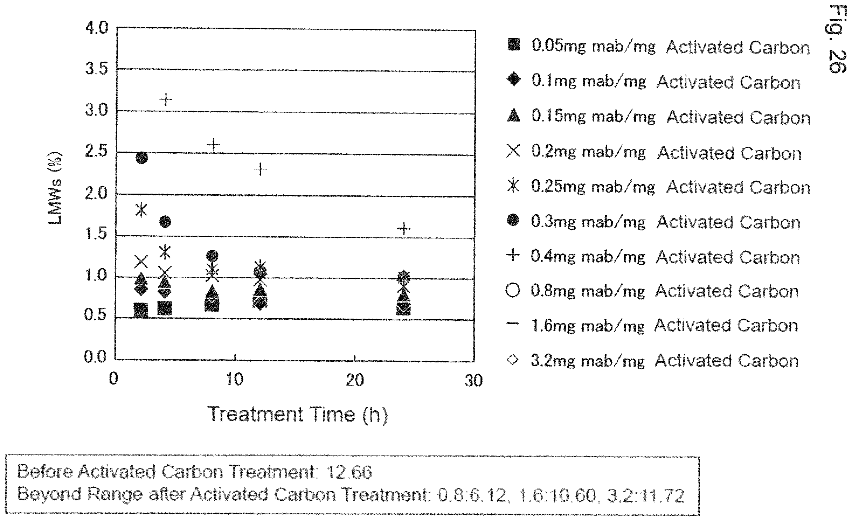

FIG. 26 shows an effect of the activated carbon treatment time applied to the content of the protein-derived degradation products (LMWs). A vertical axis indicates LMWs content (%) and a horizontal axis indicates the treatment time (h). A black square shows a result of the load volume of 0.05 mg mab/mg activated carbon, a black diamond shows a result of the load volume of 0.1 mg mab/mg activated carbon, a black triangle shows a result of the load volume of 0.15 mg mab/mg activated carbon, x shows a result of the load volume of 0.2 mg mab/mg activated carbon, * shows a result of the load volume of 0.25 mg mab/mg activated carbon, a black circle shows a result of the load volume of 0.3 mg mab/mg activated carbon, + shows a result of the load volume of 0.4 mg mab/mg activated carbon, a white circle shows a result of the load volume of 0.8 mg mab/mg activated carbon, - shows a result of the load volume of 1.6 mg mab/mg activated carbon, and a white diamond shows a result of the load volume of 3.2 mg mab/mg activated carbon. The LMWs content before the activated carbon treatment is 12.66%. In addition, the LMWs content of the treatment for 24 hours with the load volume of 0.8 mg mab/mg activated carbon is 6.12%, the LMWs content of the treatment for 24 hours with the load volume of 1.6 mg mab/mg activated carbon is 10.60%, and the LMWs content of the treatment for 24 hours with the load volume of 3.2 mg mab/mg activated carbon is 11.72%.

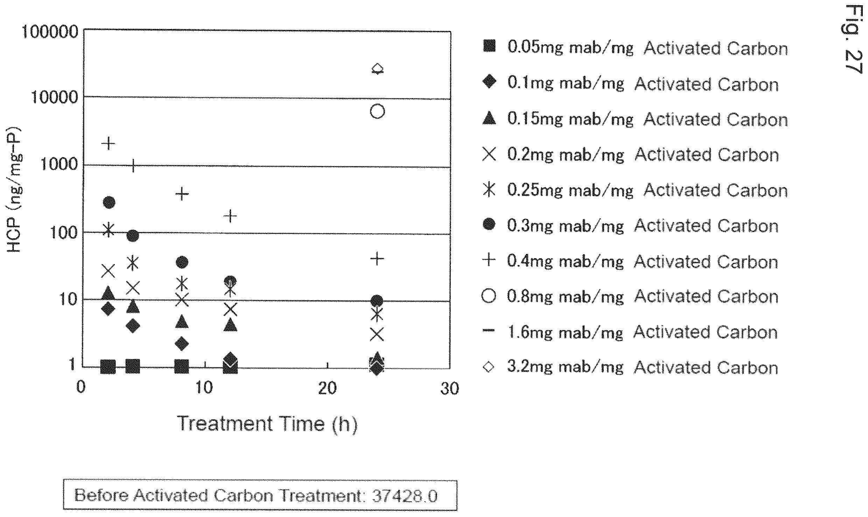

FIG. 27 shows an effect of the activated carbon treatment time applied to the concentration of the host cell protein (HCP). A vertical axis indicates the HCP concentration (ng/mg-P) and a horizontal axis indicates the treatment time (h). A black square shows a result of the load volume of 0.05 mg mab/mg activated carbon, a black diamond shows a result of the load volume of 0.1 mg mab/mg activated carbon, a black triangle shows a result of the load volume of 0.15 mg mab/mg activated carbon, x shows a result of the load volume of 0.2 mg mab/mg activated carbon, * shows a result of the load volume of 0.25 mg mab/mg activated carbon, a black circle shows a result of the load volume of 0.3 mg mab/mg activated carbon, + shows a result of the load volume of 0.4 mg mab/mg activated carbon, a white circle shows a result of the load volume of 0.8 mg mab/mg activated carbon, - shows a result of the load volume of 1.6 mg mab/mg activated carbon, and a white diamond shows a result of the load volume of 3.2 mg mab/mg activated carbon. The HCP concentration before the activated carbon treatment is 37428.0 ng/mg-P.

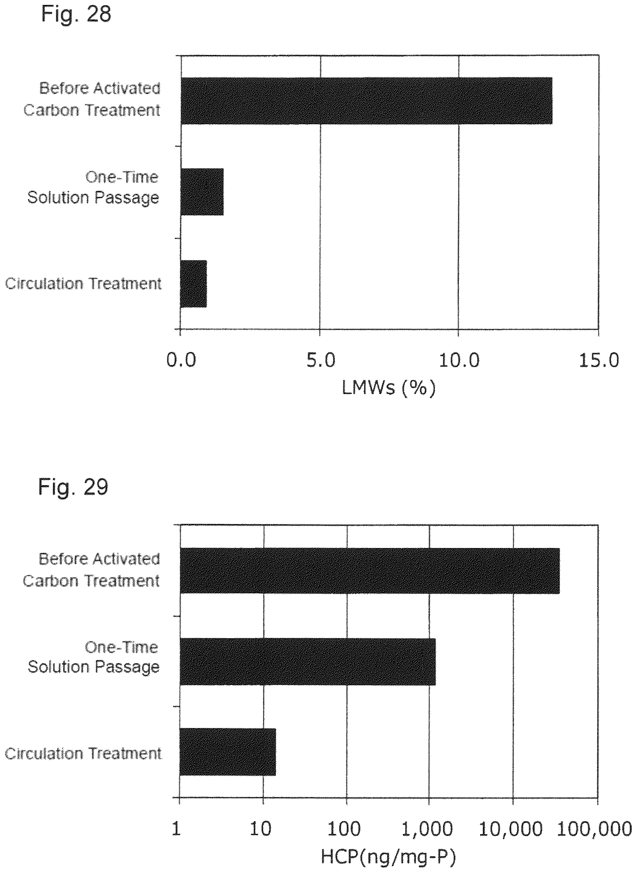

FIG. 28 shows an effect of a circulation treatment of the activated carbon pretreatment solution applied to the content of the protein-derived degradation products (LMWs).

FIG. 29 shows an effect of the circulation treatment of the activated carbon pretreatment solution applied to the concentration of the host cell protein (HCP).

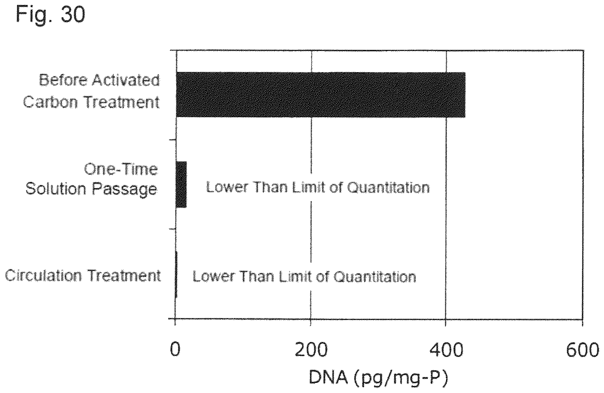

FIG. 30 shows an effect of the circulation treatment of the activated carbon pretreatment solution applied to the DNA concentration.

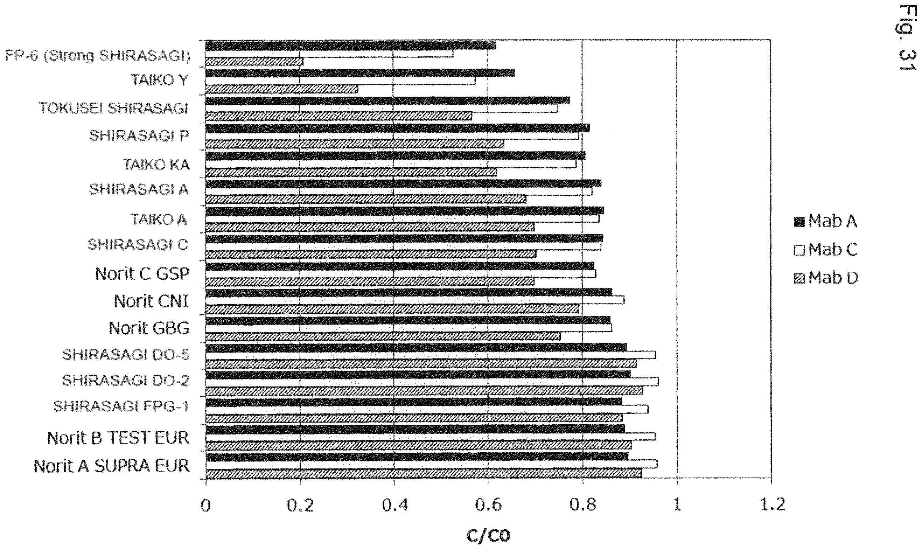

FIG. 31 shows an effect of a difference of activated carbon types applied to the recovery ratio ($C/C_0$). A black bar shows a result of the pH-adjusted clarified solution including Mab A, a white bar shows a result of the pH-adjusted clarified solution including Mab C, and an oblique line bar shows a result of the pH-adjusted clarified solution including Mab C.

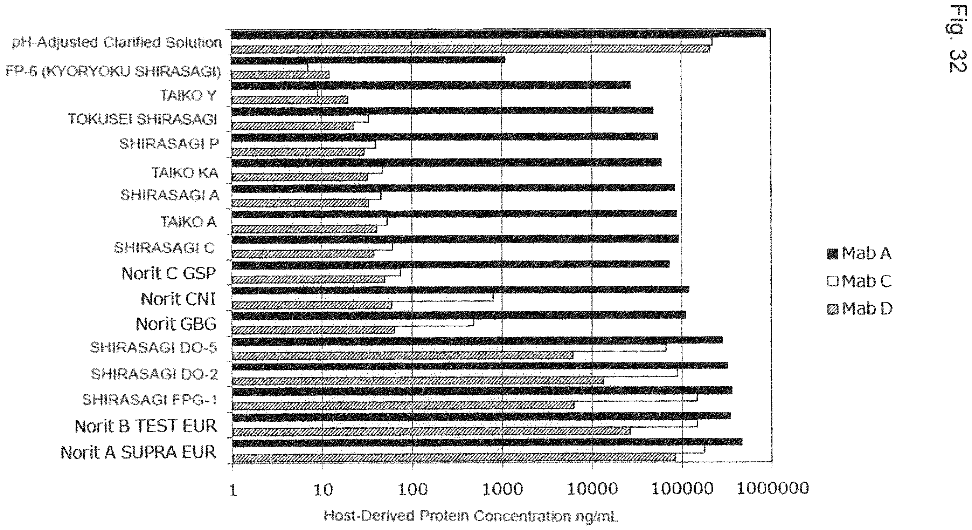

FIG. 32 shows an effect of a difference of activated carbon types applied to the concentration of the host cell protein (HCP). A black bar shows a result of the pH-adjusted clarified solution including Mab A, a white bar shows a result of the pH-adjusted clarified solution including Mab C, and an oblique line bar shows a result of the pH-adjusted clarified solution including Mab C.

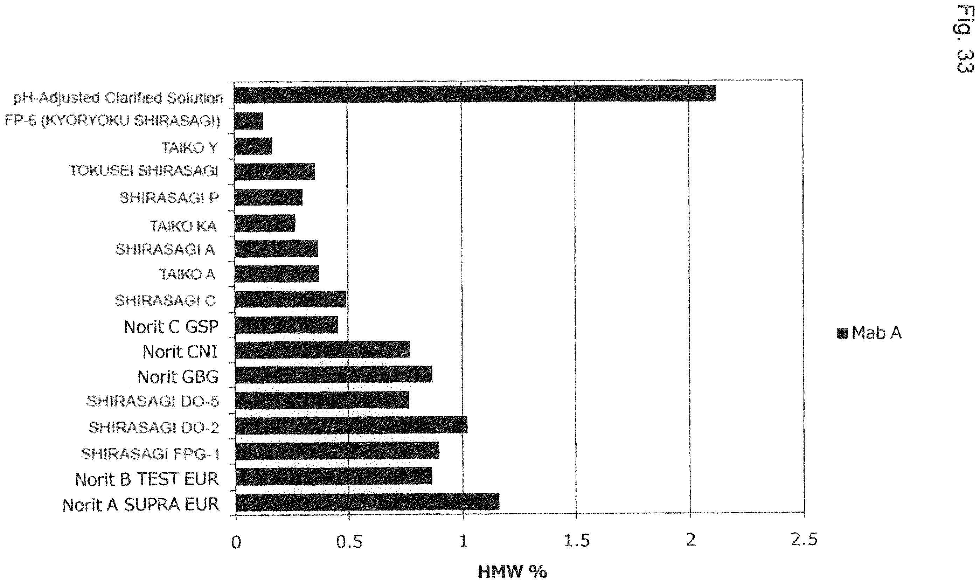

FIG. 33 shows an effect of a difference of activated carbon types applied to the content of the protein-derived polymers (HMWs).

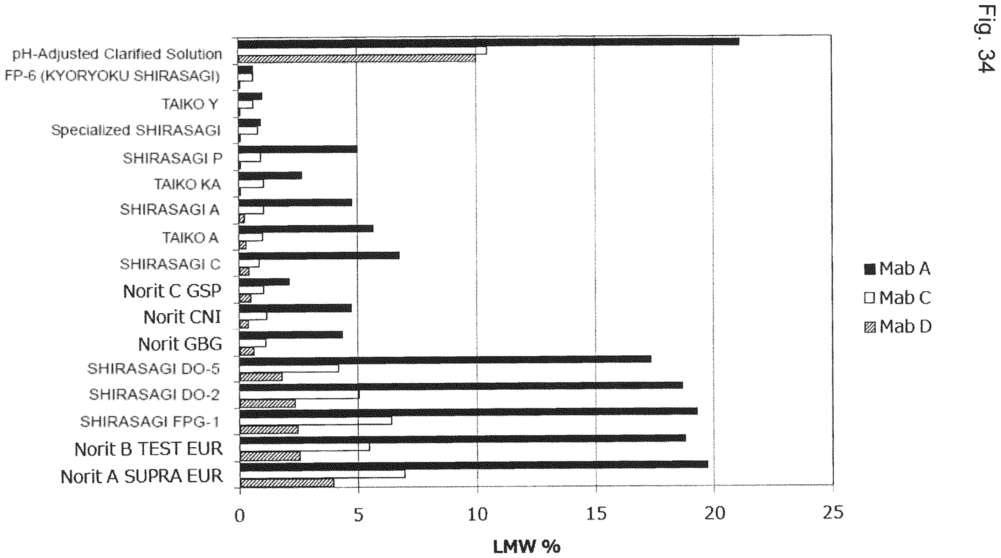

FIG. 34 shows an effect of a difference of activated carbon types applied to the content of the protein-derived degradation products (LMWs). A black bar shows a result of the pH-adjusted clarified solution including Mab A, a white bar shows a result of the pH-adjusted clarified solution including Mab C, and an oblique line bar shows a result of the pH-adjusted clarified solution including Mab C.

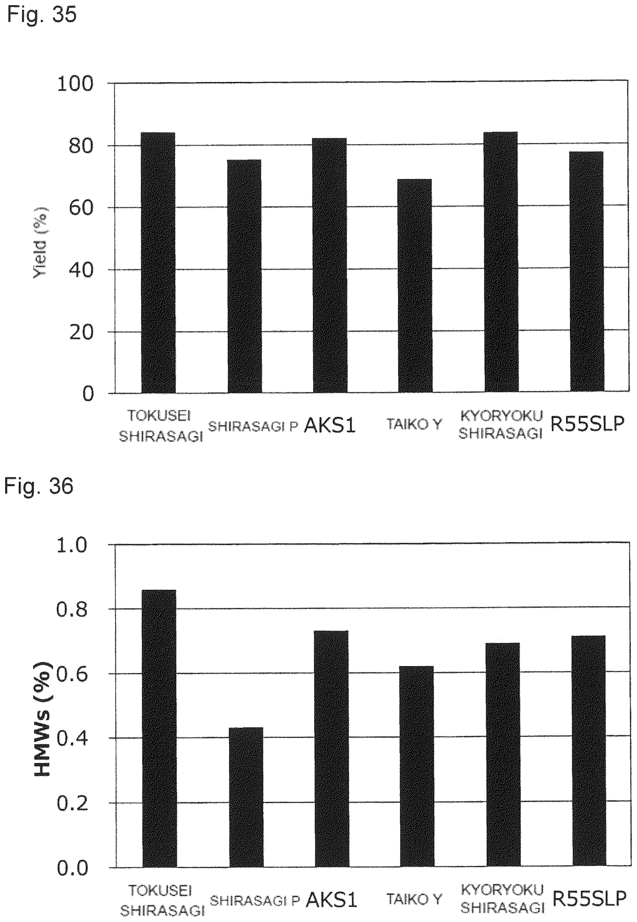

FIG. 35 shows an effect of a difference of activated carbon membrane types applied to a yield.

FIG. 36 shows an effect of a difference of activated carbon membrane types applied to the content of the protein-derived polymers (HMWs).

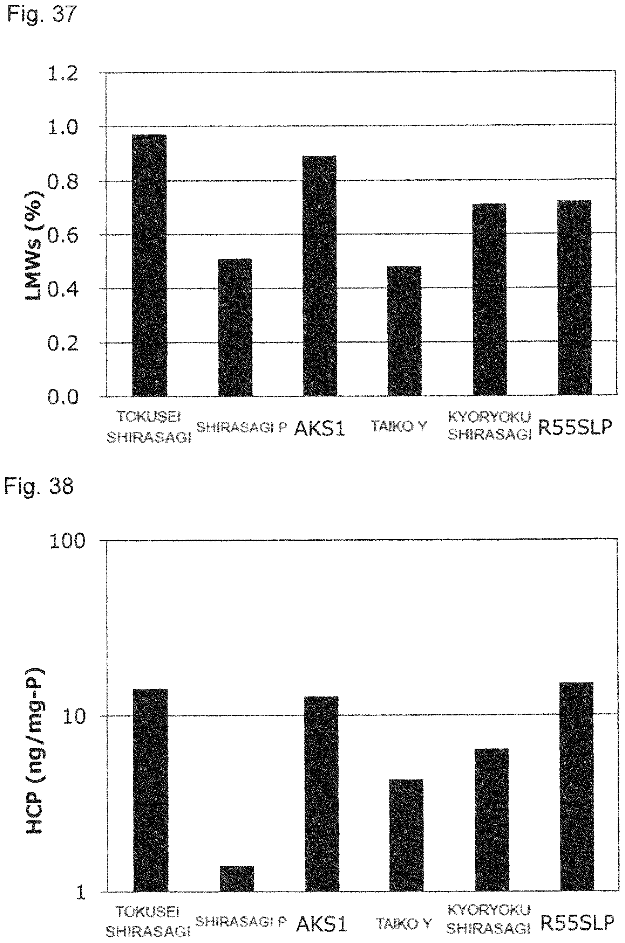

FIG. 37 shows an effect of a difference of activated carbon membrane types applied to the content of the protein-derived degradation products (LMWs).

FIG. 38 shows an effect of a difference of activated carbon membrane types applied to the concentration of the host cell protein (HCP).

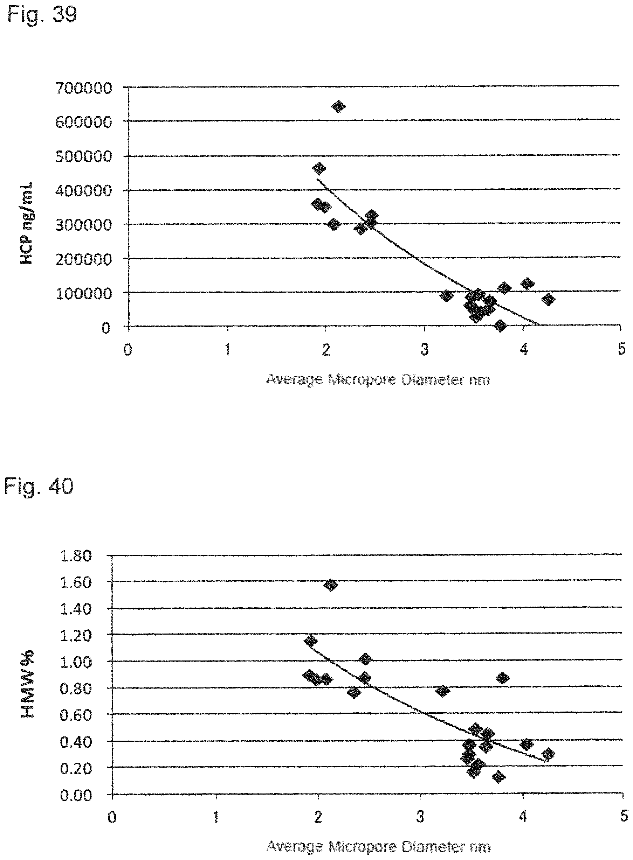

FIG. 39 shows a relationship between an average micropore diameter of the activated carbon and the concentration of the host cell protein (HCP).

FIG. 40 shows a relationship between the average micropore diameter of the activated carbon and the content of the protein-derived polymers (HMWs).

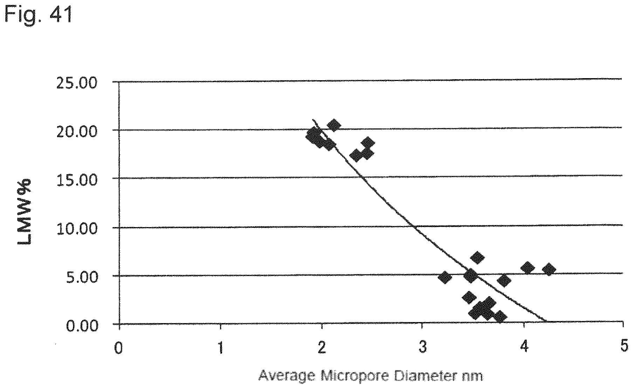

FIG. 41 shows a relationship between the average micropore diameter of the activated carbon and the content of the protein-derived degradation products (LMWs).

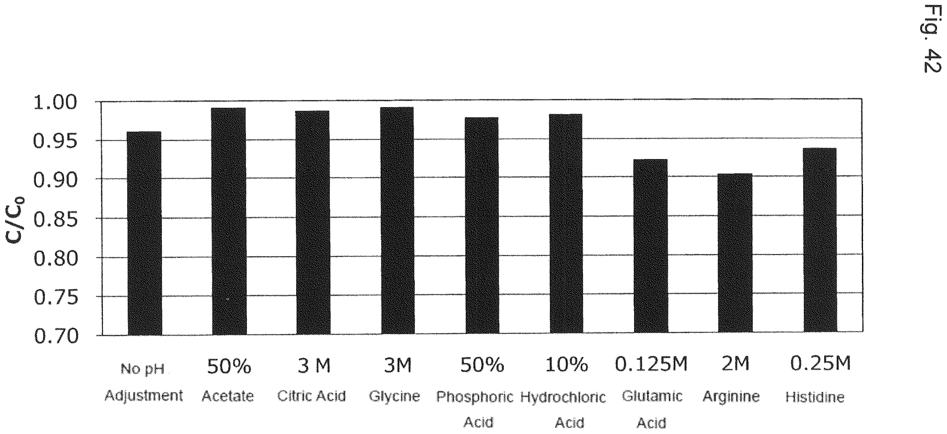

FIG. 42 shows an effect of an acid type used for pH adjustment applied to the recovery ratio ($C/C_0$).

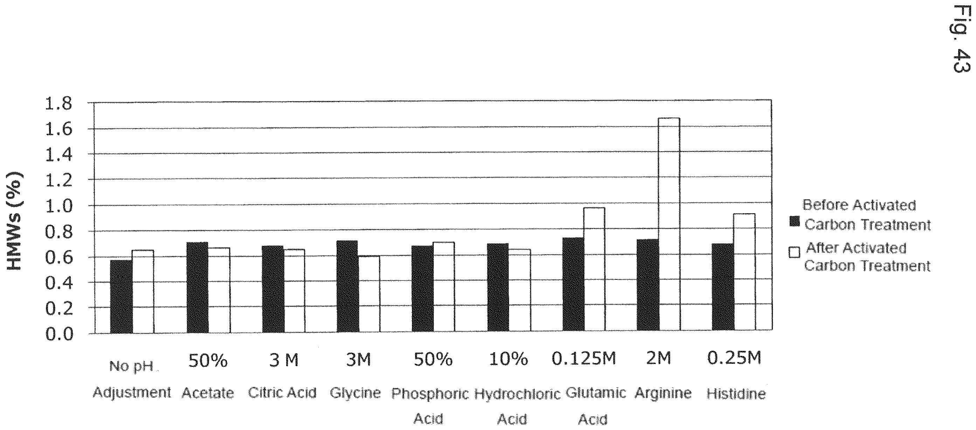

FIG. 43 shows an effect of the acid type used for pH adjustment applied to the content of the protein-derived polymers (HMWs). A black bar shows a result before the activated carbon treatment and a white bar shows a result after the activated carbon treatment.

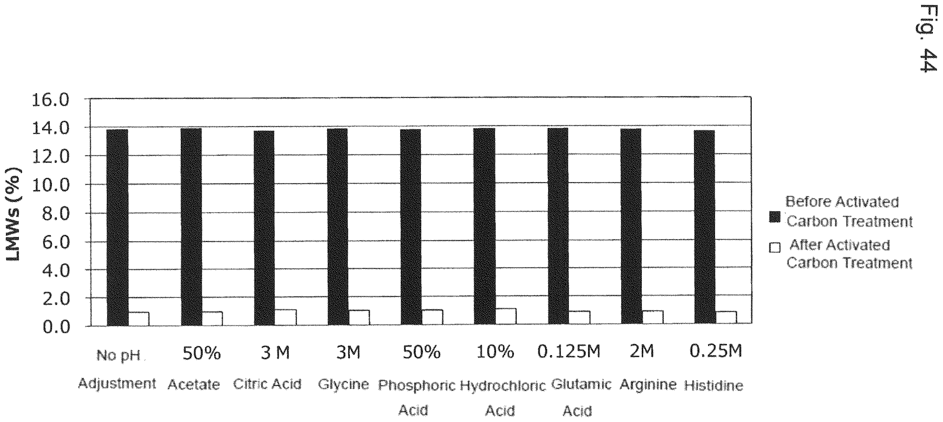

FIG. 44 shows an effect of the acid type used for pH adjustment applied to the content of the protein-derived degradation products (LMWs). A black bar shows a result before the activated carbon treatment and a white bar shows a result after the activated carbon treatment.

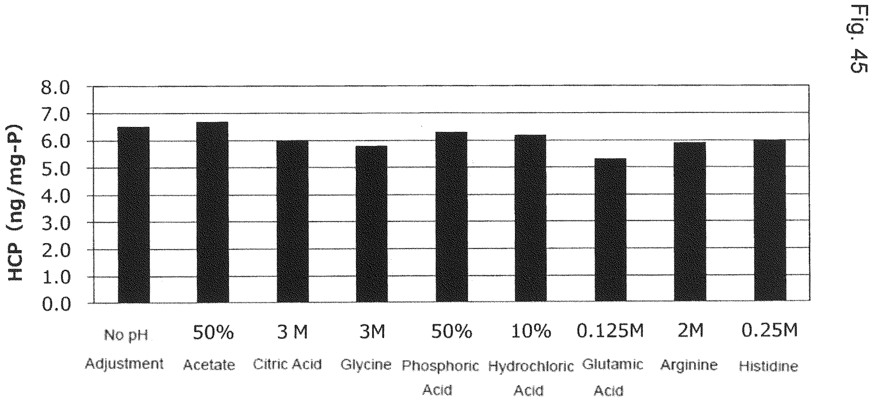

FIG. 45 shows an effect of the acid type used for pH adjustment applied to the concentration of the host cell protein (HCP).

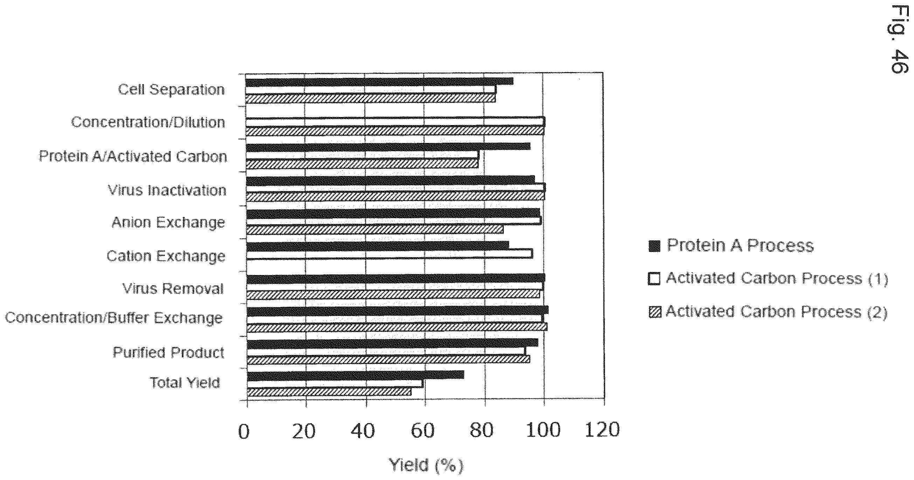

FIG. 46 shows process yields and total yields of protein A process, an activated carbon process (1), and an activated carbon process (2) with Mab A purification. A black bar shows a result of the protein A process, a white bar shows a result of the activated carbon process (1), and an oblique line bar shows a result of the activated carbon process (2). A horizontal axis indicates the yield (%).

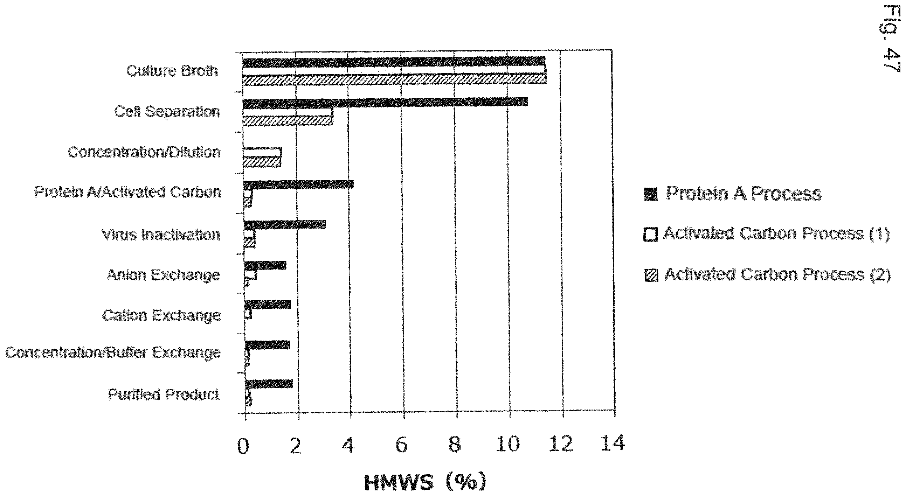

FIG. 47 shows the content of the protein-derived polymers (HMWs) of each process of the protein A process, the activated carbon process (1), and the activated carbon process (2) with Mab A purification. A black bar shows a result of the protein A process, a white bar shows a result of the activated carbon process (1), and an oblique line bar shows a result of the activated carbon process (2). A horizontal axis indicates the HMWs content (%).

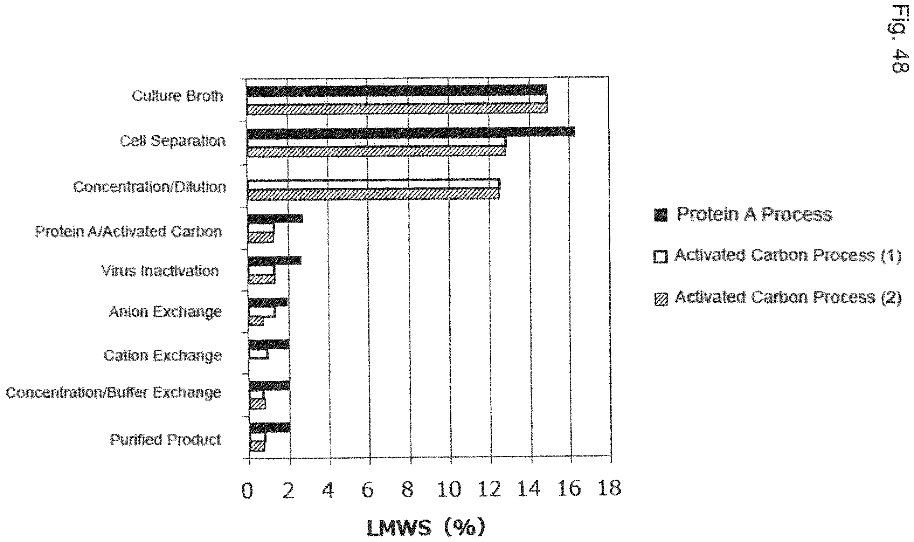

FIG. 48 shows the content of the protein-derived degradation products (LMWs) of each process of the protein A process, the activated carbon process (1), and the activated carbon process (2) with Mab A purification. A black bar shows a result of the protein A process, a white bar shows a result of the activated carbon process (1), and an oblique line bar shows a result of the activated carbon process (2). A horizontal axis indicates the LMWs content (%).

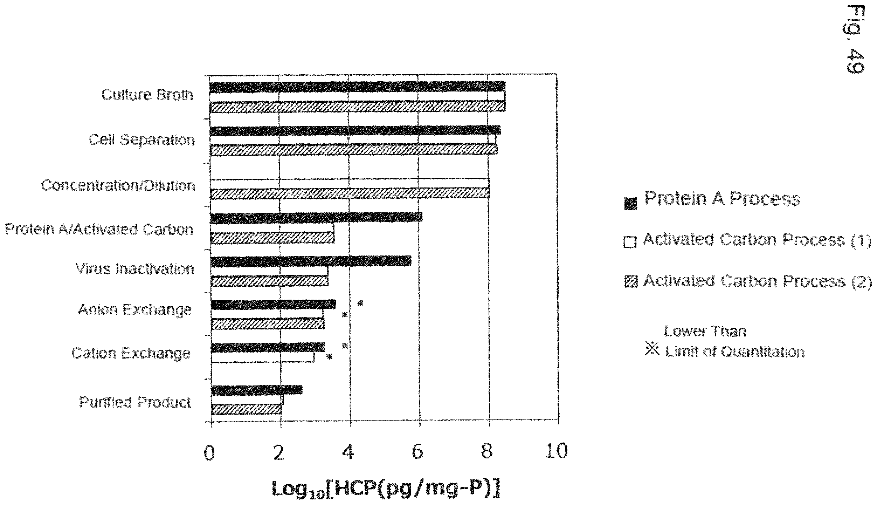

FIG. 49 shows the concentration of the host cell protein (HCP) of each process of the protein A process, the activated carbon process (1), and the activated carbon process (2) with Mab purification. A black bar shows a result of the protein A process, a white bar shows a result of the activated carbon process (1), and an oblique line bar shows a result of the activated carbon process (2). A horizontal axis indicates the HCP concentration (pg/mg-P). * indicates the value lower than a limit of quantitation.

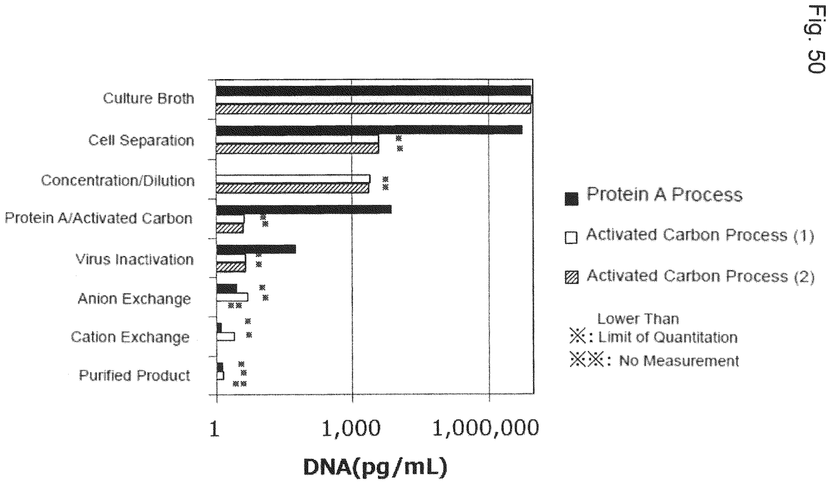

FIG. 50 shows the DNA concentration of each process of the protein A process, the activated carbon process (1), and the activated carbon process (2) with Mab A purification. A black bar shows a result of the protein A process, a white bar shows a result of the activated carbon process (1), and an oblique line bar shows a result of the activated carbon process (2). A horizontal axis indicates the DNA concentration (ng/mL). * indicates the value lower than a limit of quantitation. ** is non-measurement result.

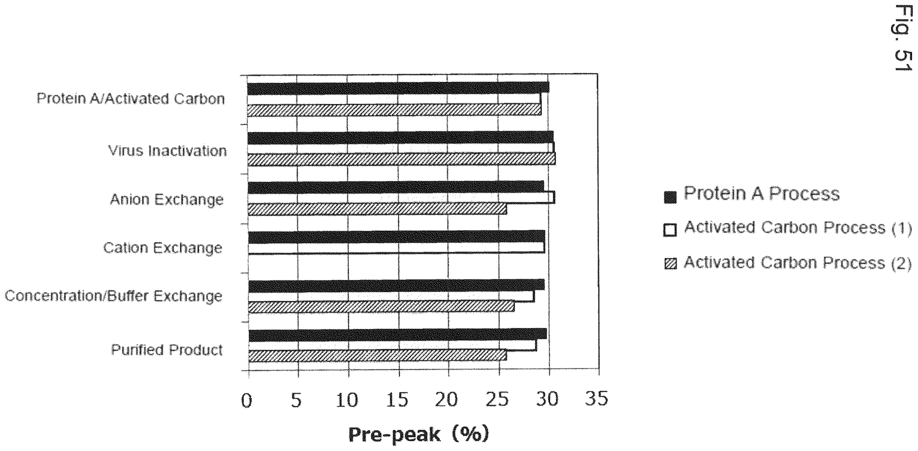

FIG. 51 shows the content of Pre-peak of cation exchange HPLC analysis of each process of the protein A process, the activated carbon process (1), and the activated carbon process (2) with Mab A purification. A black bar shows a result of the protein A process, a white bar shows a result of the activated carbon process (1), and an oblique line bar shows a result of the activated carbon process (2). A horizontal axis indicates the Pre-peak content (%).

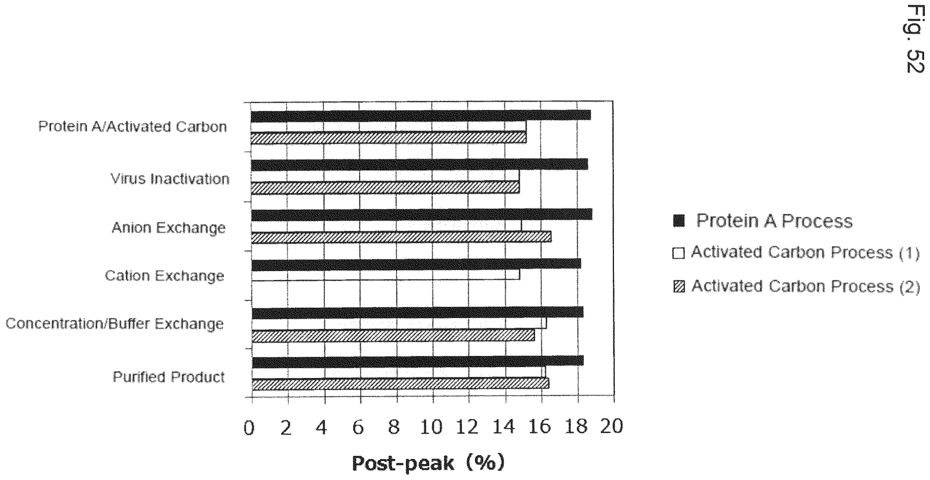

FIG. 52 shows the content of Post-peak of cation exchange HPLC analysis of each process of the protein A process, the activated carbon process (1), and the activated carbon process (2) with Mab A purification. A black bar shows a result of the protein A process, a white bar shows a result of the activated carbon process (1), and an oblique line bar shows a result of the activated carbon process (2). A horizontal axis indicates the Post-peak content (%).

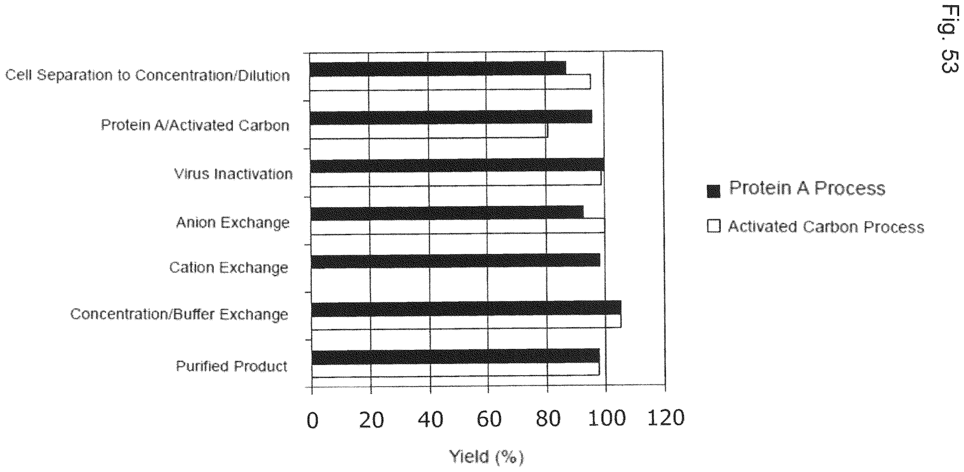

FIG. 53 shows a process yield of a protein A process and the activated carbon process with Mab B purification. A black bar shows a result of the protein A process and a white bar shows a result of the activated carbon process. A horizontal axis indicates the yield (%).

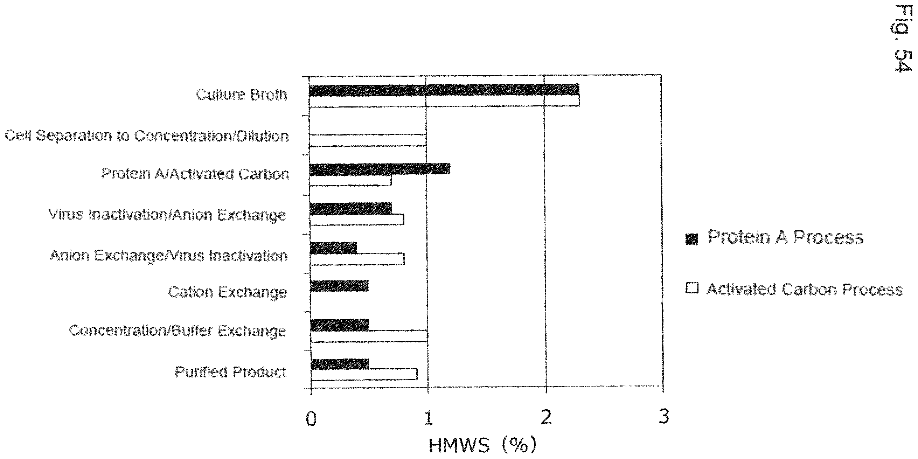

FIG. 54 shows the content of the protein-derived polymers (HMWs) of each step of the protein A process with purified Mab B and the activated carbon process. A black bar shows a result of the protein A process and a white bar shows a result of the activated carbon process. A horizontal axis indicates the HMWs content (%).

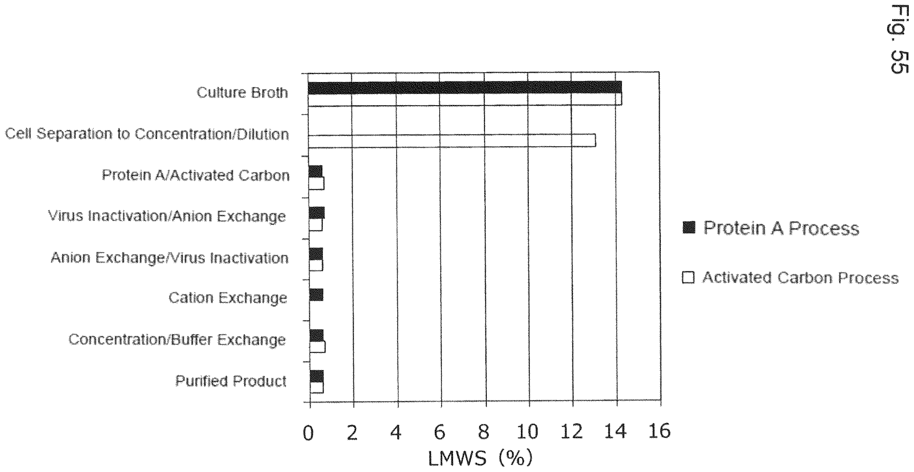

FIG. 55 shows the content of the protein-derived degradation products (LMWs) of each step of the protein A process and the activated carbon process with Mab B purification. A black bar shows a result of the protein A process and a white bar shows a result of the activated carbon process. A horizontal axis indicates the LMWs content (%).

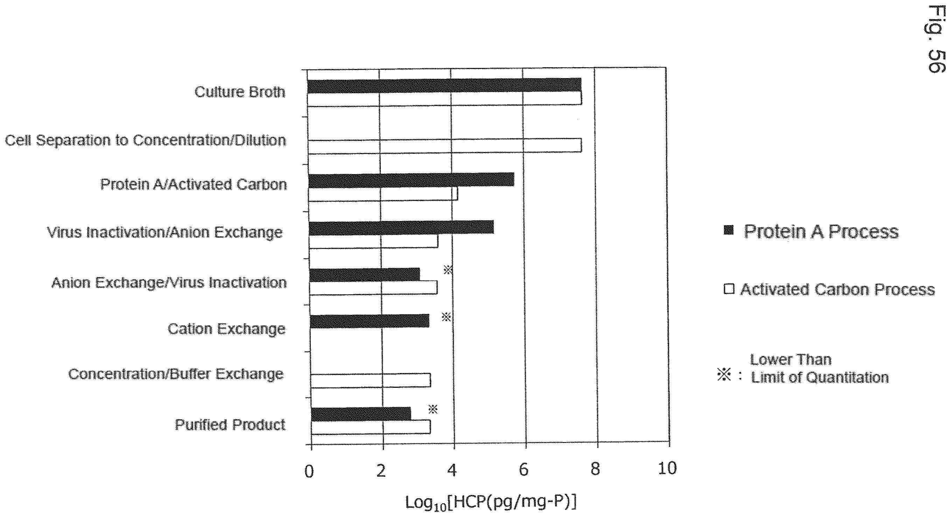

FIG. 56 shows the content of the concentration of the host cell protein (HCP) of each step of the protein A process and the activated carbon process with Mab B purification. A black bar shows a result of the protein A process and a white bar shows a result of the activated carbon process. A horizontal axis indicates a logarithmic value of the HCP concentration (pg/mg-P). * indicates the value lower than a limit of quantitation.

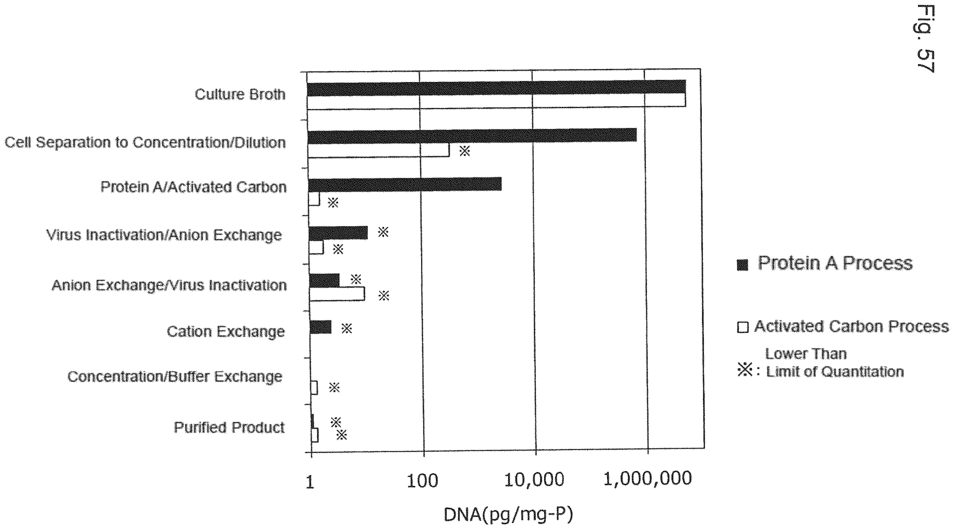

FIG. 57 shows the DNA concentration of each process of the protein A process with and the activated carbon process with Mab B purification. A black bar shows a result of the protein A process and a white bar shows a result of the activated carbon process. A horizontal axis indicates the DNA concentration (ng/mL). * indicates the value lower than a limit of quantitation.

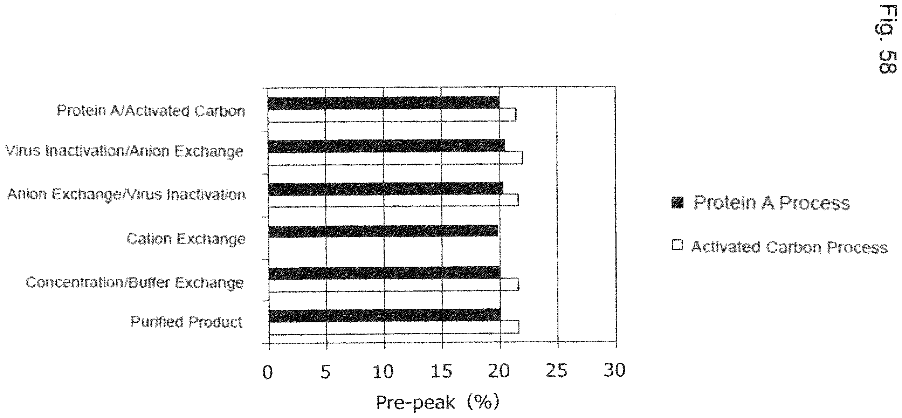

FIG. 58 shows the content of Pre-peak of cation exchange HPLC analysis of each process of the protein A process and the activated carbon process with Mab B purification. A black bar shows a result of the protein A process and a white bar shows a result of the activated carbon process. A horizontal axis indicates the Pre-peak content (%).

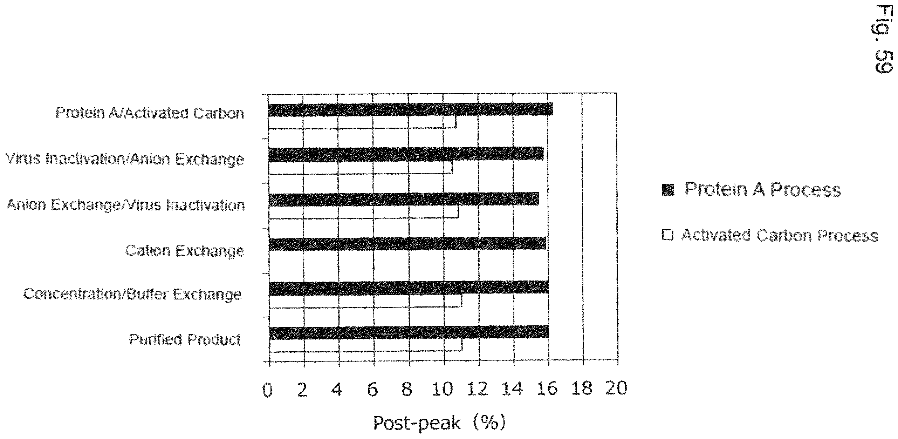

FIG. 59 shows the content of Post-peak of cation exchange HPLC analysis of each process of the protein A process and the activated carbon process with Mab B purification. A black bar shows a result of the protein A process and a white bar shows a result of the activated carbon process. A horizontal axis indicates the Post-peak content (%).

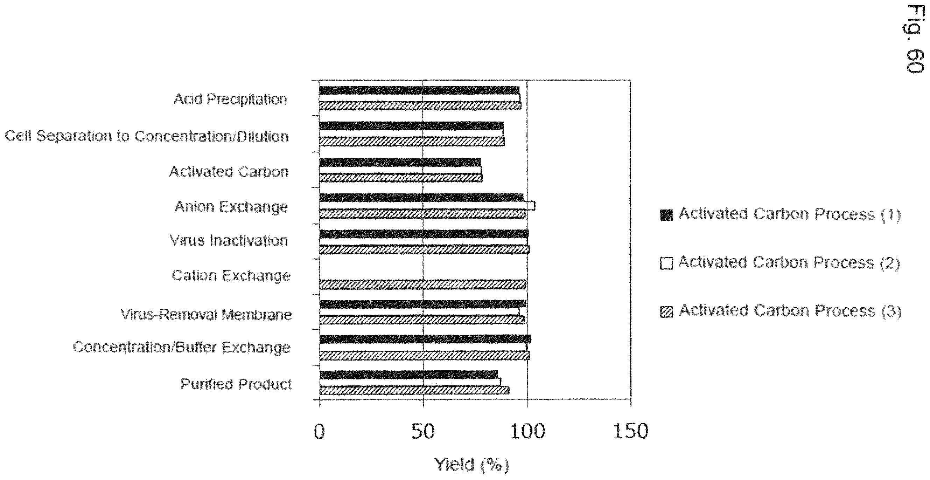

FIG. 60 shows yields of activated carbon processes (1) to (3) with Mab B purification. A black bar shows a result of the activated carbon process (1), a white bar shows a result of the activated carbon process (2), and an oblique line bar shows a result of the activated carbon process (3). A horizontal axis indicates the yield (%).

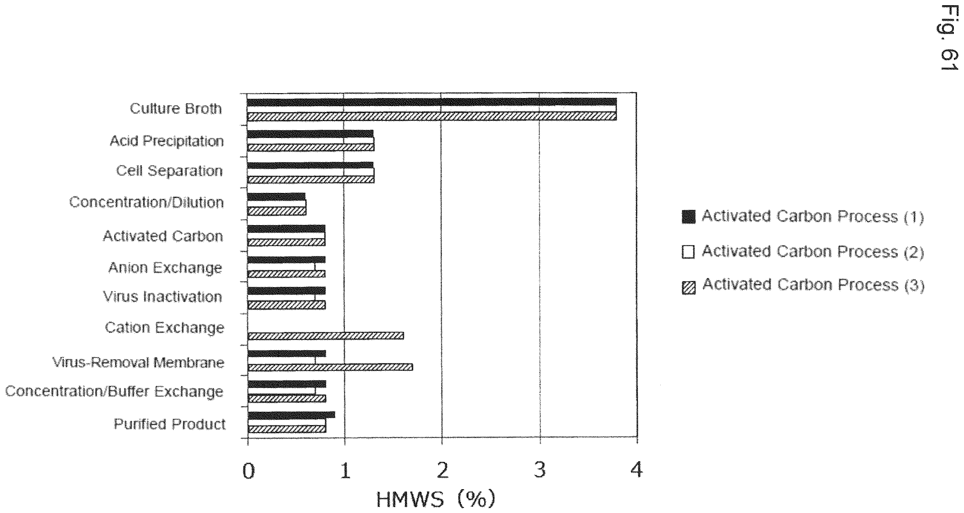

FIG. 61 shows the content of the protein-derived polymers (HMWs) of each process of the activated carbon processes (1) to (3) with Mab B purification. A black bar shows a result of the activated carbon process (1), a white bar shows a result of the activated carbon process (2), and an oblique line bar shows a result of the activated carbon process (3). A horizontal axis indicates the HMWs content (%).

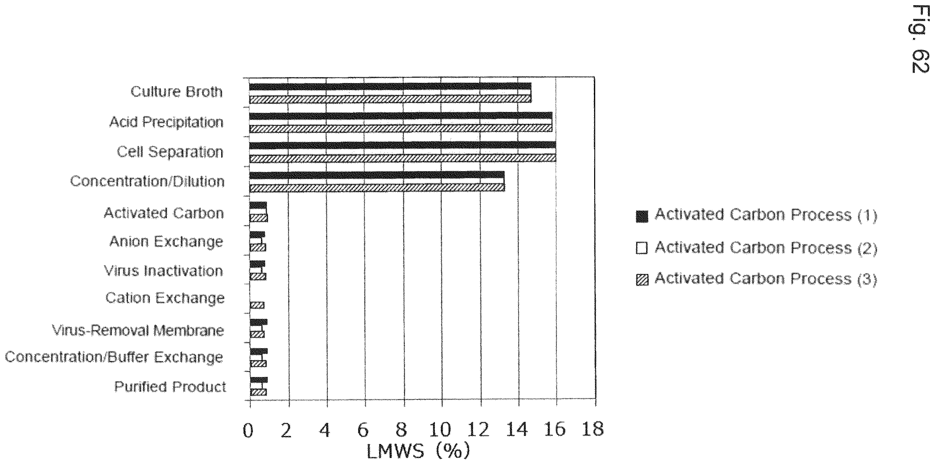

FIG. 62 shows the content of the protein-derived degradation products (LMWs) of each process of the activated carbon processes (1) to (3) with Mab B purification. A black bar shows a result of the activated carbon process (1), a white bar shows a result of the activated carbon process (2), and an oblique line bar shows a result of the activated carbon process (3). A horizontal axis indicates the LMWs content (%).

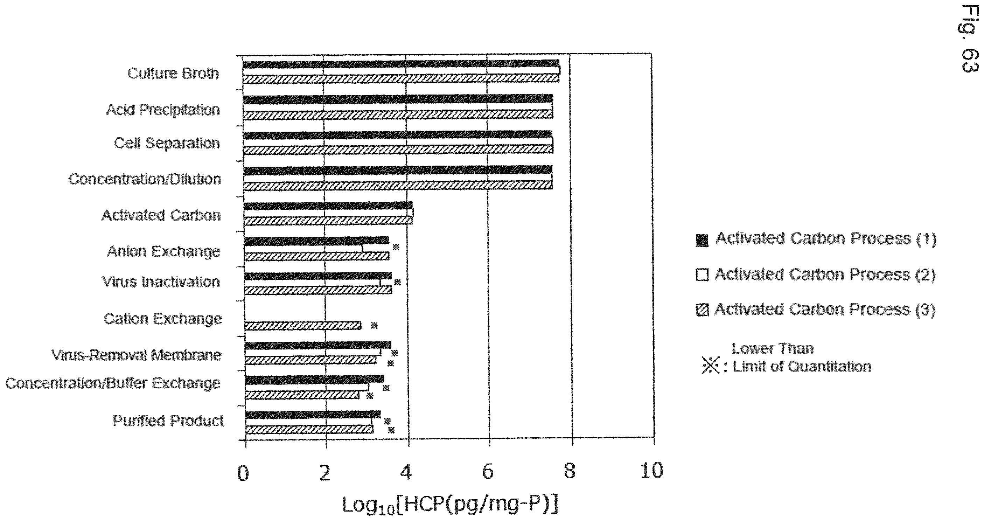

FIG. 63 shows the concentration of the host cell protein (HCP) of each process of the activated carbon processes (1) to (3) with Mab B purification. A black bar shows a result of the activated carbon process (1), a white bar shows a result of the activated carbon process (2), and an oblique line bar shows a result of the activated carbon process (3). A horizontal axis indicates a logarithmic value of the HCP concentration (pg/mg-P). * indicates the value lower than a limit of quantitation.

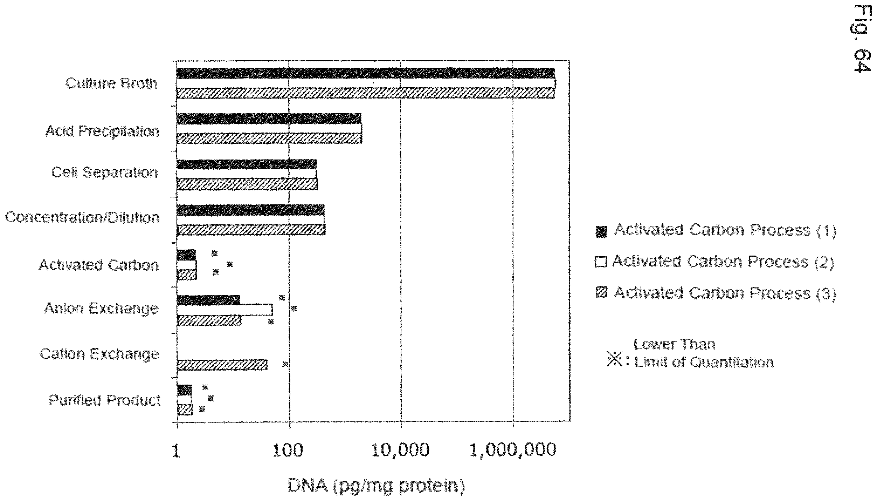

FIG. 64 shows the DNA concentration of each process of the activated carbon processes (1) to (3) with Mab B purification. A black bar shows a result of the activated carbon process (1), a white bar shows a result of the activated carbon process (2), and an oblique line bar shows a result of the activated carbon process (3). A horizontal axis indicates a logarithmic value of the DNA concentration (ng/mL). * indicates the value lower than a limit of quantitation.

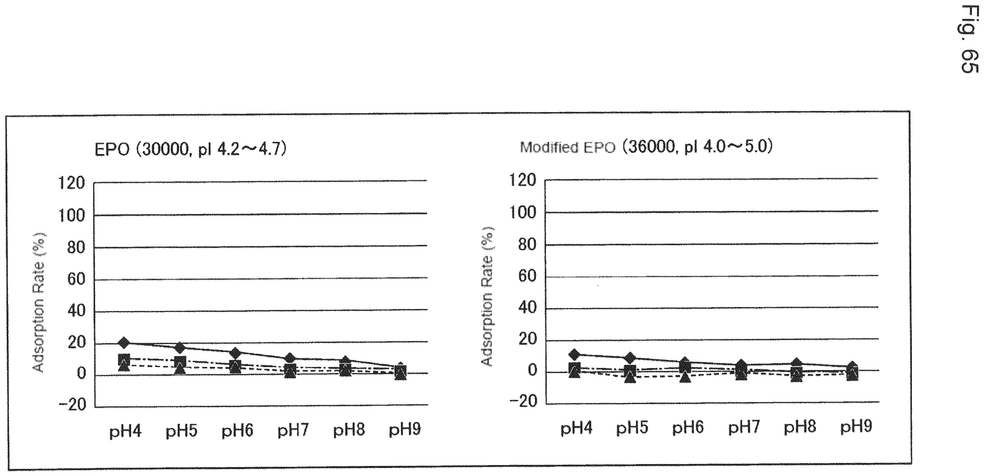

FIG. 65 shows a relationship between pH of EPO or modified EPO and an activated carbon adsorption rate. A vertical axis indicates a protein adsorption rate (%). A black diamond shows a result obtained by using TOKUSEI SHIRASAGI, a black square shows a result obtained by using SHIRASAGI P, and a black triangle shows a result obtained by using SHIRASAGI DO-5.

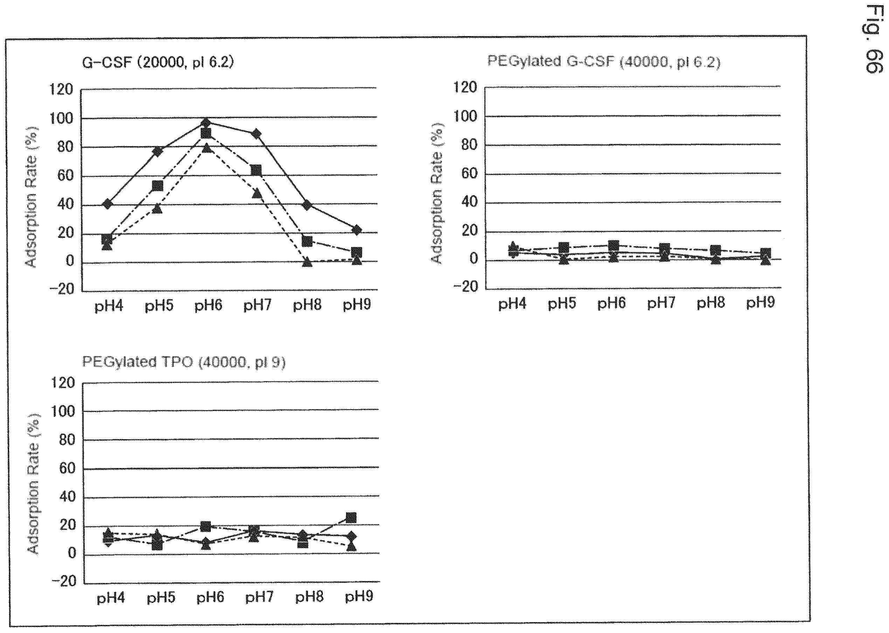

FIG. 66 shows a relationship between pH of G-CSF, PEGylated G-CSF, or PEGylated TPO, and the activated carbon adsorption rate. A vertical axis indicates the protein adsorption rate (%). A black diamond shows a result obtained by using TOKUSEI SHIRASAGI, a black square shows a result obtained by using SHIRASAGI P, and a black triangle shows a result obtained by using SHIRASAGI DO-5.

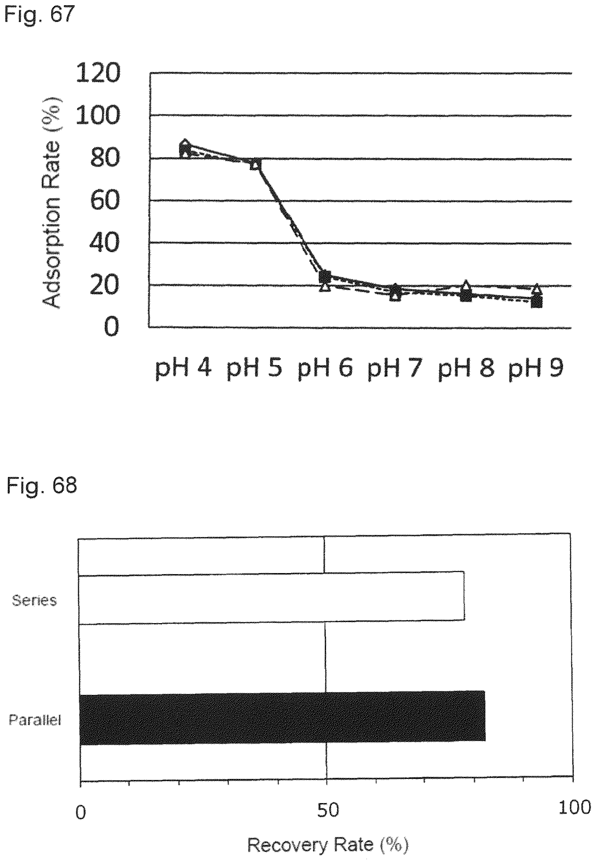

FIG. 67 shows a relationship between pH of the host cell-derived protein and the activated carbon adsorption rate. A vertical axis indicates the protein adsorption rate (%). A black diamond shows a result obtained by using TOKUSEI SHIRASAGI, a black square shows a result obtained by using SHIRASAGI P, and a black triangle shows a result obtained by using SHIRASAGI DO-5.

FIG 68 shows a relationship between a difference of connection methods of activated carbon membranes divided into plural pieces and a recovery rate. A white bar shows a result in a case where the activated carbon membranes are connected in series and a black bar shows a result in a case where the activated carbon membranes are connected in parallel. A horizontal axis indicates the recovery rate (%).

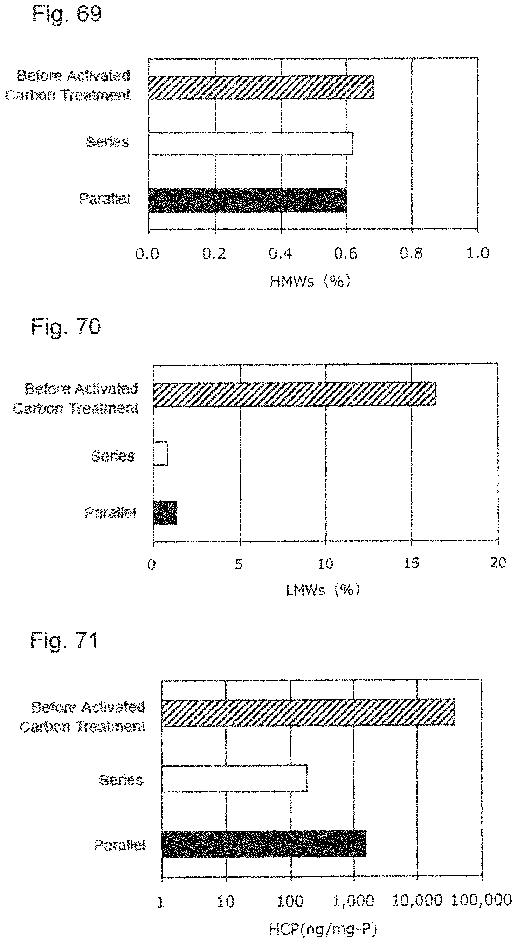

FIG. 69 shows a relationship between a difference of connection methods of the activated carbon membranes divided into plural pieces and the content of the protein-derived polymers (HMWs). An oblique line bar shows a result before the activated carbon treatment, a white bar shows a result in a case where the activated carbon membranes are connected in series, and a black bar shows a result in a case where the activated carbon membranes are connected in parallel. A horizontal axis indicates the HMWs content (%).

FIG. 70 shows a relationship between a difference of connection methods of the activated carbon membranes divided into plural pieces and the content of the protein-derived degradation products (LMWs). An oblique line bar shows a result before the activated carbon treatment, a white bar shows a result in a case where the activated carbon membranes are connected in series, and a black bar shows a result in a case where the activated carbon membranes are connected in parallel. A horizontal axis indicates the LMWs content (%).

FIG. 71 shows a relationship between a difference of connection methods of the activated carbon membranes divided into plural pieces and the concentration of the host cell protein (HCP). An oblique line bar shows a result before the activated carbon treatment, a white bar shows a result in a case where the activated carbon membranes are connected in series, and a black bar shows a result in a case where the activated carbon membranes are connected in parallel. A horizontal axis indicates the HCP concentration (ng/mg-P).

Embodiments for Carrying Out the Invention

The present invention relates to a method for purifying a protein as claimed using an activated carbon, in which an activated carbon pretreatment solution obtained by adjusting a conductivity of a protein-containing aqueous solution is brought into contact with an activated carbon, the protein and impurities are separated in a non-adsorption mode to obtain the protein of interest having reduced content of impurities at a high recovery rate.

Examples of the protein purified according to the present disclosure include high-molecular weight proteins, and among these, a protein having hydrophilicity applied due to modification or the like is preferable, and for example, a natural or non-natural glycoprotein or a protein modified with a water-soluble polymer is used.

Examples of glycoprotein include an antibody, erythropoietin, modified erythropoietin, darbepoetin, antithrombin ($\alpha$ or $\beta$ form, or mixtures thereof).

The protein modified with the water-soluble polymer indicates a protein to which at least one water-soluble polymer molecule is bonded directly or indirectly through a linker or the like. Examples of the water-soluble polymer include polyethylene glycol (PEG), a copolymer of ethylene glycol/propylene glycol, carboxymethyl cellulose, dextran, polyvinyl alcohol, polyvinyl pyrrolidone, poly-1,3-dioxolane, poly-1,3,6-trioxane, an ethylene/maleic anhydride copolymer, and polyamino acids (homopolymer or random copolymer).

Examples of the protein modified with the water-soluble polymer include PEGylated proteins such as PEGylated Granulocyte Colony Stimulating Factor (G-CSF) including pegfilgrastim or PEGylated thrombopoietin (TPO).

Examples of the antibody include polyclonal antibodies or a monoclonal antibodies, and monoclonal antibodies are preferable. Examples of the antibodies may include mouse antibodies, llama antibodies, chimeric antibodies, humanized antibodies, human antibodies, antibodies with modified Fc regions, monovalent antibodies, multispecific antibodies such as bispecific antibodies, and antibody fragments thereof. Examples of the molecular type of the antibody include IgG, IgM, IgA, IgD, IgE, Fab, Fc, Fc-fusion proteins, VH, VL, VHH, Fab'$_2$, scFv, scFab, scDb, scDbFc or the like.

Any antibody may be used as long as it has antigen binding, and examples thereof include an antibody recognizing a tumor associated antigen or antibody fragments thereof, an antibody recognizing an antigen relating to allergy or inflammation or antibody fragments thereof, an antibody recognizing an antigen relating to cardiovascular disease or antibody fragments thereof, an antibody recognizing an antigen relating to autoallergic disease or antibody fragments thereof, and an antibody recognizing an antigen relating to viruses or bacterial infection or antibody fragments thereof.

Examples of the antigen include CD la, CD2, CD3, CD4, CD5, CD6, CD7, CD9, CD10, CD13, CD19, CD20, CD21, CD22, CD25, CD28, CD30, CD32, CD33, CD38, CD40, CD40 ligand (CD40L), CD44, CD45, CD46, CD47, CD52, CD54, CD55, CD56, CD59, CD63, CD64, CD66b, CD69, CD70, CD74, CD80, CD89, CD95, CD98, CD105, CD134 (OX40), CD137, CD138, CD147, CD158, CD160, CD162, CD164, CD200, CD227, adrenomedullin, angiopoietin related protein 4 (ARP4), aurora, B7-H1, B7-DC, integlin, bone marrow stromal antigen 2 (BST2), CA125, CA19.9, carbonic anhydrase 9 (CA9), cadherin, cc-chemokine receptor (CCR)4, CCR7, carcinoembryonic antigen (CEA), cysteine-rich fibroblastgrowth factor receptor-1 (CFR-1), c-Met, c-Myc, collagen, CTA, connective tissuegrowth factor (CTGF), CTLA-4, cytokeratin-18, DF3, E-catherin, epidermalgrowth factor receptor (EGFR), EGFRvIII, EGFR2 (HER2), EGFR3 (HER3), EGFR4 (HER4), endoglin, epithelial cell adhesion molecule (EpCAM), endothelial protein C receptor (EPCR), ephrin, ephrin receptor (Eph), EphA2, endotheliase-2 (ET2), FAM3D, fibroblast activating protein (FAP), Fc receptor homolog 1 (FcRH1), ferritin, fibroblastgrowth factor8 (FGF8), FGF8 receptor, basic FGF (bFGF), bFGF receptor, FGF receptor (FGFR)3, FGFR4, FLT1, FLT3, folate receptor, frizzled homologue 10 (FZD10), frizzled receptor 4 (FZD-4), G250, G-CSF receptor, ganglioside (for example, GD2, GD3, GM2 or GM3), globo H, gp75, gp88, GPR-9-6, heparanase I, hepatocytegrowth factor (HGF), Heparin-binding EGF-like growth factor (HB-EGF), HGF receptor, HLA antigen (for example, HLA-DR), HM1.24, human milk fatglobule (HMFG), hRS7, heat shock protein 90 (hsp90), idiotype epitope, insulin-likegrowth factor (IGF), IGF receptor (IGFR), interleukin (for example, IL-6 or IL-15), interleukin receptor (for example, IL-6R or IL-15R), integrin, immune receptor translocation associated-4 (IRTA-4), kallikrein 1, KDR, KIR2DL1, KIR2DL2/3, KS1/4, lamp-1, lamp-2, laminin-5, Lewis y, sialyl Lewis x, lymphotoxin-beta receptor (LTBR), LUNX, melanoma-associated chondroitin sulfate proteoglycan (MCSP), mesothelin, MICA, Mullerian inhibiting substance type II receptor (MISIIR), mucin, neural cell adhesion molecule (NCAM), Necl-5, Notch1, osteopontin, platelet-derivedgrowth factor (PDGF), PDGF receptor, platelet factor-4 (PF-4), phosphatidylserine, Prostate Specific Antigen (PSA), prostate stem cell antigen (PSCA), prostate specific membrane antigen (PSMA), Parathyroid hormone related protein/peptide (PTHrP), receptor activator of NF-kappaB ligand (RANKL), receptor for hyaluronic acid mediated motility (RHAMM), ROBO1, SART3, semaphorin 4B (SEMA4B), secretory leukocyte protease inhibitor (SLPI), SM5-1, sphingosine-1-phosphate, tumor-associatedglycoprotein-72 (TAG-72), transferrin receptor (TfR), TGF-beta, Thy-1, Tie-1, Tie2 receptor, T cell immunoglobulin domain and mucin domain 1 (TIM-1), T cell immunoglobulin domain and mucin domain 3 (TIM-3), human tissue factor (hTF), Tn antigen, tumor necrosis factor (TNF), Thomsen-Friedenreich antigen (TF antigen), TNF receptor, tumor necrosis factor-related apoptosis-inducing ligand (TRAIL), TRAIL receptor (for example, DR4 or DR5), system ASC amino acid transporter 2 (ASCT2), trkC, TROP-2, TWEAK receptor Fn14, type IV collagenase, urokinase receptor, vascular endothelialgrowth factor (VEGF), VEGF receptor (for example, VEGFR1, VEGFR2, or VEGFR3), vimentin, and VLA-4.

Specific examples of the antibody include the following antibodies.

Examples of the antibody recognizing a tumor associated antigen include Anti-GD2 Antibody [Anticancer Res., 13,331 (1993)], Anti-GD3 Antibody [Cancer Immunol. Immunother., 36,260 (1993)], Anti-GM2 Antibody [Cancer Res., 54,1511 (1994)], Anti-HER2 Antibody [Proc. Natl. Acad. Sci. USA, 89,4285 (1992), US5725856], Anti-CD52 Antibody [Proc. Natl. Acad. Sci. USA, 89,4285 (1992)], Anti-MAGE Antibody [British J.Cancer, 83,493 (2000)], Anti-HM1.24 Antibody [Molecular Immunol., 36,387 (1999)], Antiparathyroid hormone-related protein (PTHrP) Antibody [Cancer, 88,2909(2000)], Anti-bFGF Antibody, Anti-FGF-8 Antibody [Proc. Natl. Acad. Sci. USA, 86,9911 (1989)], Anti-bFGFR Antibody, Anti-FGF-8R Antibody [J. Biol. Chem., 265,16455 (1990)], Anti-IGF Antibody [J. Neurosci. Res., 40,647 (1995)], Anti-IGF-IR Antibody [J. Neurosci. Res., 40,647 (1995)], Anti-PSMAAntibody [J. Urology, 160,2396(1998)], Anti-VEGF Antibody [Cancer Res.,57,4593 (1997)], Anti-VEGFR Antibody [Oncogene, 19,2138 (2000), International Patent Publication No. 96/30046], Anti-CD20 Antibody [Curr. Opin. Oncol., 10,548 (1998), US Patent No. 5736137], Anti-CD10 Antibody, Anti-EGFR Antibody (International Patent Publication No. 96/40201), Anti-Apo-2R Antibody (International Patent Publication No. 98/51793), Anti-ASCT2 Antibody (International Patent Publication No. 2010/008075), Anti-CEAAntibody [Cancer Res., 55 (23 suppl): 5935s-5945s (1995)], Anti-CD38 Antibody, Anti-CD33 Antibody, Anti-CD22 Antibody, Anti-EpCAM Antibody, and Anti-A33 Antibody.

Examples of the antibody recognizing an antigen relating to allergy or inflammation include Anti-Interleukin 10 Antibody [Immunol.Rev., 127, 5(1992)], Anti-Interleukin 6 receptor Antibody [Molecular Immunol., 31, 371(1994)], Anti-Interleukin 5 Antibody [Immunol.Rev., 127, 5(1992)], Anti-Interleukin 5 receptor Antibody, Anti-Interleukin 4 Antibody [Cytokine, 3, 562(1991)], Anti-Interleukin 4 receptor Antibody [J.Immunol.Methods, 217, 41(1998)], tumor necrosis factor Antibody [Lymph.Cyto.Res., 13, 183(1994)], tumor necrosis factor receptor Antibody [Molecular Pharmacol., 58, 237(2000)], Anti-CCR4 Antibody [Nature, 400, 776(1999)], Anti-chemokine Antibody [Peri et al., J.Immunol.Meth., 174, 249(1994)], and Anti-chemokine receptor Antibody [J.Exp.Med., 186, 1373(1997)].

Examples of the antibody recognizing an antigen relating to cardiovascular disease include Anti-GPIIb/IIIa Antibody [J. Immunol., 152, 2968 (1994)], platelet-derived growth factor antibody [Science, 253, 1129 (1991)], platelet-derived growth factor antibody receptor Antibody [J. BioL Chem., 272, 17400 (1997)], blood anticoagulation factor Antibody [Circulation, 101, 1158 (2000)], Anti-IgE Antibody, Anti-αVβ3 Antibody or α4β7 Antibody.

Examples of the antibody recognizing an antigen relating to viruses or bacterial infection include Anti-gp120 Antibody [Structure, 8, 385 (2000)], Anti-CD4 Antibody [J. Rheumatology, 25, 2065 (1998)], Anti-CCR5 Antibody, and Anti-verotoxin Antibody [J. Clin. Microbiol. , 37, 396 (1999)].

As Fc-fusion proteins, for example, Romplasmichim is used.

In the purification method of the present invention, a protein-containing aqueous solution that includes a protein of interest and impurities is provided.

Examples of the protein-containing aqueous solution may include an aqueous solution obtained from the living body, such as plasma, serum, breast milk, or urine, an aqueous solution formed of a culture broth obtained by culturing protein-producing cells or bacteria such as E.coli, which are obtained by a genetic recombination technique or a cell fusion technique in a culture medium, an aqueous solution obtained from transgenic non-human animals, plants or insects, an aqueous solution obtained by cell-free protein synthesis, and an aqueous solution obtained by a chemical reaction of sugar, a sugar chain, or a water-soluble polymer with respect to the protein.

Examples of the protein-producing cell may include a transformed cell in which a gene encoding a protein of interest is integrated in a host cell, or the like.

Examples of the host cell may include cell lines of animal cells, plant cells, yeast cells, or insect cells or the like.

Specific examples of the host cell may include Chinese hamster ovary cells such as CHO cells, mouse myeloma cells such as NS0 cell and SP2/0 cell, rat myeloma cells such as YB2/0 cell and IR983F cell, Syrian hamster kidney-derived BHK cells, human myeloma cells such as Namalwa cell, embryo-stem cells, amphicytula, methanol-utilizing yeast (Pichia pastoris), and Sf9 cells or Sf21 derived from Spodoptera frugiperda.

A medium for culturing the protein-producing cells may be any medium, as long as it is suitable for culturing each of the cells, and examples of the medium for culturing animal cells may include typical media used for culturing animal cells. For example, any medium of a serum-containing medium, a medium containing no animal-derived component such as serum albumin or serum fraction, a serum-free medium or a protein-free medium may be used, and preferably, the serum-free medium or the protein-free medium is used.

Specifically, for example, as the medium, RPMI1640 medium [The Journal of the American Medical Association, 199,519 (1967)], Eagle MEM medium [Science,122,501(1952)], Dulbecco's modified MEM (DMEM) medium [Virology, 8,396 (1959)], 199 medium [Proceeding of the Society for the Biological Medicine,73,1(1950)], F12 medium [Proc. Natl. Acad. Sci. USA,53,288 (1965)], Iscove's Modified Dulbecco medium (IMDM medium) [J.Experimental Medicine, 147,923 (1978)], EX-CELL302 medium, EX-CELL-CD-CHO medium, and EX-CELL 325 medium (which are manufactured by SAFC bioscience Inc., EX-CELL is registered trademark), CD-CHO medium and CD DG44 medium (which are manufactured by Invitrogen Corp.) or IS CD-CHO medium (manufactured by Irvine Scientific Sales Co., Inc.), modified media thereof, mixed media thereof, concentrated media thereof or the like is used, and preferably RPMI1640 medium, DMEM medium, F12 medium, IMDM medium, EX-CELL302 medium, CD-CHO medium, or IS CD-CHO medium is used.

If necessary, physiologically active substances or nutrient factors essential for growth of the protein-producing cells may be added to the medium for culturing the protein-producing cells. These additives may be previously included in the medium prior to cultivation, or further properly supplied to the culture solution as an additive medium or an additive solution during cultivation. A method of additive supply may be performed in any state with single solution or two or more kinds of mixed solutions, and the addition method may be performed continuously or discontinuously.

The protein-producing transgenic non-human animals, plants or insects may be non-human animals, plants or insects in which the protein-encoding gene is integrated into their cells. Examples of the non-human animals may include mouse, rat, guinea pig, hamster, rabbit, dog, sheep, pig, goat, cattle or monkey. Examples of the plants may include tobacco, potato, tomato, carrot, soybean, brassica, alfalfa, rice, wheat, barley, corn or the like. Examples of the insects may include silkworm.

Examples of the method for producing the protein-containing aqueous solution may include those described in International Patent Publication No. 2008/120801, Japanese Publication No. Hei3-198792, International Patent Publication No. 2010/018847, International Patent Publication No. 2007/062245, International Patent Publication No. 2007/114496 or the like.

Further, in the present invention, examples of the protein-containing aqueous solution include an aqueous solution obtained by the process for purifying the protein of interest from the aqueous solutions, in addition to the aqueous solution obtained from the living body, the aqueous solution obtained from transgenic non-human animals, plants or insects, the aqueous solution obtained by cell-free protein synthesis, or the aqueous solution obtained by a chemical reaction of sugar, the sugar chain, or a water-soluble polymer with respect to the protein. Specific examples thereof may include a cell-free solution, a precipitate-free solution, an alcohol fraction, a salting-out fraction, a chromatography eluate or the like.

The cell-free solution may be a solution that is prepared by removing cells from the aqueous solution obtained from the living body, the aqueous solution formed of a culture broth, the aqueous solution obtained from transgenic non-human animals, plants or insects, the aqueous solution obtained by chemical reaction of sugar, a sugar chain, or a water-soluble polymer with respect to the protein, or an aqueous solution obtained in the process for purifying the protein of interest from these aqueous solutions.

Specific examples of the cell-free solution may include solutions that are obtained by removing cells by plain sedimentation using an aqueous solution including cells, centrifugation, ultrasonic waves, aqueous two-phase distribution, cross-flow filtration (Tangential flow filtration), filtration using a depth filter, filtration using a membrane filter such as microfiltration, dialysis, combinations thereof or the like.

Specific examples of the depth filter may include a Millistak+ HC depth filter, a Millistak+ DE depth filter, a Millistak+ CE depth filter, a Clarisolve depth filter (manufactured by Merck millipore Corp., Millistak is registered trademark), a SUPRA P depth filter (manufactured by Pall Corp.), a Sartoclear PB depth filter, a Sartoclear PC depth filter (manufactured by Sartorius Corp.), a Zeta plus SP depth filter, a Zeta plus AP depth filter, a Zeta plus LA depth filter, a Zeta plus-Delipid depth filter, a Zeta plus ZA depth filter, a Zeta plus EXT charged depth filter, and an Enphase AEX hybrid purifier (manufactured by Sumitomo 3M Ltd., Zeta plus is registered trademark), but are not limited thereto.

Specific examples of the membrane filter include an SHC film (manufactured by Merck millipore Corp.), an SHF film (manufactured by Merck millipore Corp.), Durapore (registered trademark) film (manufactured by Merck millipore Corp.), and Fluorodyne (registered trademark) (manufactured by Pall Corp.), but are not limited thereto.

The precipitate-free solution may be a solution that is prepared by performing flocculation or two-phase separation of the aqueous solution obtained from the living body, the aqueous solution formed of the culture broth, the aqueous solution obtained from transgenic non-human animals, plants or insects, the aqueous solution obtained by cell-free protein synthesis, the aqueous solution obtained by a chemical reaction of sugar, a sugar chain, or a water-soluble polymer with respect to the protein, or the aqueous solution obtained from the process for purifying the protein of interest from these aqueous solutions, by low-pH treatment or by addition of caprylic acid, an organic solvent, polyethylene glycol, a surfactant, a salt, an amino acid, a polymer or the like, and then by removing precipitates therefrom. Examples of the method for removing precipitates may include natural sedimentation, centrifugation, cross-flow filtration (Tangential flow filtration), filtration using a depth filter, filtration using a membrane filter, dialysis, combinations thereof or the like. In addition, a precipitate-free solution obtained by combining a plurality of methods for obtaining the precipitate-free solution is also disclosed.

The pH of the low-pH treatment is preferably pH 3 to 6 and more preferably pH 4 to 6, and adjusted by addition of an acid such as hydrochloric acid, acetic acid, citric acid, phosphoric acid or the like.

The alcohol fraction may be a fraction that is prepared by adding alcohol or the like to the aqueous solution obtained from the living body, the aqueous solution formed of the culture broth, the aqueous solution obtained from transgenic non-human animals, plants or insects, the aqueous solution obtained by cell-free protein synthesis, the aqueous solution obtained by a chemical reaction of sugar, a sugar chain, or a water-soluble polymer with respect to the protein, or the aqueous solution obtained from the process for purifying the protein of interest from these aqueous solutions. Specific examples thereof may include fractions obtained by low temperature ethanol fraction or the like.

The salting-out fraction may be a fraction that is prepared by adding a salt such as ammonium sulfate, sodium sulfate, sodium citrate, sodium chloride, potassium chloride or the like to the aqueous solution obtained from the living body, the aqueous solution formed of the culture broth, the aqueous solution obtained from transgenic non-human animals, plants or insects, the aqueous solution obtained by cell-free protein synthesis, the aqueous solution obtained by a chemical reaction of sugar, a sugar chain, or a water-soluble polymer with respect to the protein, or the aqueous solution obtained from the process for purifying the protein of interest from these aqueous solutions, so as to precipitate proteins.

The chromatography eluate may be a protein eluate that is prepared by adsorbing the aqueous solution obtained from the living body, the aqueous solution formed of the culture broth, the aqueous solution obtained from transgenic non-human animals, plants or insects, the aqueous solution obtained by cell-free protein synthesis, the aqueous solution obtained by a chemical reaction of sugar, a sugar chain, or a water-soluble polymer with respect to the protein, or the aqueous solution obtained from the process for purifying the protein of interest from these aqueous solutions, onto a resin or a membrane used in the chromatography so as to elute it using a proper elution solution, or by non-adsorbing it.

The resin or the membrane used in the chromatography may include an ion exchange resin, an ion exchange membrane, an affinity resin, a gel filtration resin, a hydrophobic interaction resin, a reverse phase resin, a hydroxyapatite resin, a fluoroapatite resin, a cellulose sulfate resin, an agarose sulfate resin, a multimodal resin or the like.

For example, the ion exchange resin or the ion exchange membrane may be a resin or a membrane that is prepared by directly or indirectly immobilizing a molecule having an ion exchange group, such as a sulfate group, a methyl sulfate group, a sulfophenyl group, a sulfonpropyl group, a carboxymethyl group, a quaternary ammonium group, a quaternary aminoethyl group, a diethylaminoethyl group or the like onto a base resin or a membrane, for example, a polymer or a derivative thereof (including crosslinked polymer) such as cellulose, sepharose, agarose, chitosan, an acrylic acid polymer or a styrene-divinyl benzene copolymer, a polymer consisting of silica particles, glass particles, ceramic particles, or surface-treated particles thereof.

Specific examples of the ion exchange resin or the ion exchange membrane may include Q Sepharose XL, Q Sepharose FF, DEAE Sepharose FF, ANX Sepharose FF, Capto Q, Capto DEAE, Capto Q ImpRes (which are manufactured by GE Healthcare Ltd., Inc., Sepharose is registered trademark), TOYOPEARL GigaCap Q-650, TOYOPEARL SuperQ-650, TOYOPEARL GigaCap S-650, TOYOPEARL GigaCap CM-650, TOYOPEARL NH$_2$-750F (which are manufactured by TOSOH Corp., TOYOPEARL is registered trademark), Fractogel DEAE, Fractogel TMAE, Fractogel TMAE Hicap, Eshmuno (registered trademark) Q (which are manufactured by Merck millipore Corp., Fractogel is registered trademark), Cellufine MAX-Q, Cellufine MAX-S (manufactured by JNC Corp., Cellufine is registered trademark), Mustang Q (manufactured by Pall Corp.), Sartobind Q, Sartobind STIC (which are manufactured by Sartorius Corp., Sartobind is registered trademark), SP Sepharose FF, CM Sepharose FF, SP Sepharose XL, Capto S (which are manufactured by GE Healthcare Ltd., Inc., Sepharose is registered trademark), Poros 50 HS, Poros 50 XS (which are manufactured by Applied Biosystems Inc., Poros is registered trademark), Eshmuno (registered trademark) S, Fractogel COO$^-$, Fractogel SO$_3^-$, Fractogel SE Hicap (which are manufactured by Merck millipore Corp. Fractogel is registered trademark), Mustang S (manufactured by Pall Corp.) or Sartobind (registered trademark) S (manufactured by Sartorius Corp.), DIAION PK, DIAION PA, DIAION CR, DIAION CR, DIAION AMP (which are manufactured by Mitsubishi Chemical Corp., DIAION is registered trademark), Eshmuno (registered trademark) CPX (manufactured by Merck millipore Corp.), or Qyu Speed (registered trademark) D (manufactured by Asahi Kasei Medical Co., Ltd.), but are not limited thereto.

The affinity resin may be a resin that is prepared by directly or indirectly immobilizing a molecule having an affinity for the protein of interest, for example, heparin, protein A, protein G protein L or a derivative thereof, onto the above base resin.

Specific examples of the affinity resin may include Heparin Sepharose (registered trademark) 6 Fast Flow (manufactured by GE Healthcare Ltd., Inc.), Procep-heparin (manufactured by Merck millipore Corp.), TOYOPEARL (registered trademark) AF-Heparin-650 (manufactured by TOSOH Corp.), Heparin HyperD (manufactured by Pall Corp.), MabSelect, Protein A Sepharose FF, MabSelect Xtra, MabSelect SuRe, MabSelect SuRe LX, Protein G Sepharose FF, Capto L (which are manufactured by GE Healthcare Ltd., Inc., Sepharose and MabSelect are registered trademark), Prosep vA Hicapacity, Prosep vA Ultra, Prosep Ultraplus (which are manufactured by Merck millipore Corp., Prosep is registered trademark), TOYOPEARL (registered trademark) AF-rProtein A-650F (manufactured by TOSOH Corp.), TOYOPEARL (registered trademark) AF-rProtein A HC-650F (manufactured by TOSOH Corp.), MabSpeed (registered trademark) RP101 (manufactured by Mitsubishi Chemical Corp.), MabSpeed (registered trademark) RP102( manufactured by Mitsubishi Chemical Corp.), JWT203 (manufactured by JSR), KanCap (registered trademark) A (manufactured by DescriptionKaneka Corporation), UNOsphere SUPrA manufactured by Bio-Rad Inc.), ADREPMA (registered trademark) A-20 (manufactured by Osaka Soda Co., Ltd.), ADREPMA (registered trademark) A-50 (manufactured by Osaka Soda Co., Ltd.), and ProSep (registered trademark)-vA High Capacity (manufactured by Merck millipore Corp.), but are not limited thereto.

Examples of the gel filtration resin may include a resin composed of a polymer consisting of dextran, allyl dextran, N,N'-methylenebisacrylamide, cellulose, agarose, styrene, divinylbenzene, polyvinyl alcohol, silica, chitosan or the like.

Specific examples of the gel filtration resin may include Sephacryl S series, Sepharose (registered trademark) series, Sephadex series, Superdex series, Sephacryl series (which are manufactured by GE Healthcare Ltd., Inc.), TOYOPEARL HW series, TSKgel PW series (which are manufactured by TOSOH Corp., TOYOPEARL is registered trademark), Bio gel Agarose, Bio gel P Polyacrylamide (which are manufactured by Bio-Rad Inc.), Cellufine GH, Cellufine GCL (which are manufactured by JNC Corp., Cellufine is registered trademark), Trisacryl GF05, Trisacryl GF2000, Ultrogel AcA (which are manufactured by Pall Corp., Trisacryl is registered trademark) or Fractogel BioSEC (manufactured by Merck millipore Corp.) or the like, but are not limited thereto.

The hydrophobic interaction resin may be a resin that is prepared by directly or indirectly immobilizing a hydrophobic molecule, for example, methyl group, ethyl group, propyl group, isopropyl group, butyl group, tert-butyl group, octyl group, ether group, phenyl group or the like onto the above base resin.

Specific examples of the hydrophobic interaction resin may include Phenyl Sepharose 6 Fast Flow (high-sub), Phenyl Sepharose 6 Fast Flow (low-sub), Octyl Sepharose 4 Fast Flow, Butyl Sepharose 4 Fast Flow (which are manufactured by GE Healthcare Ltd., Inc., Sepharose is registered trademark), TOYOPEARL Hexyl-650, TOYOPEARL Butyl-650, TOYOPEARL Phenyl-650, TOYOPEARL Ether-650, TOYOPEARL PPG-600, TOYOPEARL Butyl-600, TOYOPEARL Super Butyl-550 (which are manufactured by TOSOH Corp., TOYOPEARL is registered trademark), Mactro-Prep t-Butyl, Macro-Prep Methyl (which are manufactured by Bio-Rad Inc., Mactro-Prep is registered trademark), QMA Spherosil (registered trademark), Methyl Ceramic HyperD (registered trademark) (which are manufactured by Pall Corp.), Fractogel Phenyl(S), Fractogel Propyl(S) (which are manufactured by Merck millipore Corp., Fractogel is registered trademark), phenyl-Cellufine (manufactured by JNC Corp., Cellufine is registered trademark), DIAION HP, DIAION SP (which are manufactured by Mitsubishi Chemical Corp., DIAION is registered trademark), butylated Chitopearl, phenylated Chitopearl (which are manufactured by FUJIBO Holdings, Inc., Chitopearl is registered trademark) or the like, but are not limited thereto.

The reverse phase resin may be, for example, a resin that is prepared by directly or indirectly immobilizing a hydrocarbon group onto a solid-phase matrix. Examples of the hydrocarbon group may include trimethyl group, butyl group, phenyl group, octyl group, octadecyl group, terminus-modified functional group thereof or the like. Specific examples thereof may include RESOURCE (registered trademark) RPC series, SOURCE (trademark) RPC series (which are manufactured by GE Healthcare Ltd., Inc.) or the like, but are not limited thereto.

Examples of the hydroxyapatite resin may include CHT (trademark) Ceramic Hydroxyapatite Type I, Type II (which are manufactured by Bio-Rad Inc.) or the like, but are not limited thereto. In addition, examples of the fluoroapatite resin may include CFT (trademark) Ceramic Fluoroapatite (manufactured by Bio-Rad Inc.) or the like, but are not limited thereto.

Examples of the cellulose sulfate resin or the agarose sulfate resin may include Cellufine sulfate, Cellufine sulfate m, Cellufine sulfate c, Cellulofine sulfate m, Cellulofine sulfate c, Cellufine sulfate m or Cellufine sulfate c (which are manufactured by JNC Corp., Cellufine is registered trademark), Capto (registered trademark) DeVirS (manufactured by GE Healthcare Ltd., Inc.) or the like, but are not limited thereto.

For example, the multimodal resin may be a resin that is prepared by directly or indirectly immobilizing two or more types of functional groups having different selectivity, preferably, the above ion exchange group and the above hydrophobic interaction group, onto the above base resin.

Specific examples of the multimodal resin may include Capto adhere, Capto MMC (which are manufactured by GE Healthcare Ltd., Inc., Capto is registered trademark), HEA HyperCel, PPA HyperCel, MEP HyperCel (which are manufactured by Pall Corp., HyperCel is trademark), TOYOPEARL (registered trademark) MX-Trp-650M (manufactured by TOSOH Corp.) or the like, but are not limited thereto.

The chromatography may be carried out in an adsorption mode or in a non-adsorption mode, depending on the purpose. Preferably, at least one of the chromatography is carried out in the adsorption mode.

The adsorption mode in the chromatography means that an aqueous solution provided in the chromatography is contacted with the corresponding resin or membrane, the protein of interest is adsorbed onto the corresponding resin or membrane, if necessary, washing is performed, and then the protein of interest is eluted using a buffer of which pH, conductivity, buffer components, salt concentration or additive or the like is altered, thereby recovering the adsorption fraction including the protein of interest.

The non-adsorption mode in the chromatography means that an aqueous solution provided in the chromatography is contacted with the corresponding resin or membrane, the protein of interest is not adsorbed onto the corresponding resin or membrane, thereby recovering the non-adsorption fraction including the protein of interest.

The conditions of the aqueous solution provided in the chromatography and the buffer used in washing or elution are properly selected with respect to the pH, conductivity, buffer components, salt concentration, additives or the like. In the selection of the chromatographic conditions, differences in the physicochemical characteristics between the protein of interest and the compounds desired to be separated, for example, differences in isoelectric point, charge, hydrophobicity, molecular size, or steric structure or the like may be utilized.

The elution method of the adsorption mode may include a one step elution method of using a buffer having a specific salt concentration or pH to reduce affinity between the protein of interest and the resin, a stepwise method of eluting the protein of interest by changing the salt concentration or pH in a stepwise manner, or a gradient method of eluting the protein of interest by continuously changing the salt concentration or pH.

Examples of the salt constituting the buffer may include phosphate, citrate, acetate, succinate, maleate, borate, Tris(base), HEPES, MES, PIPES, MOPS, TES, Tricine or the like.

These salts may be used in combinations with other salts, for example, sodium chloride, potassium chloride, calcium chloride, sodium citrate, sodium sulfate or ammonium sulfate. The buffer components, for example, amino acids such as glycine, alanine, arginine, serine, threonine, glutamic acid, aspartic acid or histidine or the like, sugars such as glucose, sucrose, lactose, sialic acid or the like, or derivatives thereof or the like may be used in combinations.

In the present invention, in a case where the protein is an antibody, the protein-containing aqueous solution may be preferably a protein-containing aqueous solution that is obtained without using affinity chromatography, and more preferably, a protein-containing aqueous solution that is obtained without using protein A affinity chromatography.

Further, if insoluble materials such as particles or the like are present in the protein-containing aqueous solution, they are removed in advance, and the resulting insoluble-free solution may be provided in the purification method of the present invention. Examples of the method of removing insoluble materials such as particles may include centrifugation, cross-flow filtration (Tangential flow filtration), filtration using a depth filter, filtration using a membrane filter, dialysis, or combinations thereof.

The activated carbon pretreatment solution provided for the method for purifying a protein using an activated carbon of the present invention can be prepared by adjusting the conductivity by performing dilution, concentration dilution, buffer exchange of the protein-containing aqueous solution described above or addition of salt such as sodium chloride.

The present inventors found that the protein of interest with a significantly decreased content of impurities can be obtained at a high recovery rate, by optimally preparing the activated carbon pretreatment solution or carrying out the activated carbon treatment using the activated carbon pretreatment solution under the optimal conditions.

The conductivity means ability of the aqueous solution of transferring a current between two electrodes. In the solution, the current flows by ion transport. Accordingly, in a case where the amount of ions present in the activated carbon pretreatment solution increases, a high conductivity is obtained. By decreasing the amount of ions present in the activated carbon pretreatment solution to reduce the conductivity, it is possible to efficiently separate impurities, reduce the amount of proteins of interest adsorbed to the activated carbon, and improve the recovery rate. The preferable conductivity of the activated carbon pretreatment solution is 0 to 2 mS/cm, and particularly preferably 0 to 1 mS/cm. The conductivity can be measured using a commercially available conductivity meter.

As the method for purifying the activated carbon pretreatment solution by diluting the protein-containing aqueous solution, a method for adjusting the conductivity to the preferable conductivity, of diluting the protein-containing aqueous solution with water such as ultrapure water (milli-Q water) or 0.05% polysorbate 80, 10 mmol/L Sodium L-glutamate, or 262 mmol/L, D-sorbitol (pH 5.5) buffer having conductivity less than 1 mS/cm is used. The dilution ratio is, for example, 2 to 20 times.

As the method for preparing the activated carbon pretreatment solution by concentration dilution or buffer exchange of the protein-containing aqueous solution, ultrafiltration using an ultrafiltration membrane, precipitation, a method of performing an operation of diluting the protein-containing aqueous solution with ultrapure water, phosphate, citrate, acetate, succinate, maleate, borate, Tris(base), HEPES, MES, PIPES, MOPS, TES, or Tricine after concentration using a dialysis membrane or a gel filtration column once or plural times, a method of exchanging the protein-containing aqueous solution using dialysis filtration using a ultrafiltration membrane, or a method of exchanging the protein-containing aqueous solution with ultrapure water or buffer using dialysis filtration after concentration of the protein-containing aqueous solution by the ultrafiltration using a ultrafiltration membrane is used. In addition, a method for preparing the activated carbon pretreatment solution by causing the protein of interest to be adsorbed to the adsorption column, and diluting with ultrapure water or buffer to perform concentration dilution or buffer exchange is used.

The concentration rate is, for example, 5 to 200 times and the dilution rate is, for example, 2 to 100 times. The volume of the ultrapure water or buffer in a case of using the dialysis filtration is, for example, 2 to 30 times. Theses may be combined randomly for the preparation.

The ultrafiltration membrane includes a positively or negatively charged ultrafiltration membrane, in addition to the typical ultrafiltration membranes, and specific examples thereof may include Pellicon 3 Ultracel membrane, Pellicon 3 biomax membrane, Pellicon 2 Ultracel membrane, Pellicon 2 biomax membrane (which are manufactured by Merck millipore Corp., Pellicon is registered trademark), omega membrane (manufactured by Pall Corp), Kvick membrane (manufactured by GE Healthcare Ltd., Inc.), Sartocon Cassette (manufactured by Sartorius Stedim Japan K.K.), and PROPOR TFF (manufactured by Parker Hannifin Corporation), but are not limited thereto.

The activated carbon pretreatment solution can be prepared by adjusting the conductivity by adding salt such as sodium chloride to the protein-containing aqueous solution. Examples of salt to be added include sodium chloride, potassium chloride, calcium chloride, sodium citrate, sodium sulfate, and ammonium sulfate, and sodium chloride is preferable. These salts may be used alone or in combination thereof. A final salt concentration of the prepared activated carbon pretreatment solution is preferably 0 to 10 mmol/L and more preferably 0 to 5 mmol/L.

Examples of the buffer component constituting the activated carbon pretreatment solution include phosphate, citrate, acetate, succinate, maleate, borate, Tris(base), HEPES, MES, PIPES, MOPS, TES, Tricine, and amino acids such as glycine, alanine, arginine, serine, threonine, glutamic acid, aspartic acid or histidine. The concentration of these is preferably 0.01 mol/L to 0.5 mol/L.

The pH of the activated carbon pretreatment solution is preferably 2 to 9 and more preferably 3 to 8. Particularly, in a case where the protein is an antibody, the pH of the activated carbon pretreatment solution is preferably 2 to 8, more preferably 3 to 7, particularly preferably 4 to 6, and most preferably 4 to 5.

The protein of interest is an antibody, the concentration thereof is 5.5 to 23 mg/mL, and most preferably 11 to 23 mg/mL.

In the present invention, for example, the impurities may include host cell proteins (HCP), protein-derived polymers (HMWs), protein-derived degradation products (LMWs), protein-derived modification products resulting from denaturation, removal of sugar chain components, oxidation, deamidation or the like, DNAs, viruses, medium-derived components, culture additives or enzymes secreted from host cells, and preferably host cell proteins, protein-derived polymers, protein-derived degradation products DNAs and/or viruses. More preferably, the host cell proteins (HCP), protein-derived polymers (HMWs), protein-derived degradation products (LMWs), DNAs, and viruses can be removed at the same time.

Examples of the enzymes secreted from host cells may include glycolytic enzymes, proteolytic enzymes, oxidation/reduction enzymes, or amino acid isomerization enzymes.

Specific examples of the glycolytic enzymes may include neuraminidase (sialidase), galactosidase, glycanase or the like. Specific examples of the proteolytic enzymes may include serine protease, esterase, cysteine protease, trypsin-like protease, aminopeptidase, aspartic protease, cathepsin or the like.

Specific examples of the oxidation/reduction enzymes may include thioredoxin-related enzymes such as thioredoxin reductase or the like. Specific examples of the amino acid isomerizing enzyme may include transglutaminase or the like.

Examples of viruses may include a retrovirus, a parvovirus, a reovirus, and a herpesvirus, and specific examples thereof include a murine lymphoma virus, a mouse parvovirus, an adenovirus, a cytomegalovirus, a herpes simplex virus, an influenza virus, and a vaccinia virus.

The activated carbon used in the purification method of the present invention may be any one, as long as it is suitable for the drug preparation, and one type of activated carbon may be used alone, or two or more types of activated carbon may be used alone or in combination.

Examples of the activated carbon may include mineral-based activated carbon, plant-based activated carbon or the like. Specific examples of the mineral-based activated carbon may include coal-based activated carbon, petroleum-based activated carbon or the like. Specific examples of the plant-based activated carbon may include wood-based activated carbon, coconut-shell-based activated carbon or the like, and preferably wood-based activated carbon.

The raw material of the activated carbon may be any one, as long as it is carbonaceous, and examples thereof may include wood materials such as sawdust, charcoal, ash, peat moss, peat or wood chip, coconut-shell, coals such as lignite, brown coal or anthracite, coal pitch, petroleum pitch, oil carbon, rayon, acrylonitrile or phenol resin or the like.

The preparation method of the activated carbon is not particularly limited, but examples thereof may include a chemical solution activation method of adding and penetrating a chemical such as zinc chloride or phosphoric acid at a high temperature and performing carbonization at a high temperature, or a gas activation method of reacting carbonized raw materials and gas such as water vapor, carbon dioxide, air or combustion gas at a high temperature. Preferable examples thereof may include a zinc chloride activation method, an acid activation method using phosphoric acid, or a water vapor activation method.

The form of the activated carbon may be any one, as long as it is suitable for the drug preparation, and examples thereof may include a particle form of activated carbon, such as pulverized carbon, granular carbon, spherical carbon, pellet carbon or the like, a fibrous form of activated carbon such as fiber, cross fiber or the like, a specialized form of activated carbon such as a sheet form, a compact, a honeycomb shape or the like, powder activated carbon or the like.

The positively or negatively charged activated carbon or activated carbon modified with a surface modifier such as polyhydroxyethylmethacrylate (PHEMA), heparin, cellulose, polyurethane or the like may be also included in the activated carbon used in the purification method of the present invention. Further, carbon gel prepared by a sol-gel method is included in the activated carbon used in the purification method of the present invention. Examples of a raw material used in the sol-gel method may include phenol, melamine, resorcinol, or formaldehyde.

An average micropore diameter of the activated carbon may be, but is not particularly limited to, typically 0.1 to 20 nm, preferably 0.5 to 5.0 nm, more preferably 2.0 to 5.0 nm, and even more preferably 3.0 to 5.0 nm. The average micropore diameter of the activated carbon can be calculated using a BJH method with a nitrogen adsorption isothermal adsorption curve.

Specific examples of the activated carbon may include Carboraffin, KYORYOKU SHIRASAGI, purified SHIRASAGI, TOKUSEI SHIRASAGI, SHIRASAGI A, SHIRASAGI C, SHIRASAGI C-1, SHIRASAGI DO-2, SHIRASAGI DO-5, SHIRASAGI DO-11, SHIRASAGI DC, SHIRASAGI DO, SHIRASAGI Gx, SHIRASAGI G, SHIRASAGI GH, SHIRASAGI FAC-10, SHIRASAGI FPG-1, SHIRASAGI M, SHIRASAGI P, SHIRASAGI PHC, SHIRASAGI Gc, SHIRASAGI GH, SHIRASAGI GM, SHIRASAGI GS, SHIRASAGI GT, SHIRASAGI GAA, SHIRASAGI GOC, SHIRASAGI GOX, SHIRASAGI APRC, SHIRASAGI TAC, SHIRASAGI MAC, SHIRASAGI XRC, SHIRASAGI NCC, SHIRASAGI SRCX, SHIRASAGI Wc, SHIRASAGI LGK, SHIRASAGI KL, SHIRASAGI WH, SHIRASAGI W, SHIRASAGI WHA, SHIRASAGI LH, SHIRASAGI KL, SHIRASAGI LGK, SHIRASAGI MAC-W, SHIRASAGI S, SHIRASAGI Sx, SHIRASAGI X2M, SHIRASAGI X7000, SHIRASAGI X7100, SHIRASAGI DX7-3, MOLSIEVON (which are manufactured by Japan EnviroChemicals, Ltd., SHIRASAGI is registered trademark), ACF, GLC (manufactured by KURARAY CHEMICAL CO., Ltd.), TAIKO A, TAIKO S, TAIKO K, TAIKO KA, TAIKO Q, TAIKO Y (which are manufactured by FUTAMURA Chemical CO., Ltd.), Norit GSP, Norit CNI, Norit GBG, Norit TEST EUR, Norit SUPRA EUR, GAC, CN, CG, CAP/CGP, SX, CA (which are manufactured by Norit Japan Co., Ltd., Norit is registered trademark) or the like, but are not limited thereto.

Among these, examples of the activated carbon from wood may include TOKUSEI SHIRASAGI, KYORYOKU SHIRASAGI, SHIRASAGI P, SHIRASAGI C, SHIRASAGI A (which are manufactured by Japan EnviroChemicals, Ltd., SHIRASAGI is registered trademark), TAIKO Y, TAIKO KA, TAIKO M, TAIKO A (which are manufactured by FUTAMURA Chemical CO., Ltd., TAIKO is registered trademark), Norit GSP, or Norit CNI, (which are manufactured by Norit Japan Co., Ltd., Norit is registered trademark) or the like.

Examples of the means of the purification method using the activated carbon of the present invention may include, but are not particularly limited to, a batch method, a membrane treatment method, column chromatography or the like. Depending on each means, a suitable form of the activated carbon is selected. If necessary, a particle form or the like prepared by encapsulating the activated carbon in a porous polymer or a gel, a membrane or cartridge form or the like prepared by adsorbing, fixing or molding the activated carbon using a resin such as polypropylene or cellulose, or fiber or the like.

Specific examples of a membrane or a cartridge including the activated carbon may include a CUNO activated carbon filter cartridge, a Zeta plus activated carbon filter cartridge (manufactured by Sumitomo 3M Ltd., CUNO and Zeta plus are registered trademarks), a Millistak+ activated carbon filter (manufactured by Merck millipore Corp., Millistak is registered trademark), a SUPRA AKS1 filter, a AKS1 filter, a Stax (trademark) AKS1 (which are manufactured by Pall Corp.), Adol (manufactured by UNITIKA Ltd.), a K filter (registered trademark), an activated carbon sheet (which are manufactured by TOYOBO CO., Ltd), Hemax (manufactured by KURARAY Co., Ltd.), Hemosorba (registered trademark) (manufactured by Asahi Kasei Medical Co., Ltd.), Hemocolumn (manufactured by TERUMO Corp.), Hecellose (manufactured by TEIJIN Ltd.) or the like, but are not limited thereto. Among these, examples of a membrane or a cartridge including the activated carbon from wood may include a Zeta plus activated carbon filter cartridge (manufactured by Sumitomo 3M Ltd., Zeta plus is a registered trademark), a SUPRA AKS1 filter, a AKS1 filter, or a Stax (trademark) AKS1 (which are manufactured by Pall Corp.).

Depending on the protein of interest and the means of the purification method, packing density, granularity, rigidity, drying loss, residue on ignition, specific surface area, or pore volume, of the used activated carbon may be properly selected.

The purification method using the activated carbon of the present invention is carried out in a non-adsorption mode. The non-adsorption mode means that the protein-containing aqueous solution is contacted with the activated carbon, and the protein of interest is not adsorbed onto the activated carbon to recover a non-adsorption fraction. In detail, the load volume to the activated carbon, the contact time, and the like of the activated carbon pretreatment solution having an adjusted conductivity are adjusted in advance, and then, contacted with the activated carbon. Thus, the protein of interest is not adsorbed onto the activated carbon, but impurities are only adsorbed onto the activated carbon, thereby recovering the non-adsorption fraction having the protein with a low content of impurities at a high recovery rate.

Regarding the load volume and the contact time of the activated carbon pretreatment solution with the adjusted conductivity by the method in advance with respect to the activated carbon, in a case of contact with the activated carbon, in a case where the load volume is 0.1 to 0.3 mg protein/mg activated carbon, the contact time is preferably 8 to 24 hours and more preferably 12 to 24 hours. In addition, in a case where the load volume is 0.05 to 0.15 mg protein/mg activated carbon, the contact time is preferably 0.1 to 24 hours, more preferably 2 to 24 hours, and particularly preferably 2 to 12 hours.

In addition, according to the amount of activated carbon necessary for the purification method using the activated carbon and the amount of the activated carbon included per one membrane or one cartridge including the plurality of activated carbon or one column filled with the activated carbon, the membrane or cartridge including the activated carbon or the column filled with the activated carbon can be divided into a plurality of them, and they can be suitably combined. For example, the plurality of the membrane or cartridge including activated carbon or the column filled with the activated carbon are connected in series or in parallel, and the activated carbon pretreatment solution is caused to pass through.

Particularly, in a case where the plurality of the membrane or cartridge including activated carbon or the column filled with the activated carbon are connected in series and the activated carbon pretreatment solution is caused to pass through continuously, as the number of the membrane including the plurality of activated carbon, the cartridge, or the column filled with the activated carbon increases, the impurities can be more effectively removed. In this case, the number of the membrane or cartridge including the plurality of activated carbon or the column filled with the activated carbon is preferably at least 2 or more, more preferably 2 to 20, even more preferably 2 to 10, and particularly preferably 2 to 4.

In a case of performing the purification by the membrane treatment or the column chromatography, for example, the activated carbon pretreatment solution may be caused to pass once through the activated carbon film, the activated carbon cartridge, or the membrane, cartridge, or column filled with the activated carbon. However, a method of continuously circulating the activated carbon pretreatment solution and bringing the activated carbon pretreatment solution into contact with the activated carbon plural times is preferable.

In addition, a flow rate, a viscosity, and the like of the activated carbon pretreatment solution in the purification method using the activated carbon can also be suitably selected.

In the present invention, the present invention for obtaining the protein of interest with the significantly decreased content of impurities at a high recovery rate can be completed, by optimally preparing the activated carbon pretreatment solution and carrying out the activated carbon treatment using the activated carbon pretreatment solution under the optimal conditions.

In detail, with respect to the content of the impurities, the content (ng/mg-P) of the host cell proteins is preferably 100 ng or less per 1 mg of the protein and more preferably 10 ng or less per 1 mg of the protein. The content of the host cell proteins may be determined by ELISA (Enzyme-Linked Immunosorbent Assay), Western blotting, an electrochemiluminescence assay or the like.

The content of the protein-derived polymers is preferably 2% or less and more preferably 1% or less. The content of the protein-derived degradation products is preferably 2% or less and more preferably 1% or less. The content of the protein-derived polymers or the protein-derived degradation products may be determined by gel filtration HPLC, ion exchange HPLC, polyacrylamide gel electrophoresis, a light scattering method, an ultracentrifugal method or the like. The DNA concentration can be, for example, determined by an analysis method such as Pico-green, Threshold, QPCR or the like.

A Logarithm Reduction Factor (LRF) showing a virus removal rate is preferably 3 or more and more preferably 3.5 or more. A virus tilter can be, for example, measured by measurement using infectivity or quantitative Polymerase Cain Reaction (qPCR).

The virus removal rate can be measured by the following calculation equation.

$$\mathrm{LRF} = \{\log 10(V1) + \log 10(T1)\} - \{\log 10(V2) + \log 10(T2)\}$$

V1: amount of added solution
V2: amount of recovery solution
T1: virus tilter of added solution
T2: virus tilter of recovery solution

In the present invention, the protein of interest with the decreased content of impurities can be measured at a recovery ratio or the recovery rate. In the present specification, the recovery rate is also referred to as a yield.

The recovery ratio of the protein of interest is preferably 0.80 or more, more preferably 0.85 or more, and even more preferably 0.90 or more, The recovery rate of the protein of interest is preferably 70% or more, more preferably 80% or more, and even more preferably 90% or more. The recovery ratio or the recovery rate of the protein of interest can be, for example, measured by absorbance or affinity HPLC such as protein A.

In the present invention, for example, the recovery ratio of the protein of interest can be calculated by the following calculation equation.

Recovery ratio ($C/C_o$) = concentration of protein of interest after the activated carbon treatment (C)/concentration of protein of interest before the activated carbon treatment ($C_o$)

In addition, the recovery rate of the protein of interest can be, for example, calculated by the following calculation equation.

Recovery rate = (concentration of protein of interest after the activated carbon treatment $\times$ amount of recovery solution/concentration of protein of interest before the activated carbon treatment $\times$ amount of solution before the activated carbon treatment) $\times$ 100

The present invention relates to a method for purifying a protein as claimed, comprising the step of separating the protein of interest from impurities using the activated carbon to obtain the protein of interest with the decreased content of impurities.

In the preparation method of the protein of the present invention, any of other purification method to be used in combination with the purification method as claimed using the activated carbon may be used, as long as it is a method suitable for the preparation of drugs, for example, the ion exchange membrane used for preparing the protein-containing aqueous solution (for example, anion exchange membrane) chromatography (for example, anion exchange membrane, cation exchange chromatography, and multimodal chromatography), alcohol fraction, removal of precipitates, salting out, buffer exchange, concentration, dilution, filtration, virus inactivation, virus removal or the like can be used. The other purifications method to be used in combination with the purification method using the activated carbon may be used in combinations of a plurality of types and numbers thereof, and at least one selected from anion exchange membrane, anion exchange chromatography, cation exchange chromatography, and multimodal chromatography is preferably used.

In a case where the protein in the present invention is an antibody, the chromatography to be used in combination with the purification method using the activated carbon may be preferably a preparation method comprising no affinity chromatography, and more preferably a preparation method comprising no protein A affinity chromatography. If the protein is an antibody, examples of the chromatography to be used in combination with the activated carbon may include ion exchange chromatography, multimodal chromatography or combinations thereof.

As the method for preparing the protein of the present invention, for example, in a case where the protein in the present invention is an antibody, the preparation method using the activated carbon as claimed is carried out, and subsequently, the non-adsorption mode-anion exchange chromatography is carried out, followed by the adsorption mode-cation exchange chromatography, or the preparation method using the activated carbon is carried out, and subsequently, the adsorption mode-cation exchange chromatography is carried out, followed by non-adsorption mode-anion exchange chromatography.

More preferably, all the chromatographies to be used in combination with the purification method using the activated carbon may be, for example, a protein purification method that is carried out in the non-adsorption mode (All negative chromatography). Specifically, for example, in a case where the protein is an antibody, the purification method using the activated carbon is carried out, and subsequently, the preparation method of performing non-adsorption mode-anion exchange chromatography or chromatography using an anion exchange membrane without using a column is used.

Examples

Hereinafter, the present invention will be described more specifically with reference to examples, but the present invention is not limited to these examples. In the examples, content of the protein-derived polymer (hereinafter, also abbreviated as the polymer content) shows a rate of peaks detected on the side of a high molecular weight from the protein of interest by gel filtration HPLC analysis. The content of the protein-derived degradation product (hereinafter, also abbreviated as the degradation product content) shows a rate of peaks detected on the side of a low molecular weight from the protein of interest by gel filtration HPLC analysis.

Example 1 Examination for Amount of Treated Activated Carbon Using Monoclonal Antibodies (Mab A, C, D, or E)

A CHO cell culture broth containing monoclonal antibodies [Mab A, C, D (which are IgG1), or E (IgG4)] was subjected to centrifugation (2,900 g, 10 minutes) and then membrane filter filtration (0.22 $\mu$m filter), and accordingly, the cell was removed, thereby preparing each CHO cell culture supernatant.

Then, each CHO cell culture supernatant was adjusted to pH 4.5 with acid or alkali. After leaving for 1 hour, the formed precipitates were removed by centrifugation (2,900 g, 10 minutes) and then membrane filter filtration (0.22 $\mu$m filter), and each pH-adjusted clarified solution was obtained.

Then, approximately 5 mL of each obtained pH-adjusted clarified solution was moved into 15 mL tube, and 25, 50, 100, and 200 mg of the activated carbon [SHIRASAGI (registered trademark) P: manufactured by Japan Enviro-Chemicals, Ltd.] were respectively added and mixed. The mixed solution was stirred with a rotator at room temperature for 17 hours, the activated carbon was removed by centrifugation (2,900 g, 10 minutes) and then membrane filter filtration (0.22 $\mu$m filter), and each activated carbon-treated solution was obtained.

The concentration of the host cell protein of the each obtained activated carbon-treated solution was analyzed by an ELISA method. FIG. 1 shows a relationship between the activated carbon addition amount and the concentration of host cell protein. As shown in FIG. 1, it was clear that, even in a case where any pH-adjusted clarified solution was treated, the concentration of the host cell protein changes in accordance with the amount of the activated carbon. It was clear that, the inclination relationship therebetween varies depending on each pH-adjusted clarified solution used, but the inclination has a significantly strong correlation with the natural logarithm of the concentration of the host cell protein.

As described above, it was determined that, as the activated carbon addition amount increases, the host cell protein can be rapidly removed.

FIG. 2 shows a result obtained by analyzing the content of each degradation product of the activated carbon eluate by gel filtration HPLC. As shown in FIG. 2, it was confirmed that the degradation product can be effectively removed from any activated carbon-treated solution in accordance with the activated carbon addition amount.

The antibody concentration of each activated carbon-treated solution was analyzed by protein A affinity HPLC. FIG. 3 shows a relationship between the activated carbon addition and the antibody concentration. As shown in FIG. 3, it was clear that, in a case where any pH-adjusted clarified solution was treated, the antibody concentration decreased in accordance with the amount of activated carbon, and the relationship therebetween has a significantly high correlation. The inclination thereof is the same, in a case of using the pH-adjusted clarified solution including Mab A, Mab C, and Mab D in which the subclass of the antibody is IgG1, and the inclination showed a large negative value, in a case of Mab E in which the subclass is IgG4.

From the results shown in FIGS. 1 to 3, increasing of the addition amount of the activated carbon is effective, in order to significantly remove impurities using the activated carbon, and it was confirmed that, since the adsorption amount of the antibody increases in accordance with an increase in the amount of the activated carbon, it was necessary to find the activated carbon treatment conditions for improving the efficiency of the removal of impurities and improving the recovery rate.

Example 2 Dilution Result of Activated Carbon Pretreatment Solution

The CHO cell culture broth containing monoclonal antibodies (Mab A) was subjected to microfiltration, and the cell was removed, thereby adjusting the CHO cell culture supernatant to pH 4.5 with acid or alkali. After leaving for 1 hour, the formed precipitates were removed by centrifugation (2,900 g, 10 minutes) and then membrane filter filtration (0.22 $\mu$m filter), and a pH-adjusted clarified solution was obtained.

The activated carbon [SHIRASAGI (registered trademark) P: manufactured by Japan EnviroChemicals, Ltd.] was added to approximately 5 mL of the pH-adjusted clarified solution or 2-fold dilute solution obtained by adding approximately 5 mL of buffer to approximately 5 mL of the obtained pH-adjusted clarified solution, so that the load volume becomes 0.1 to 0.5 mg antibody/mg activated carbon, and stirred with a rotator at room temperature for 17 hours, and the activated carbon was removed by centrifugation (2,900 g, 10 minutes) and then membrane filter filtration (0.22 $\mu$m filter), thereby obtaining each activated carbon-treated solution.

For the buffer for dilution, 0.05% polysorbate 80, 10 mmol/L Sodium L-glutamate, 262 mmol/L, D-sorbitol (pH 5.5) having conductivity less than 1 mS/cm were used. As a control, the pH-adjusted clarified solution without addition of the activated carbon, or the 2-fold dilute solution of the pH-adjusted clarified solution was used.

The antibody concentration of the activated carbon-treated solution having each load volume was analyzed by protein A affinity HPLC, and the recovery ratio ($C/C_0$) was shown in FIG. 4. As shown in FIG. 4, as a result of an increase in amount of the activated carbon a decrease in load volume, the recovery ratio was decreased. The recovery ratio was not greatly affected by the dilution of the pH-adjusted clarified solution, it was confirmed that a slightly high value is shown with the low load volume due to the dilution.

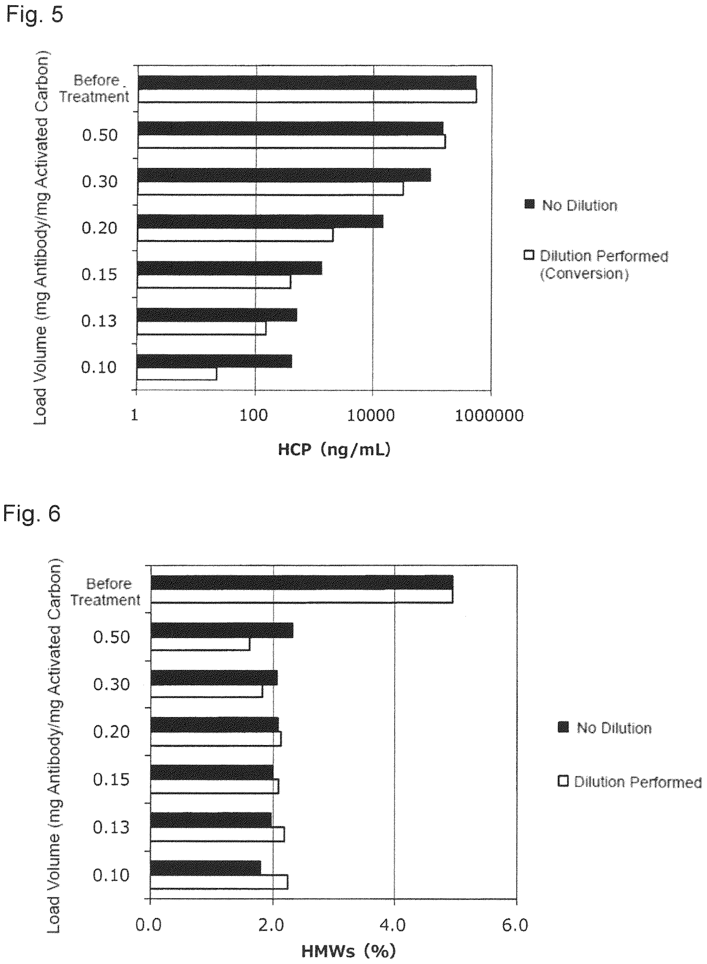

FIG. 5 shows a result obtained by analyzing the concentration of the host cell protein (HCP) of the activated carbon-treated solution of each load volume by the ELISA method. The diluted sample was shown with a value based on the dilution ratio. As shown in FIG. 5, it was clear that the host cell protein is removed by diluting the pH-adjusted clarified solution with the low load volume.

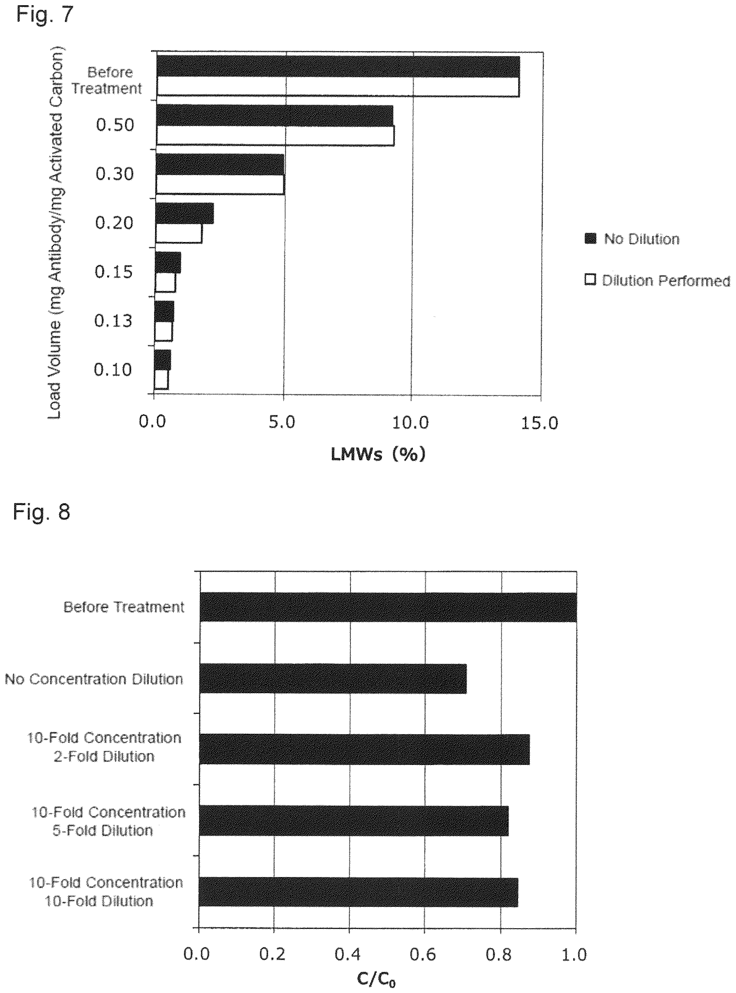

FIGS. 6 and 7 respectively show results obtained by analyzing the content of the polymer (HMWs) of the activated carbon eluate of each load volume and the content of the degradation products (LMWs) by gel filtration HPLC. As shown in FIGS. 6 and 7, the content of the polymer and the content of the degradation products were not greatly fluctuated depending on the dilution.

From the result described above, by preparing the activated carbon pretreatment solution with diluted clarified solution, the effect of the removal of various impurities was further improved in a case where the amount of activated carbon was increased and the load volume was decreased, and the recovery ratio was equivalently or slightly improved. The load volume most satisfying both of the recovery ratio and the effect of the removal of various impurities was 0.15 to 0.20 mg antibody/mg activated carbon.

Example 3 Effect of Concentration Dilution of Activated Carbon Pretreatment Solution

A CHO cell culture broth containing the monoclonal antibodies (Mab A) was subjected to continuous centrifugation, and the cell was removed using a depth membrane (manufactured by Merck millipore Corp.: A1HC). The cell-free solution was adjusted to pH 4.5 through an MF membrane [manufactured by Pall Corp.: Fluorodyne (registered trademark)] with acid or alkali. After leaving at 4°C for 6 hours, the formed precipitates were removed with an SHC membrane (manufactured by Merck millipore Corp.). The obtained pH-adjusted clarified solution was subjected to 10-fold concentration with a UF membrane [manufactured by Merck millipore Corp.: Pellicon (registered trademark) 3 30kD], and the concentrated dilute diluted 2-fold, 5-fold, or 10-fold using milli-Q water was obtained.

The activated carbon (TOKUSEI SHIRASAGI: manufactured by Japan EnviroChemicals, Ltd., SHIRASAGI is registered trademark) was added to approximately 5mL of each concentrated dilute so as to obtain the load volume of 0.2 mg antibody/mg activated carbon, stirred with a rotator at room temperature for 17 hours, and subjected to centrifugation (2,900 g, 10 minutes) and then membrane filter filtration (0.22 $\mu$m filter), and the activated carbon-treated solution of each concentrated dilute with the removed activated carbon was obtained.

As a control, each concentrated dilute, to which the activated carbon was not added, was used. The antibody concentration of the activated carbon-treated solution and the control of the each obtained concentrated dilute was analyzed by protein A affinity HPLC, and the recovery ratio $((C/C_0)$ was shown in FIG. 8. As shown in FIG. 8, it was confirmed that the recovery ratio was improved due to the concentration dilution of the pH-adjusted clarified solution.

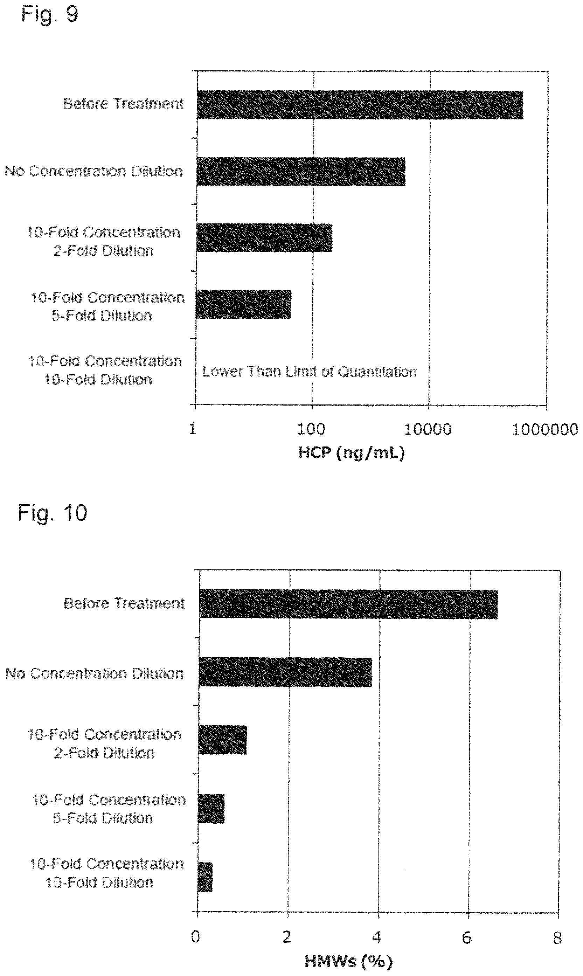

FIG. 9 shows a result obtained by analyzing the concentration of the host cell protein (HCP) of the activated carbon-treated solution of each concentrated dilute by the ELISA method. As shown in FIG. 9, it was clear that the concentration of the host cell protein was decreased due to the concentration dilution of the pH-adjusted clarified solution, and the HCP could be efficiently removed by increasing a dilution rate.

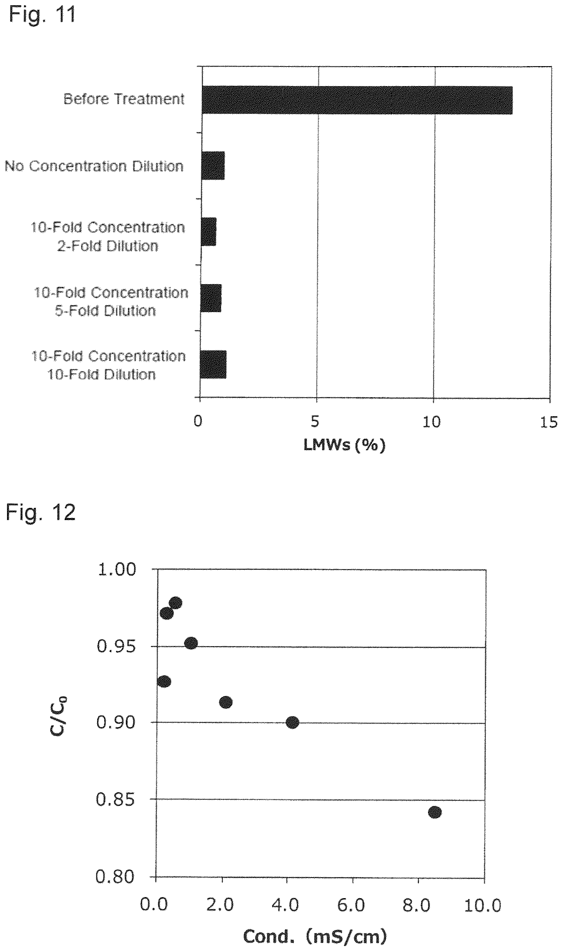

FIGS. 10 and 11 respectively show the content of the polymer (HMWs) of the activated carbon-treated solution of each concentrated dilute and the content of degradation products (LMWs). As shown in FIG. 10, it was confirmed that the polymer content was decreased by the concentration dilution of the pH-adjusted clarified solution, and the HMWs could be efficiently removed by increasing a dilution rate. In addition, as shown in FIG. 11, all of the LMWs could be efficiently removed regardless of the concentration dilution of the pH-adjusted clarified solution.

From the results described above, it was determined that, by preparing the activated carbon pretreatment solution with concentration-diluted pH-adjusted clarified solution, it is possible to achieve the high recovery ratio and the effective removal of the impurities such as the host cell protein, the polymer, and the degradation products at the same time.

Example 4 conductivity of activated carbon pretreatment solution suitable for activated carbon treatment

The CHO cell culture supernatant containing monoclonal antibodies (Mab B) was adjusted to pH 4.5 with acetate. After leaving for 1 hour, the cell and the formed precipitates were removed with a depth filter [manufactured by Merck millipore Corp.: Clarisolve (registered trademark)] and an MF membrane (manufactured by Merck millipore Corp.: SHC).

The obtained pH-adjusted clarified solution was subjected to 5-fold concentration with a UF membrane [manufactured by Merck millipore Corp.: Pellicon (registered trademark) 3 30kD], and continuously hydrated with milli-Q water, and each conductivity-adjusted solution with an antibody concentration of 20 mg/mL, and conductivity of 0.15 mS/cm, 0.3 mS/cm, 0.6 mS/cm, 1.2 mS/cm, 2.4 mS/cm, 4.8 mS/cm, or 9.6 mS/cm.

The activated carbon (manufactured by Japan EnviroChemicals, Ltd.: TOKUSEI SHIRASAGI, SHIRASAGI is registered trademark) was added to approximately 5 g of each conductivity-adjusted solution so as to obtain the load volume of 0.2 mg antibody/mg activated carbon, and was stirred at room temperature overnight. The activated carbon-treated solution having each conductivity obtained by performing centrifugation (2,900 g, 10 minutes) and filtration with a 0.2 μm filter of each solution after the stirring was obtained. As a control, each conductivity-adjusted solution to which the activated carbon was not added was used.

The antibody concentration of the obtained activated carbon-treated solution having each conductivity and the control was analyzed by protein A affinity HPLC, and the recovery ratio ($(C/C_0)$) was shown in FIG. 12. As shown in FIG. 12, the low conductivity side of the recovery ratio tends to increase, and in a case where the conductivity was 5 mS/cm, a high recovery ratio of 90% or higher was confirmed.

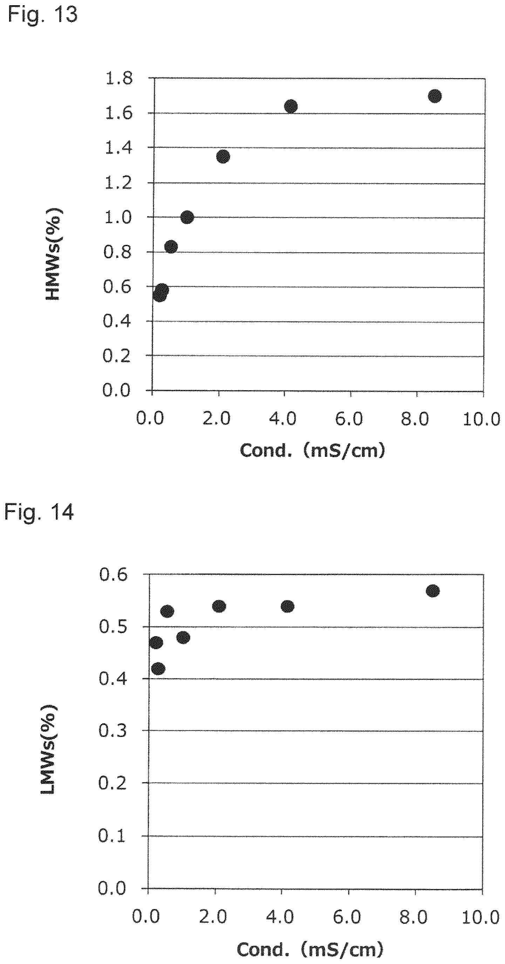

FIGS. 13 and 14 show results obtained by analyzing the content of polymer of the activated carbon-treated solution having each conductivity and the content of degradation products by gel filtration HPLC. As shown in FIG. 13, the content of polymer was decreased in accordance with a decrease in conductivity, and in a case where the conductivity was 1 mS/cm or less, a recovery ratio was less than 1%. In addition, as shown in FIG. 14, the content of degradation products was decreased in accordance with a decrease in conductivity, and in a case where the conductivity was 0 to 10 mS/cm, all of the recovery ratio was less than 1%.

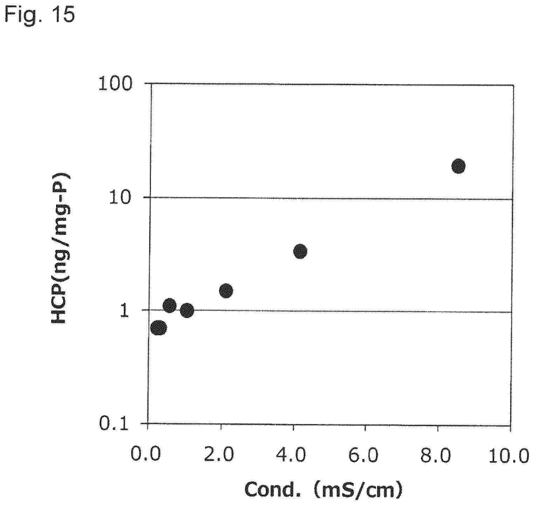

FIG. 15 shows a result obtained by analyzing the content of the host cell protein per 1 mg of protein by the ELISA method. As shown in FIG. 15, the content of the host cell protein was decreased in accordance with a decrease in conductivity, and in a case where the conductivity was 5 mS/cm or less, a low content of the host cell protein less than 10 ppm was confirmed.

From the result described above, it was determined that, by adjusting the conductivity of the pH-adjusted clarified solution to preferably 0 to 5 mS/cm, more preferably 0 to 2 mS/cm, and particularly preferably 0 to 1 mS/cm, it was possible to achieve the high recovery ratio and the effective removal of impurities such as the host cell protein, the polymer, and the degradation products at the same time.

Example 5 Concentration of Sodium Chloride of Activated Carbon-Pretreatment Solution Suitable for Activated Carbon Treatment

The CHO cell culture supernatant containing monoclonal antibodies (Mab B) was adjusted to pH 4.5 with acetate. After leaving for 1 hour, the cell and the formed precipitates were removed with a depth filter [manufactured by Merck millipore Corp.: Clarisolve (registered trademark)] and an SHC membrane (manufactured by Merck millipore Corp.).

The obtained pH-adjusted clarified solution was subjected to 5-fold concentration with a UF membrane [manufactured by Merck millipore Corp.: Pellicon (registered trademark) 3 30kD], and continuously hydrated with milli-Q water, to adjust the conductivity to 1 mS/cm or less. Sodium chloride was added to this solution, and each sodium chloride concentration-adjusted solution having sodium chloride concentration of 0 mmol/L, 5 mmol/L, 10 mmol/L, 20 mmol/L, 40 mmol/L, 80 mmol/L, and 160 mmol/L with the antibody concentration of 20 mg/mL was obtained.

The activated carbon (manufactured by Japan EnviroChemicals, Ltd.: TOKUSEI SHIRASAGI, SHIRASAGI is registered trademark) was added to approximately 5 g of each sodium chloride concentration-adjusted solution so as to obtain the load volume of 0.2 mg antibody/mg activated carbon, and was stirred at room temperature overnight. The activated carbon-treated solution having each sodium chloride concentration obtained by performing centrifugation (2,900 g, 10 minutes) and filtration with a membrane filter (0.2 μm filter) of each solution after the stirring was obtained. As a control, each sodium chloride concentration-adjusted solution to which the activated carbon was not added was used.

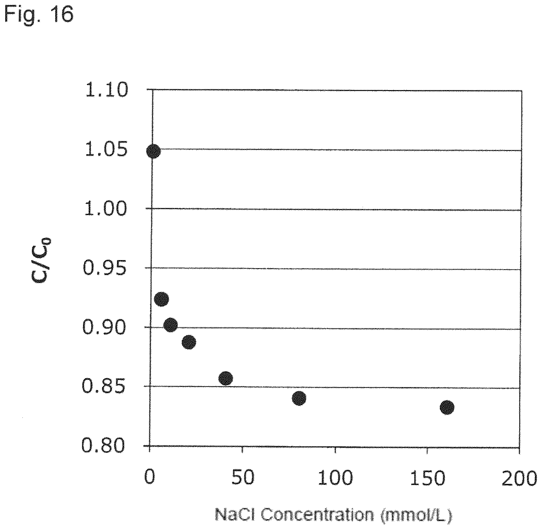

The antibody concentration of the obtained activated carbon-treated solution having each sodium chloride concentration was analyzed by protein A affinity HPLC, and the recovery ratio ($(C/C_0)$) was shown in FIG. 16. As shown in FIG. 16, the recovery ratio was increased in accordance with a decrease in sodium chloride concentration, and in a case where the sodium chloride concentration was 10 mmol/L or less, a high recovery ratio of 90% or higher was confirmed.

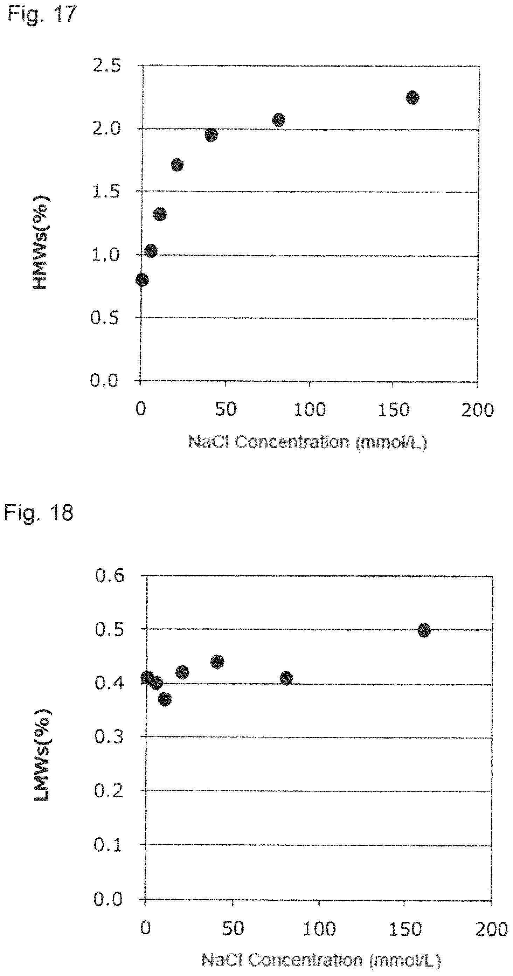

FIGS. 17 and 18 show results obtained by analyzing the content of polymer of the activated carbon-treated solution having each sodium chloride concentration and the content of degradation products by gel filtration HPLC. As shown in FIG. 17, the content of polymer was decreased in accordance with a decrease in sodium chloride concentration, and in a case where the sodium chloride concentration was 5 mmol/L or less, the polymer content was less than 1%. In addition, as shown in FIG. 18, the content of degradation products tended to decrease in accordance with a decrease in sodium chloride concentration, and in a case where the sodium chloride concentration was 0 to 160 mmol/L, the content of all degradation products was less than 1%.

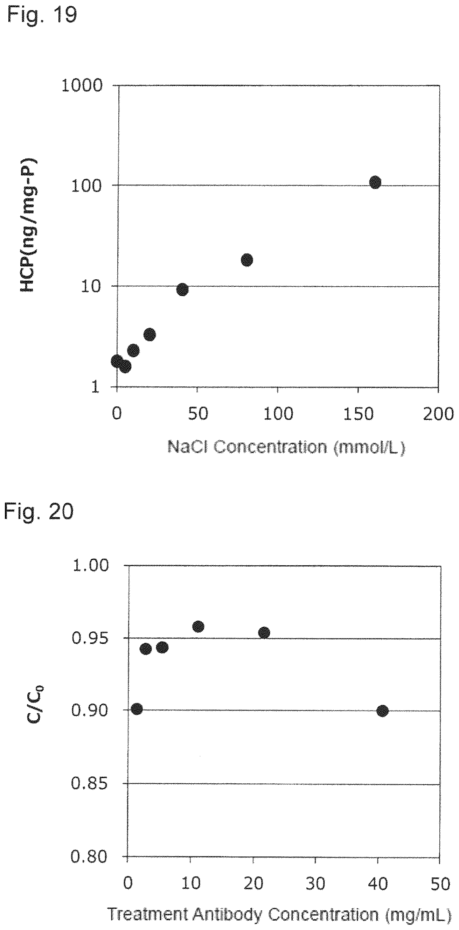

FIG. 19 shows a result obtained by analyzing the content of the host cell protein per 1 mg of protein by the ELISA method. As shown in FIG. 19, the content of the host cell protein was decreased in accordance with a decrease in sodium chloride concentration, and in a case where the sodium chloride concentration was 40 mmol/L or less, a low content of the host cell protein less than 10 ppm was confirmed.

From the result described above, it was determined that, by adjusting the sodium chloride concentration of the pH-adjusted clarified solution to preferably 0 to 10 mmol/L and more preferably 0 to 5 mmol/L, it was possible to achieve the high recovery ratio and the effective removal of impurities such as the host cell protein, the polymer, and the degradation products at the same time.

Example 6 Concentration of Antibody of Activated Carbon-Pretreatment Solution Suitable for Activated Carbon Treatment

The CHO cell culture supernatant containing monoclonal antibodies (Mab B) was adjusted to pH 4.5 with acetate. After leaving for 1 hour, the cell and the formed precipitates were removed with a depth filter [manufactured by Merck millipore Corp.: Clarisolve (registered trademark)] and an SHC membrane (manufactured by Merck millipore Corp.).

The concentration/milli-Q water hydration of the obtained pH-adjusted clarified solution was repeatedly performed with a UF membrane [manufactured by Merck millipore Corp.: Pellicon (registered trademark) 3 30kD], and the conductivity was adjusted to 1 mS/cm or less. The milli-Q water was added to the solution, and each antibody concentration-adjusted solution having the antibody concentration of 45.1 mg/mL, 22.5 mg/mL, 11.4 mg/mL, 5.5 mg/mL, 2.8 mg/mL, and 1.4 mg/mL was obtained.

The activated carbon (manufactured by Japan EnviroChemicals, Ltd.: TOKUSEI SHIRASAGI, SHIRASAGI is registered trademark) was added to approximately 5 g of each antibody concentration-adjusted solution so as to obtain the load volume of 0.2 mg antibody/mg activated carbon, and was stirred at room temperature overnight. The activated carbon-treated solution having each antibody concentration obtained by performing centrifugation (2,900 g, 10 minutes) and filtration with a membrane filter (0.2 $\mu$m filter) of each solution after the stirring was obtained. As a control, each antibody concentration-adjusted solution to which the activated carbon was not added was used.

The antibody concentration of the obtained activated carbon eluate having each antibody concentration and the control was analyzed by protein A affinity HPLC, and the recovery ratio ($(C/C_0)$) was shown in FIG. 20. As shown in FIG. 20, in a case of any antibody concentration, a high recovery ratio of 90% or higher was confirmed.

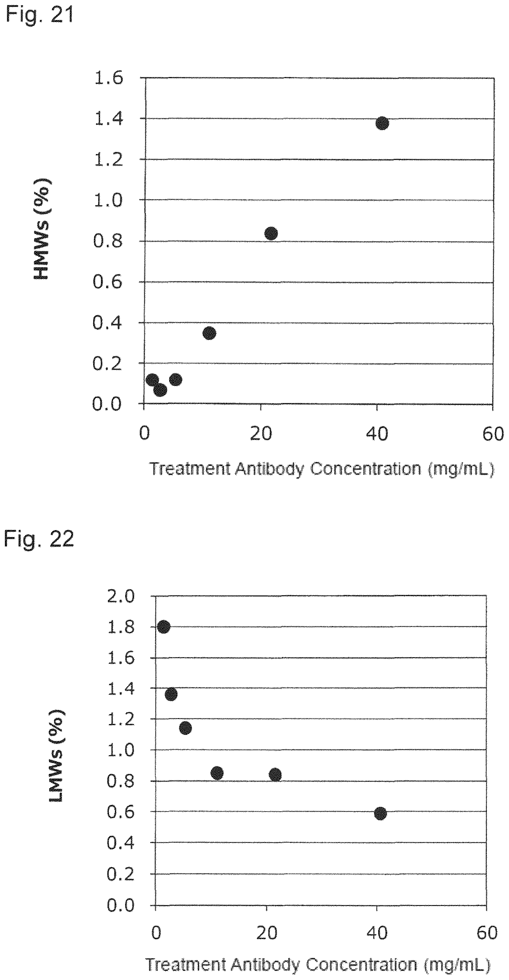

FIGS. 21 and 22 show results obtained by analyzing the content of polymer of the activated carbon eluate having each antibody concentration and the content of degradation products by gel filtration HPLC. As shown in FIG. 21, the content of polymer was decreased in accordance with a decrease in antibody concentration, and in a case where the antibody concentration was 23 mg/mL or less, the content of polymer was less than 1%. In addition, as shown in FIG. 22, the content of degradation products was decreased in accordance with an increase in antibody concentration, and in a case where the antibody concentration was 11 mg/mL or more, the content of all of the degradation products was less than 1%.

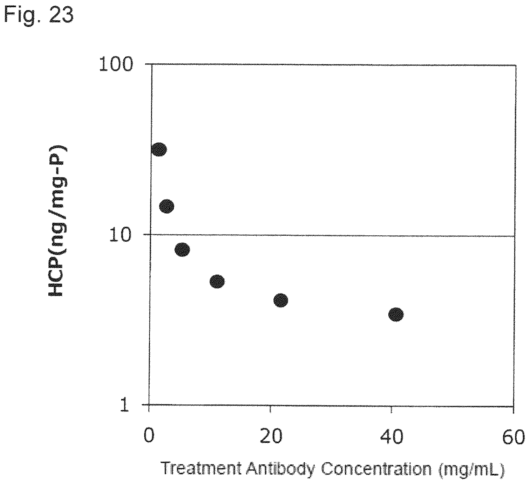

FIG. 23 shows a result obtained by analyzing the content of the host cell protein per 1 mg of protein by the ELISA method.

As shown in FIG. 23, the content of the host cell protein was decreased in accordance with an increase in antibody concentration, and in a case where the antibody concentration was 5.5 mg/mL or more, a low content of the host cell protein less than 10 ppm was confirmed.

From the result described above, it was determined that, by adjusting the antibody concentration of the pH-adjusted clarified solution to preferably 5.5 to 23 mg/mL and more preferably 11 to 23 mg/mL, it was possible to achieve the high recovery ratio and the effective removal of impurities such as the host cell protein, the polymer, and the degradation products at the same time.

Example 7 Load Volume Suitable For Activated Carbon Treatment and Activated Carbon Treatment Time

The CHO cell culture supernatant containing monoclonal antibodies (Mab B) was adjusted to pH 4.5 with acetate. After leaving for 1 hour, the cell and the formed precipitates were removed with a depth filter [manufactured by Merck millipore Corp.: Clarisolve (registered trademark)] and an SHC membrane (manufactured by Merck millipore Corp.).

The concentration/milli-Q water hydration of the obtained pH-adjusted clarified solution was repeatedly performed with a UF membrane [manufactured by Merck millipore Corp.: Pellicon (registered trademark) 3 30kD], and the conductivity was decreased to 1 mS/cm or less. The milli-Q water was added to the solution, and the antibody concentration was adjusted to 10 mg/mL.

The activated carbon (manufactured by Japan EnviroChemicals, Ltd.: TOKUSEI SHIRASAGI, SHIRASAGI is registered trademark) was added to approximately 5 g of the obtained solution so as to obtain the load volume of 0.05 to 3.2 mg antibody/mg activated carbon, and the activated treatment was performed for the treatment time of 2 to 24 hours (2, 4, 8, 12, or 24 hours). Each activated carbon-treated solution obtained by performing centrifugation (2,900 g, 10 minutes) and filtration with a membrane filter (0.2 $\mu$m filter) of each solution after the treatment was obtained. As a control, each solution to which the activated carbon was not added was used. The antibody concentration of the activated carbon-treated solution that is treated with each load volume for treatment time and the control was analyzed by protein A affinity HPLC.

As a result, as shown in FIG. 24, a decrease in recovery ratio ($(C/C_o)$) was recognized with the treatment with the load volume of 0.05 mg antibody/mg activated carbon for 24 hours, but the recovery ratio was maintained with the other load volume, even in the treatment for 24 hours.

FIGS. 25 and 26 show results obtained by analyzing the content of polymer of each activated carbon-treated solution and the content of degradation products by gel filtration HPLC. As shown in FIG. 25, in any examination conditions with the load volume of 0.05 to 3.2 mg antibody/mg activated carbon for treatment time of 2 to 24 hours, the content of polymer was less than 1%. In addition, as shown in FIG. 26, the content of degradation products was decreased in accordance with a decrease in load volume or an increase in treatment time, and in a case where the load volume was 0.05 to 0.3 mg antibody/mg activated carbon and the treatment time was 12 to 24 hours, the content of all of the degradation products was less than 1%.

FIG. 27 shows an analysis result of each activated carbon-treated solution obtained by analyzing the content of the host cell protein per 1 mg of protein by the ELISA method. As shown in FIG. 27, the content of the host cell protein was decreased in accordance with a decrease in load volume or an increase in treatment time, and in a case where the load volume was 0.05 to 0.2 mg antibody/mg activated carbon and the treatment time was 8 to 24 hours, the content of host cell protein was less than 10 ppm.

From the results of FIGS. 24 to 27, it was confirmed that a combination of the load volume and the activated carbon treatment time, with which all impurities can be sufficiently removed and a high recovery ratio is obtained, is the load volume of 0.1 to 0.3 mg antibody/mg activated carbon and the treatment time of 8 to 24 hours, or the load volume of 0.05 to 0.15 mg antibody/mg activated carbon and the treatment time of 2 to 12 hours.

Example 8 Effect of Removal of Impurities due to One-Time Solution Passage Treatment and Circulation Solution Passage Treatment of Activated Carbon Membrane

185 L of the CHO cell culture broth containing monoclonal antibodies (Mab B) was adjusted to pH 4.5 with acid or alkali. After leaving the pH-adjusted CHO cell culture broth for 1 hour, the cell was removed using a cell separation depth membrane [manufactured by Merck millipore Corp.: Clarisolve (registered trademark)], and the pH-adjusted clarified solution was obtained with an SHC membrane (manufactured by Merck millipore Corp.).

The pH-adjusted clarified solution was concentrated to 5 L with a UF membrane [manufactured by Merck millipore Corp.: Pellicon (registered trademark) 3], and diluted to 18.5 L using pure water, and the concentrated dilute was prepared. 17.5 L of the concentrated dilute was passed through an activated carbon membrane of 2.8 m² [manufactured by Pall Corp.: Stax (trademark) AKS1] once, and a one-time solution passage-treated solution was obtained. Then, the circulation solution passage was performed at room temperature for 16 hours and a circulated solution was recovered. In addition, 18.5 L of pure water was passed through the activated carbon membrane and recovered, and this was mixed with the circulated solution. The obtained mixed and recovered solution was passed through the SHC membrane (manufactured by Merck millipore Corp.) and the circulated solution passage-treated solution was obtained.

FIG. 28 shows a result obtained by analyzing the content of degradation products of each activated carbon membrane-treated solution with the gel filtration HPLC. As shown in FIG. 28, the content of degradation products was decreased by the solution passage through the activated carbon film once and was further decreased by the circulation solution passage.

FIG. 29 shows a result obtained by analyzing the content of host cell protein per 1 mg of protein by the ELISA method. As shown in FIG. 29, the content of host cell protein was decreased to 1/30 amount by performing the one-time solution passage through the activated carbon membrane, and was significantly decreased to a level of 1/80 amount in a case of the one-time solution passage, by performing the circulation solution passage.

FIG. 30 shows a result obtained by the DNA concentration of each activated carbon membrane-treated solution by a threshold method. As shown in FIG. 30, it was clear that, by only performing the one-time solution passage through the activated carbon membrane, the DNA concentration was removed to a value lower than the limit of quantitation.

From the results described above, regarding the activated carbon membrane treatment, it was confirmed that the impurities can be more effectively removed by the circulation solution passage treatment, compared to the one-time solution passage treatment.

Example 9 Type of Activated Carbon Suitable For Activated Carbon Treatment

The CHO cell culture broth containing monoclonal antibodies (Mab A, C, and D) clarified by microfiltration was adjusted to pH 4.5 with acid or alkali. After leaving for 1 hour, the formed precipitates were removed by centrifugation (13,200 g, 10 minutes) and then membrane filter filtration (0.22 μm filter), and each pH-adjusted clarified solution was obtained.

Then, 20 to 40 mg of each activated carbon shown in FIGS. 31 and 32 (manufactured by Japan EnviroChemicals, Ltd., manufactured by Norit Japan Co., Ltd., manufactured by FUTAMURA Chemical CO., Ltd.) was added and mixed to approximately 2 mL of the obtained each pH-adjusted clarified solution. The mixed solution was stirred with a rotator at room temperature for 16 hours, the activated carbon was removed by centrifugation (13,200 g, 10 minutes) and then membrane filter filtration (0.22 μm filter), and each activated carbon-treated solution was obtained.

FIG. 31 shows the recovery ratio ($C/C_0$) obtained by analyzing the antibody concentration of each activated carbon-treated solution by protein A affinity HPLC. In addition, FIG. 32 shows a result obtained by analyzing the concentration of host cell protein of each of the obtained activated carbon-treated solution by the ELISA method.

As shown in FIGS. 31 and 32, removal properties of the host cell protein were confirmed in all activated carbons, and it was confirmed that, particularly, KYORYOKU SHIRASAGI, TAIKO Y, TOKUSEI SHIRASAGI, SHIRASAGI P, SHIRASAGI A, SHIRASAGI C (which are manufactured by Japan EnviroChemicals, Ltd., SHIRASAGI is registered trademark), TAIKO KA, TAIKO A (which are manufactured by FUTAMURA Chemical CO., Ltd.), Norit C GSP, or Norit CNI, (which are manufactured by Norit Japan Co., Ltd., Norit is registered trademark) which are activated carbons from wood, and Norit GBG (manufactured by Norit Japan Co., Ltd., Norit is registered trademark) which is activated carbon formed of wood and beet have particularly high removal properties of the host cell protein. In addition, among the activated carbons from wood, KYORYOKU SHIRASAGI, TAIKO Y, and TOKUSEI SHIRASAGI which are zinc chloride-activated activated carbons particularly have high removal properties of the host cell protein.

Then, FIGS. 33 and 34 show results obtained by analyzing the content of polymer of each activated carbon eluate and the content of degradation product by gel filtration HPLC.

As shown in FIG. 33, a significant decrease in content of polymer in Mab A was confirmed. A particularly great decrease in content of polymer was confirmed with KYORYOKU SHIRASAGI, TOKUSEI SHIRASAGI (which are manufactured by Japan EnviroChemicals, Ltd., SHIRASAGI is registered trademark), TAIKO Y (manufactured by FUTAMURA Chemical CO., Ltd.) which are zinc chloride-activated activated carbons from wood, SHIRASAGI P, SHIRASAGI A, SHIRASAGI C (which are manufactured by Japan EnviroChemicals, Ltd., SHIRASAGI is registered trademark), TAIKO KA, TAIKO A (which are manufactured by FUTAMURA Chemical CO., Ltd.) which are vapor-activated activated carbons from wood.

In addition, as shown in FIG. 34, a decrease in content of degradation products due to the activated carbon treatment was confirmed in all antibodies. Among the activated carbons, a significant decrease in content of degradation products was confirmed by performing the treatment with KYORYOKU SHIRASAGI, TOKUSEI SHIRASAGI, SHIRASAGI P, SHIRASAGI A, SHIRASAGI C (which are manufactured by Japan EnviroChemicals, Ltd., SHIRASAGI is registered trademark), TAIKO Y, TAIKO KA, TAIKO A (which are manufactured by FUTAMURA Chemical CO., Ltd.), Norit C GSP, and Norit CNI which are activated carbons from wood, and Norit GBG (manufactured by Norit Japan Co., Ltd., Norit is registered trademark) which is activated carbon formed of wood and beet. Particularly, it was confirmed that the content of degradation product is effectively decreased by performing the treatment with KYORYOKU SHIRASAGI, TOKUSEI SHIRASAGI (which are manufactured by Japan EnviroChemicals, Ltd., SHIRASAGI is registered trademark), TAIKO Y (manufactured by FUTAMURA Chemical CO., Ltd.) which are zinc chloride-activated activated carbons from wood.

Example 10 Type of Activated Carbons Suitable For Activated Carbon Membrane Treatment

The CHO cell culture supernatant containing monoclonal antibodies (Mab B) was adjusted to pH 4.5 with acetate. After leaving for 1 hour, the cell and the formed precipitates were removed with a depth filter [manufactured by Merck millipore Corp.: Clarisolve (registered trademark)] and an SHC membrane (manufactured by Merck millipore Corp.).

The concentration/dilution with milli-Q water of the obtained pH-adjusted clarified solution was repeatedly performed with a UF membrane [manufactured by Merck millipore Corp.: Pellicon (registered trademark) 3 30kD], and the conductivity was decreased to 1 mS/cm or less. The milli-Q water was added to the solution, and the antibody concentration was adjusted to 30 mg/mL.

The obtained solution was passed through the activated carbon membrane shown in Table 1 under the treatment conditions, and the circulation treatment of returning the filtered solution to the activated carbon membrane again was performed at room temperature for 16 hours. After that, each activated carbon membrane-treated solution, in which the antibodies remaining in the circulated solution and the membrane inner portion were recovered, was obtained. As a control, each solution not subjected to the activated carbon membrane treatment was used.

[Table 1]

| Table 1 Activated Carbon Membrane Treatment Conditions | | | | |
|---|---|---|---|---|
| Membrane type | Membrane area (cm$^2$) | Treatment solution amount (g) | Circulation flow rate (mL/min) | Load volume (mg antibody/mg activated carbon) |
| TOKUSEI SHIRASAGI (3M) | 25 | 16.7 | 2.1 | 0.2 |

(continued)

| Table 1 Activated Carbon Membrane Treatment Conditions | | | | |
|---|---|---|---|---|
| Membrane type | Membrane area (cm$^2$) | Treatment solution amount (g) | Circulation flow rate (mL/min) | Load volume (mg antibody/mg activated carbon) |
| SHIRASAGI P (3M) | 25 | 16.7 | 2.1 | 0.2 |
| AKS1 (Pall) | 22 | 15.3 | 1.8 | 0.2 |
| TAIKO Y (3M) | 25 | 16.7 | 2.1 | 0.2 |
| KYORYOKU SHIRASAGI (3M) | 25 | 16.7 | 2.1 | 0.2 |
| R55SLP (3M) | 25 | 16.7 | 2.1 | 0.2 |

The antibody concentration of each of the obtained activated carbon membrane-treated solution and the control was analyzed by protein A affinity HPLC, and the recovery rate was shown in FIG. 35. As shown in FIG. 35, a high recovery rate of 80% or higher was obtained with TOKUSEI SHIRASAGI and KYORYOKU SHIRASAGI (which are manufactured by Japan EnviroChemicals, Ltd., manufactured by 3M, SHIRASAGI is registered trademark), or AKS1 filter (manufactured by Pall Corp.).

FIGS. 36 and 37 show results obtained by analyzing the content of polymer of the activated carbon-treated solution and the content of degradation products by gel filtration HPLC. As shown in FIGS. 36 and 37, the content of polymer and the content of degradation products were less than 1%, with any membrane type.

FIG. 38 shows a result obtained by analyzing the content of the host cell protein per 1 mg of protein by the ELISA method. As shown in FIG. 38, the content of the host cell protein was approximately less than 10 ppm with any membrane type.

From the result described above, it was confirmed that the effect of removal of impurities was obtained to approximately the same low level, with any activated carbon membrane type, and a higher recovery rate was obtained with TOKUSEI SHIRASAGI, KYORYOKU SHIRASAGI (which are manufactured by Japan EnviroChemicals, Ltd., manufactured by 3M, SHIRASAGI is registered trademark), or AKS1 filter (manufactured by Pall Corp.).

Example 11 Micropore Diameter of Activated Carbon Suitable for Activated Carbon Treatment

FIG. 39 shows a relationship between an average micropore diameter of the activated carbon, in a case where the pH-adjusted clarified solution containing Mab A was treated with various activated carbons, and HCP concentration after the activated carbon treatment. The average micropore diameter of the activated carbon was calculated with nitrogen adsorption isothermal adsorption curve by the BJH method.

As shown in FIG. 39, the average micropore diameter and the HCP concentration have a correlation, and a significant decrease in HCP concentration was confirmed in accordance with an increase in average micropore diameter. Particularly, it was confirmed that the HCP concentration was effectively decreased by performing the treatment with the activated carbon having an average micropore diameter greater than 3.2 nm.

FIGS. 40 and 41 respectively show a relationship between the average micropore diameter of the activated carbons, in a case where the pH-adjusted clarified solution containing Mab A was treated with various activated carbons, and the content of polymer and the content of degradation product after the activated carbon treatment. As shown in FIGS. 40 and 41, the average micropore diameter, and the content of polymer and the content of degradation product have a correlation, and a significant decrease in these contents was confirmed in accordance with an increase in average micropore diameter. Particularly, it was confirmed that these contents were effectively decreased by performing the treatment with the activated carbon having an average micropore diameter greater than 3.2 nm.

Example 12 Addition of Acid or Amine Acid Suitable For Activated Carbon Pretreatment Solution

The CHO cell culture supernatant containing monoclonal antibodies (Mab B) was adjusted to pH 4.5 with acetate. After leaving for 1 hour, the cell and the formed precipitates were removed with a depth filter [manufactured by Merck millipore Corp.: Clarisolve (registered trademark)] and an SHC membrane (manufactured by Merck millipore Corp.).

The concentration/milli-Q water hydration of the obtained pH-adjusted clarified solution was repeatedly performed with a UF membrane [manufactured by Merck millipore Corp.: Pellicon (registered trademark) 3 30kD], and the conductivity of the solution was decreased. The antibody concentration was adjusted to approximately 20 mg/mL with milli-Q water, acid or amine acid solution shown in FIGS. 42 to 44 was added, and each activated carbon pretreatment solution adjusted to pH 4.25 was prepared.

Next, the activated carbon (manufactured by Japan EnviroChemicals, Ltd., TOKUSEI SHIRASAGI, SHIRASAGI is registered trademark) was added to approximately 5 g of each of the obtained pH-adjusted solution so as to obtain the load volume of 0.2 mg antibody/mg activated carbon and stirred at room temperature for 16 hours. After that, each solution was subjected to centrifugation (2,900 g, 10 minutes) and then membrane filter filtration (0.2 $\mu$m filter), and each activated carbon-treated solution was obtained. As a control each acid or amine acid-added solution, to which activated carbon was not added, was used.

The antibody concentration of each activated carbon-treated solution and the control was analyzed by protein A affinity HPLC, and the recovery ratio ($(C/C_o)$) was shown in FIG. 42. As shown in FIG. 42, in a case where 50% of acetate, 3 M citric acid, or 3 M glycine were added, a particularly high recovery ratio ($C/C_o$) was shown.

The content of polymer of each activated carbon eluate and the content of degradation products were analyzed by gel filtration HPLC. FIGS. 43 and 44 show the content of polymer and the content of degradation products due to the load of each acid or amine acid due to the activated carbon treatment. As shown in FIG. 43, even in a case where 2M arginine is excluded and any of acid or amine acid was added, the content of polymer was less than 1%. As shown in FIG. 44, all of the content of degradation products was less than 1%, with each acid or amine acid examined.

The content of the host cell protein per 1 mg of protein was analyzed by the ELISA method. FIG. 45 shows the content of the host cell protein due to addition of each acid or amine acid. As shown in FIG. 45, all of HCPs had low content less than 10 ppm, in a case where acid or amine acid was added.

From the results described above, in a case of adding acid or amine acid suitable for the activated carbon pretreatment solution having a high effect of removal of impurities and a high recovery ratio, 50% acetate, 3M citric acid, or 3 M glycine was particularly preferable.

Example 13 Mab A Purification [Activated Carbon Process (1)]

A CHO cell culture broth containing the monoclonal antibodies (Mab A) was adjusted to pH 4.5 with acid or alkali. After leaving for 1 hour, the cell was removed using a cell separation depth membrane [manufactured by Merck millipore Corp.: Clarisolve (registered trademark)], and the pH-adjusted clarified solution was obtained through the SHC membrane (manufactured by Merck millipore Corp.).

20 L of the pH-adjusted clarified solution was concentrated to 400 mL with the UF membrane manufactured by Merck millipore Corp.: Pellicon (registered trademark) 3], and concentrated dilute diluted to 4,000 mL with the milli-Q water was prepared. 3,700 mL of the concentrated dilute was moved to two 3L spinner flasks (manufactured by Corning Inc.), 81.41 g of KYORYOKU SHIRASAGI (manufactured by Japan EnviroChemicals, Ltd., SHIRASAGI is registered trademark) was respectively added thereto, mixed and stirred at room temperature for 16 hours, and an activated carbon mixed solution was adjusted. The activated carbon mixed solution was subjected to centrifugation and the supernatant was recovered.

In addition, 220 mL of the milli-Q water was added and suspended to the precipitated activated carbon and subjected to the centrifugation again, and the supernatant was recovered. This operation was repeated once more and the obtained supernatant was all mixed. 4,580 mL of the activated carbon-treated solution obtained by clarifying the mixed supernatant with the SHC membrane (manufactured by Merck millipore Corp.) was obtained.

2 mol/L of acetate was added to the activated carbon-treated solution to adjust pH to 3.5, and held for 1 hour to perform virus inactivation. After the virus activation, a virus inactivation-treated solution having pH adjusted to 8.0 with 3 mol/L tris solution was obtained.

2 L of the virus inactivation-treated solution was passed through an anion exchange membrane [manufactured by Asahi Kasei Medical Co.: Qyu Speed (trademark) D, 150 mL] that was equilibrated with an equilibration buffer consisting of 10 mmol/L Tris buffer (pH 8.0) in advance. Then, the equilibration buffer was passed through and the non-adsorbed fraction was recovered, and QSD-treated solution having pH adjusted to 5.0 with 2M acetate was obtained.

The QSD-treated solution was added to a cation exchange column [manufactured by Merck millipore Corp.: Eshmuno (registered trademark) CPX, 50 mm ID $\times$ 20 cm] that was equilibrated with an equilibration buffer consisting of 10 mmol/L sodium acetate buffer (pH 5.0) in advance. After completion of the addition, the column was washed with 5 column volumes of the equilibration buffer. Then, a CPX eluate was obtained by linear salt concentration gradient (10-column volumes) using the equilibration buffer and the 10 mmol/L sodium acetate buffer (pH 5.0) containing 0.3 mol/L sodium chloride.

The CPX eluate was passed through the virus-free membrane [manufactured by Asahi Kasei Medical Co., Ltd.: Planova (trademark) 20N, 0.01 m$^2$] that was equilibrated with the milli-Q water in advance, and a virus filtration membrane-treated solution was obtained. The virus filtration membrane-treated solution was concentrated to approximately 120 mg/mL with the UF membrane [manufactured by Merck millipore Corp.: Pellicon (registered trademark) 3, 0.11 m$^2$], the buffer exchange was performed to 10 mmol/L histidine buffer (pH 5.2) containing 245 mmol/L D-sorbitol, and a concentration · buffer exchange solution was obtained.

The 10 mmol/L histidine buffer (pH 5.2) containing 2.20 g/L of polysorbate 80 and 245 mmol/L of D-sorbitol was added to the concentration · buffer exchange solution so that the total concentration of polysorbate 80 becomes 0.2 g/L, and filtered with a filter (0.22 μm filter), and an activated carbon process (1) purified product was obtained.

Example 14 Mab A Purification [Activated Carbon Process (2)]

2 L of the virus inactivation-treated solution obtained in Example 13 was passed through an anion exchange resin [manufactured by TOSOH Corp.: TOYOPEARL (registered trademark) NH$_2$-750F, 32 mm ID × 24 cm] that was equilibrated with an equilibration buffer consisting of 100 mmol/L Tris buffer (pH 8.0) in advance. Then, the equilibration buffer was passed through and the column non-adsorbed fraction was recovered, and a NH$_2$ Toyopearl-treated solution having pH adjusted to 5.0 with 2M acetate was obtained.

The NH$_2$ Toyopearl-treated solution was passed through a virus-free membrane [manufactured by Asahi Kasei Medical Co.: Planova (registered trademark) 20N, 0.01 m$^2$] that was equilibrated with the milli-Q water in advance, and the virus filtration membrane-treated solution was obtained. The virus filtration membrane-treated solution was concentrated to approximately 120 mg/mL with the UF membrane [manufactured by Merck millipore Corp.: Pellicon (registered trademark) 3, 0.11 m$^2$], the buffer exchange was performed to 10 mmol/L histidine buffer (pH 5.2) containing 245 mmol/L D-sorbitol, and a concentration · buffer exchange solution was obtained.

The 10 mmol/L histidine buffer (pH 5.2) containing 2.20 g/L of polysorbate 80 and 245 mmol/L of D-sorbitol was added to the concentration · buffer exchange solution so that the total concentration of polysorbate 80 becomes 0.2 g/L, and filtered with a filter (0.22 μm filter), and an activated carbon process (2) purified product was obtained.

Comparative Example 1 Mab A Purification (process comprising protein A affinity chromatography)

The CHO cell culture broth containing the monoclonal antibodies (Mab A) which are the same as that in Example 13 was subjected to centrifugation to remove the cell, and the obtained supernatant was passed through the depth membrane [manufactured by Merck millipore Corp.: Millistak (registered trademark) POD filter] and the microfiltration membrane to obtain a clarified solution.

The clarified solution was added to the protein A affinity chromatography column [manufactured by GE Healthcare Ltd., Inc.: MabSelect (registered trademark) SuRe] that equilibrated with an equilibration buffer consisting of 10 mmol/L Tris buffer (pH 7.0) in advance. After completion of the addition, the column was washed with 10 mmol/L tris buffer (pH 7.0) containing 5 column volumes of 1 mol/L sodium chloride and equilibration buffer.

Then, the column-eluted fraction that was eluted by 5 column volumes of 100 mmol/L glycine buffer (pH 3.2) was pooled as a MabSelect SuRe eluate. 0.1 mol/L hydrochloric acid was added to the MabSelect SuRe eluate to adjust the pH to 3.5, and maintained for 1 hour to perform virus inactivation. After the virus inactivation, the pH was neutralized to 7.0 with 0.5 M tris solution, and a virus inactivation-treated solution was obtained through a depth filter [manufactured by Merck millipore Corp.: Millistak (registered trademark) POD filter].

The virus inactivation-treated solution was added to an anion exchange chromatography column (manufactured by GE Healthcare Ltd., Inc., Q Sepharose XL, Sepharose is registered trademark) that was equilibrated with an equilibration buffer consisting of 10 mmol/L Tris buffer (pH 7.0), in advance. After completion of the addition, 5 column volumes of the equilibration buffer were passed through the column, the column non-adsorbed fraction was pooled, and then, a Q Sepharose eluate having the pH adjusted to 5.0 with 1 mol/L citric acid was obtained.

The Q Sepharose eluate was added to a cation exchange chromatography column (manufactured by Merck millipore Corp.: Fractogel (trademark) EMD SE Hicap) that was equilibrated with an equilibration buffer consisting of 20 mmol/L sodium citrate buffer (pH 5.0) containing 50 mmol/L sodium chloride in advance. After completion of the addition, 5 column volumes of the equilibration buffer were passed through the column.

Then, a Fractogel recovered solution was obtained by linear salt concentration gradient (10-column volumes) using the equilibration buffer and 20 mmol/L sodium citrate buffer (pH 5.0) containing 0.3 mol/L sodium chloride.

The Fractogel recovered solution was passed through the virus-free membrane [manufactured by Asahi Kasei Medical Co., Ltd.: Planova (registered trademark) 20N] that was equilibrated with the 20 mmol/L sodium citrate buffer (pH 5.0) containing 50 mmol/L sodium chloride in advance, and a virus filtration membrane-treated solution was obtained.

The virus filtration membrane-treated solution was concentrated to approximately 120 mg/mL with the UF membrane [manufactured by Merck millipore Corp.: Pellicon (registered trademark) 3], the buffer exchange was performed to 10 mmol/L histidine buffer (pH 5.2) containing 245 mmol/L D-sorbitol, and a concentration · buffer exchange solution was obtained.

The 10 mmol/L histidine buffer (pH 5.2) containing 2.20 g/L of polysorbate 80 and 245 mmol/L of D-sorbitol was added to the concentration · buffer exchange solution so that the total concentration of polysorbate 80 becomes 0.2 g/L, and filtered with a filter (0.22 μm filter), and a protein A process purified product was obtained.

The results obtained by the comparison of the protein A affinity chromatography and the activated carbon process (1) and the activated carbon process (2) are shown below.

FIG. 46 shows a total recovery rate of each process of the purification and a total recovery rate based on the entire purification process analyzed with the concentration quantitation by protein A affinity HPLC. FIG. 47 shows a result of the content of polymer of the purification intermediate and the final purified product analyzed by the gel filtration HPLC, and FIG. 48 shows the result of the content of degradation product, respectively.

As shown in FIGS. 46 to 48, in the activated carbon processes (1) and (2), the recovery rate is slightly decreased, compared to that of the protein A affinity chromatography, but the recovery rate of 50% or more is shown and the polymer and the degradation product were effectively removed.

FIG. 49 shows a result obtained by analyzing the concentration of the host cell protein of the purification intermediate and the final purified product by the ELISA method. As shown in FIG. 49, it was clear that, in the activated carbon processes (1) and (2), 100 times or more of the host cell protein was removed compared to that of the protein A chromatography. In addition, in a case of bulk drug, the concentration of the host cell protein was low in the activated carbon processes (1) and (2), compared to that of the protein A process.

FIG. 50 shows a result obtained by analyzing the DNA concentration of the purification intermediate and the final purified product by the Threshold method. It was clear that, in the activated carbon processes (1) and (2), the concentration was decreased to less than 1/1800, compared to the protein A chromatography.

FIGS. 51 and 52 show results of a pre-peak content and a post-peak content of the purification intermediate and the final purified product analyzed by cation exchange HPLC. As shown in FIGS. 51 and 52, it was confirmed that, the post-peak content was decreased by the activated carbon treatment, compared to that of protein A process, and a more homogeneous antibody composition was obtained.

From the results described above, in the activated carbon processes (1) and (2) of preparing the pretreatment solution by the concentration dilution, compared to the protein A process of the related art, all of impurities such as the polymers, degradation products, the host cell proteins, the DNAs, and the post-peak were effectively removed.

As a result, in the protein A process of the related art for removing these impurities to a drug product level, both ion exchange chromatography of the anion exchange and the cation exchange was necessary, but it was confirmed that, in the activated carbon processes (1) and (2), it is possible to remove the impurities to the drug product level only by any one of ion exchange chromatography.

Example 15 Mab B Purification (activated carbon process)

185 L of CHO cell culture broth containing monoclonal antibodies (Mab B) was adjusted to pH 4.5 with acid or alkali. After leaving the pH-adjusted CHO cell culture broth for 1 hour, the cell was removed using a cell separation depth membrane [manufactured by Merck millipore Corp.: Clarisolve (registered trademark)], and the pH-adjusted clarified solution was obtained with an SHC membrane (manufactured by Merck millipore Corp.).

The pH-adjusted clarified solution was concentrated to 5 L with a UF membrane [manufactured by Merck millipore Corp.: Pellicon (registered trademark) 3], and diluted to 18.5 L using pure water, and the concentrated dilute was prepared. 17.5 L of the concentrated dilute was circulated and passed through an activated carbon membrane of 2.8 m$^2$ [manufactured by Pall Corp.: Stax (trademark) AKS1] overnight, and a circulated solution was recovered. Further, 18.5 L of pure water was passed through the activated carbon membrane and recovered, and this and circulated solution was combined and mixed with each other. The obtained mixed recovered solution was passed through the SHC membrane (manufactured by Merck millipore Corp.) and an activated carbon-treated solution was obtained.

3 mol/L tris was added to 28.8 L of the activated carbon-treated solution to adjust the pH to 7.0, and the resulting solution was passed through an anion exchange membrane [manufactured by Asahi Kasei Medical Co.: Qyu Speed (trademark) D, 150 mL] that was equilibrated with an equilibration buffer consisting of 10 mmol/L Tris buffer (pH 7.0) in advance. Then, the equilibration buffer was passed through the anion exchange membrane, and the non-adsorbed fraction was recovered as a QSD-treated solution.

50% (W/W) acetic acid was added to the QSD-treated solution to adjust the pH to 3.5, and held for 1 hour to perform virus inactivation. After the virus activation, a virus inactivation-treated solution having pH adjusted to 5.0 with 3M tris was obtained.

26.9 L of the virus inactivation-treated solution was concentrated to approximately 120 mg/mL with the UF membrane [manufactured by Merck millipore Corp.: Pellicon (registered trademark) 3, 0.11 m$^2$], the buffer exchange was performed to 10 mmol/L glutamic acid buffer (pH 5.2) containing 262 mmol/L D-sorbitol, and a concentration · buffer exchange solution was obtained. The concentration · buffer exchange solution was subjected to filter filtration (0.22 μm filter) and an activated carbon process purified product was obtained.

Comparative Example 2 Mab B Purification (process including protein A affinity chromatography)

The CHO cell culture broth containing the monoclonal antibodies (Mab B) which is the same as that in Example 15 was subjected to centrifugation to remove the cell, and a clarified solution was obtained with the depth membrane [manufactured by Merck millipore Corp.: Millistak (registered trademark) POD filter] and the microfiltration membrane.

The clarified solution was added to the protein A affinity chromatography column [manufactured by GE Healthcare Ltd., Inc.: MabSelect (registered trademark) SuRe] that equilibrated with an equilibration buffer consisting of 10 mmol/L Tris buffer (pH 7.0) in advance. After completion of the addition, the column was washed with 10 mmol/L tris buffer (pH 7.0) containing 5 column volumes of 1 mol/L sodium chloride and equilibration buffer.

After the washing, the column-eluted fraction that was eluted by 5 column volumes of 100 mmol/L glycine buffer (pH 3.2) was pooled as a MabSelect SuRe eluate. 0.1 mol/L hydrochloric acid was added to the MabSelect SuRe eluate to adjust the pH to 3.5, and maintained for 1 hour to perform virus inactivation. After the virus inactivation, the pH was neutralized to 7.0 with 0.5 M tris solution, and a virus inactivation-treated solution was obtained through a depth filter [manufactured by Merck millipore Corp.: Millistak (registered trademark) POD filter].

The virus inactivation-treated solution was added to an anion exchange chromatography column (manufactured by GE Healthcare Ltd., Inc., Q Sepharose XL, Sepharose is registered trademark) that was equilibrated with an equilibration buffer consisting of 10 mmol/L Tris buffer (pH 7.0), in advance. After completion of the addition, 5 column volumes of the equilibration buffer were passed through the column, the column non-adsorbed fraction was pooled, and then, a Q Sepharose eluate having the pH adjusted to 5.0 with 1 mol/L citric acid was obtained.

The Q Sepharose eluate was added to a cation exchange chromatography column (manufactured by Merck millipore Corp.: Fractogel (trademark) EMD SE Hicap) that was equilibrated with an equilibration buffer consisting of 20 mmol/L sodium citrate buffer (pH 5.0) containing 50 mmol/L sodium chloride in advance.

After completion of the addition, 5 column volumes of the equilibration buffer were passed through the column. Then, a Fractogel recovered solution was obtained by elution buffer consisting of 20 mmol/L sodium citrate buffer (pH 5.0) containing 0.3 mol/L sodium chloride.

The Fractogel recovered solution was passed through the virus-free membrane [manufactured by Asahi Kasei Medical Co., Ltd.: Planova (registered trademark) 20N, 0.01 m$^2$] that was equilibrated with the 20 mmol/L sodium citrate buffer (pH 5.0) containing 50 mmol/L sodium chloride in advance, and a virus filtration membrane-treated solution was obtained.

The virus filtration membrane-treated solution was concentrated to approximately 120 mg/mL with the UF membrane [manufactured by Merck millipore Corp.: Pellicon (registered trademark) 3], the buffer exchange was performed to 10 mmol/L glutamic acid buffer (pH 5.2) containing 262 mmol/L D-sorbitol, and a concentration · buffer exchange solution was obtained. The concentration · buffer exchange solution was subjected to filter filtration (0.22 μm filter) and a protein A process purified product was obtained.

The comparison of the process including the protein A affinity chromatography and the activated carbon process is shown below.

FIG. 53 shows a recovery rate of each process of the purification analyzed with the concentration quantitation by protein A affinity HPLC. As shown in FIG. 53, in the protein A affinity chromatography and the activated carbon process, the recovery rate at the same level was obtained.

FIG. 54 shows a result of the content of polymer of the purification intermediate and the final purified product analyzed by the gel filtration HPLC, and FIG. 55 shows the result of the content of degradation product, respectively. As shown in FIGS. 54 and 55, it was confirmed that the impurities together with the polymer and the degradation products were removed in the activated carbon process, to the same level as in the protein A process.

FIG. 56 shows a result obtained by analyzing the concentration of host cell protein by the ELISA method. As shown in FIG. 56, it was clear that, the concentration of the host cell protein after the activated carbon treatment was decreased to the concentration which is 1/400, compared to that after the protein A chromatography.

FIG. 57 shows a result obtained by the DNA concentration of the purification intermediate and the final purified product by the Threshold method. As shown in FIG. 57, it was clear that, the DNA concentration after the activated carbon treatment was decreased to the concentration to be less than 1/1000, compared to that after the protein A chromatography.

FIGS. 58 and 59 show results of a pre-peak content and a post-peak content of the purification intermediate and the final purified product analyzed by cation exchange HPLC.

As shown in FIG. 59, it was confirmed that, the post-peak content was decreased by the activatted carbon treatment, compared to that of protein A process, and a more homogeneous antibody composition was obtained.

From the results described above, even in the process of preparing the pretreatment solution by the concentration dilution and circulating the solution through the activated carbon membrane, compared to the protein A process of the related art, all of impurities such as the polymers, degradation products, the host cell proteins, the DNAs, and the post-peak were effectively removed.

As a result, in the protein A process of the related art for removing these impurities to a drug product level, both ion exchange chromatography of the anion exchange and the cation exchange was necessary, but it was confirmed that, in the process of circulating the solution through the activated carbon membrane, it is possible to remove the impurities to the drug product level only by the anion exchange membrane without using a column, and to achieve the high recovery rate having the same level to the protein A process of the related art.

Example 16 Mab B Purification [activated carbon process (1)]

400 L of CHO cell culture broth containing monoclonal antibodies (Mab B) was adjusted to pH 4.5 with acid or alkali. After leaving the pH-adjusted CHO cell culture broth for 1 hour, the cell was removed using a cell separation depth membrane [manufactured by Merck millipore Corp.: Clarisolve (registered trademark)], and the pH-adjusted clarified solution was obtained with an MF membrane [manufactured by Pall Corp.: Fluorodyne (registered trademark)].

The pH-adjusted clarified solution was concentrated to 16 L with a UF membrane [manufactured by Merck millipore Corp.: Pellicon (registered trademark) 3], and the concentration dilution of performing the dilution by adding 16 L of pure water was repeatedly performed three times. Further, after performing the same concentration, pure water was added, and a concentrated dilute diluted to 40 L was prepared.

38 L of the concentrated dilute was repeatedly subjected to a circulation process of passing through an activated carbon membrane of 11.4 m$^2$ [manufactured by Pall Corp.: Stax (trademark) AKS1] and then passing through the SHC membrane (manufactured by Merck millipore Corp.) overnight. After recovery of the circulated solution obtained through this circulation process, 26.4 L of pure water was passed through the activated carbon membrane and the SHC membrane, to recover a washing solution 1. A washing solution 2 was recovered by passing the pure water of the same volume through the activated carbon membrane and the SHC membrane again, the washing solution 1, the washing solution 2, and the circulated solution were combined and mixed with each other, and the activated carbon-treated solution was obtained.

3 mol/L tris was added to 82.6 L of the activated carbon-treated solution to adjust the pH to 7.0 and passed through the anion exchange membrane [manufactured by Asahi Kasei Medical Co.: Qyu Speed (trademark) D, 550 mL] that was equilibrated with an equilibration buffer consisting of 10 mmol/L Tris buffer (pH 7.0) in advance. Then, the equilibration buffer was passed and the non-adsorbed fraction was recovered as a QSD-treated solution.

50% (W/W) acetic acid was added to the QSD-treated solution to adjust the pH to 3.5, and held for 1 hour to perform virus inactivation. After the virus activation, a virus inactivation-treated solution having pH adjusted to 5.0 with 3M tris was obtained.

97 L of the virus inactivation-treated solution was passed through the virus-free membrane [manufactured by Asahi Kasei Medical Co., Ltd.: Planova (registered trademark) 20N, 1.00 m$^2$]. Then, 2L of pure water was passed through to obtain a virus-free membrane-treated solution. The virus-free membrane-treated solution was concentrated to approximately 82 mg/mL with the UF membrane [manufactured by Merck millipore Corp.: Pellicon (registered trademark) 3, 4.56 m$^2$], the buffer exchange was performed to 10 mmol/L glutamic acid buffer (pH 5.2) containing 262 mmol/L D-sorbitol, and a concentration · buffer exchange solution was obtained by passing through the SHC membrane (manufactured by Merck millipore Corp.).

The 10 mmol/L glutamic acid buffer (pH 5.2) containing 2.55 g/L of polysorbate 80 and 262 mmol/L of D-sorbitol was added to the concentration · buffer exchange solution so that the total concentration of polysorbate 80 becomes 0.05%, and filtered with a filter (0.22 μm filter), and an activated carbon process purified product (1) was obtained.

Example 17 Mab B Purification [Activated Carbon Process (2)]

3M tris solution was added to 1 L of the activated carbon-treated solution obtained in Example 16 to adjust the pH to 7.0, and passed through the anion exchange resin [manufactured by TOSOH Corp.: TOYOPEARL (registered trademark) NH$_2$-750F, 26 mm ID × 20 cm] that was equilibrated with an equilibration buffer consisting of 100 mmol/L Tris buffer (pH 7.0) in advance. Then, the equilibration buffer was passed through and the column non-adsorbed fraction was recovered as a NH$_2$ Toyopearl-treated solution.

50% (W/W) acetic acid was added to the NH$_2$ Toyopearl-treated solution to adjust the pH to 3.5, and held for 1 hour to perform virus inactivation. After the virus activation, a virus inactivation-treated solution having pH adjusted to 5.0 with 3M tris solution was obtained.

The virus inactivation-treated solution was passed through the virus-free membrane [manufactured by Asahi Kasei Medical Co., Ltd.: Planova (registered trademark) 20N, 0.01 m$^2$]. Then, 100 mL of milli-Q water was passed through to obtain a virus-free membrane-treated solution. The virus-free membrane-treated solution was concentrated to approximately 82 mg/mL with the UF membrane [manufactured by Merck millipore Corp.: Pellicon (registered trademark) 3, 0.11 m$^2$], the buffer exchange was performed to 10 mmol/L glutamic acid buffer (pH 5.2) containing 262 mmol/L D-sorbitol, and a concentration · buffer exchange solution was obtained.

The 10 mmol/L glutamic acid buffer (pH 5.2) containing 2.55 g/L of polysorbate 80 and 262 mmol/L of D-sorbitol was added to the concentration · buffer exchange solution so that the total concentration of polysorbate 80 becomes 0.05%, and filtered with a filter (0.22 $\mu$m filter), and an activated carbon process purified product (2) was obtained.

Example 18 Mab B Purification [Activated Carbon Process (3)]

2.5 L of the virus inactivation-treated solution obtained in Example 16 was added to a cation exchange column [manufactured by Merck millipore Corp.: Eshmuno (registered trademark) CPX, 50 mm ID $\times$ 20 cm] that was equilibrated with an equilibration buffer consisting of 10 mmol/L sodium acetate buffer (pH 5.0) in advance. After completion of the addition, the column was washed with 5 column volumes of the equilibration buffer.

Then, a CPX eluate was obtained by linear salt concentration gradient (10-column volumes) using the equilibration buffer and the 10 mmol/L sodium acetate buffer (pH 5.0) containing 0.3 mol/L sodium chloride.

The CPX eluate was passed through the virus-free membrane [manufactured by Asahi Kasei Medical Co., Ltd.: Planova (trademark) 20N, 0.01 m²]. Then, 100 mL of the milli-Q water was passed through and a virus filtration membrane-treated solution was obtained. The virus filtration membrane-treated solution was concentrated to approximately 82 mg/mL with the UF membrane [manufactured by Merck millipore Corp.: Pellicon (registered trademark) 3, 0.11 m²], the buffer exchange was performed to 10 mmol/L glutamic acid buffer (pH 5.2) containing 262 mmol/L D-sorbitol, and a concentration · buffer exchange solution was obtained.

The 10 mmol/L glutamic acid buffer (pH 5.2) containing 2.55 g/L of polysorbate 80 and 262 mmol/L of D-sorbitol was added to the concentration · buffer exchange solution so that the total concentration of polysorbate 80 becomes 0.05%, and filtered with a filter (0.22 $\mu$m filter), and an activated carbon process purified product (3) was obtained.

The comparison of the activated carbon processes (1) to (3) described in Examples 16 to 18 is shown below.

FIG. 60 shows a recovery rate of each purification process analyzed with the concentration quantitation by protein A affinity HPLC. FIG. 61 shows a result of the content of polymer of the purification intermediate and the final purified product analyzed by the gel filtration HPLC, and FIG. 62 shows the result of the content of degradation product, respectively. As shown in FIGS. 61 and 62, it was confirmed that the impurities together with the polymer and the degradation products were removed to approximately the same level, in the activated carbon processes (1) to (3).

FIG. 63 shows a result obtained by the concentration of the host cell protein of the purification intermediate and the final purified product by the ELISA method. As shown in FIG. 63, it was confirmed that the host cell protein was removed to approximately the same level, in the activated carbon processes (1) to (3).

FIG. 64 shows a result obtained by the DNA concentration of the purification intermediate and the final purified product by the Threshold method. As shown in FIG. 64, it was clear that the DNA was removed to approximately the same level, in the activated carbon processes (1) to (3).

Example 19 Examination of adsorption of high sugar chain-attached protein and PEGylated protein to activated carbon

The examination of the activated carbon adsorption was performed using erythropoietin (EPO) which is protein having high content of sugar chain and modified erythropoietin (modified EPO) obtained by increasing the number of added sugar chains.

2.5 mL of the protein solution prepared by weighing 10 mg of TOKUSEI SHIRASAGI (manufactured by Japan EnviroChemicals, Ltd., SHIRASAGI is registered trademark) activated carbon and set to have concentration of 2.0 mg/mL in advance, and 2.5 mL of each buffer prepared the pH of 4.0 to 9.0 by 1.0 were added to 15 mL tube, and stirred at room temperature for 17 hours. At this time, the amount of protein added per 1 mg of activated carbon is 0.5 mg.

Then, the activated carbon was removed by centrifugation separation (2,900 g, 10 minutes) and then membrane filter filtration (0.22 $\mu$m filter), and the concentration of protein remaining was measured by absorptiometry. In the same procedure described above, the examination was performed regarding SHIRASAGI P and SHIRASAGI DO-5 (which are manufactured by Japan EnviroChemicals, Ltd., SHIRASAGI is registered trademark).

FIGS. 65 to 67 show a relationship between the pH and the adsorption rate of the buffer used for the activated carbon treatment. As a result, it was determined that, the pH of the EPO and the modified EPO having a high content of sugar chain was 4 to 9 and the adsorption rate was low, but the pH of the host cell-derived protein was 4 to 6 and the adsorption rate was high, and accordingly, these can be used for purification using the activated carbon in a non-adsorption mode.

In addition, the examination of the adsorption of the activated carbon was performed using unmodified G-CSF, PEGylated G-CSF which is PEGylated protein thereof, and PEGylated TPO. 2.5 mL of the protein solution prepared by weighing 10 mg of TOKUSEI SHIRASAGI (manufactured by Japan EnviroChemicals, Ltd., SHIRASAGI is registered trademark) activated carbon and set to have concentration of 2.0 mg/mL in advance, and 2.5 mL of each buffer prepared the pH of 4.0 to 9.0 by 1.0 were added to 15 mL tube, and stirred at room temperature for 17 hours. At this time, the amount of protein added per 1 mg of activated carbon is 0.5 mg.

Then, the activated carbon was removed by centrifugation separation (2,900 g, 10 minutes) and then membrane filter filtration (0.22 µm filter), and the concentration of protein remaining was measured by absorptiometry. In the same procedure described above, the examination was performed regarding SHIRASAGI P and SHIRASAGI DO-5 (which are manufactured by Japan EnviroChemicals, Ltd., SHIRASAGI is registered trademark). FIG. 66 shows a relationship between the pH and the adsorption rate of the buffer used for the activated carbon treatment.

As a result, as shown in FIG. 66, it was clear that, in a case of the comparison of the unmodified G-CSF and PEGylated G-CSF, the maximum adsorption rate was significantly decreased due to the PEGylation. In addition, it was determined that, the adsorption rate of PEGylated TPO that is subjected to PEGylation was also low, in the same manner, and accordingly, this can be used for purification using the activated carbon in a non-adsorption mode.

Example 20 Examination of Adsorption of Virus to Activated Carbon

The examination for the removal of retrovirus due to the activated carbon membrane using three types of antibody solutions (Mab B, Mab A, and Mab E) was performed.

Regarding the Mab B, Mab A, and Mab E, the antibody concentration was adjusted to 30 to 60 mg/mL, the pH was adjusted to 4.0 to 5.0, and the conductivity was 0 to 2 mS/cm. 5 vol% of murine leukemia virus (MuLV) which is a model virus of the retrovirus was added to the respective antibody solutions, and 25 mL of each virus added solution was passed to an activated carbon membrane (manufactured by Pall Corp.: AKS1 filter) of 22 $m^2$ at a flow rate of 1.5 mL/mL.

At this time, the treatment was performed under two conditions of the condition for passing the film once and recovering (one-time solution passage) and the condition for performing circulation solution passage and recovering (circulation solution passage). In addition, regarding the circulation solution passage, the recovering was performed after 4, 8, and 23 hours. At each time, the amount of virus included in the recovered solution was estimated by quantitative Polymerase Chain Reaction (qPCR). The results are shown in Table 2.

[Table 2]

| Table 2 MuLV removal rate (LRF) due to activated carbon membrane | | | | |
|---|---|---|---|---|
| Antibody type | | Mab B | Mab A | Mab E |
| One-time solution passage | Hold control | 0.00±0.70 | 0.10±0.07 | 0.06±0.34 |
| | Recovered solution | ≥ 3.86±0.44 | ≥ 3.64±0.07 | ≥ 3.55±0.32 |
| Circulation solution passage | Hold control | 0.59±0.39 | 0.41±0.25 | 0.15±0.33 |
| | Recovered solution after 4 hours | ≥ 4.08±0.35 | ≥ 3.88±0.23 | ≥ 3.70±0.11 |
| | Recovered solution after 8 hours | ≥ 4.08±0.35 | ≥ 3.88±0.23 | ≥ 3.70±0.11 |
| | Recovered solution after 23 hours | ≥ 4.08±0.35 | ≥ 3.88±0.23 | ≥ 3.70±0.11 |

As shown in Table 2, it was confirmed that MuLV was effectively removed by the activated carbon membrane-passed solution. In a case of comparing the one-time solution passage and the circulation solution passage, a significant difference was not observed between two. The Logarithm Reduction Factor (LRF) (3 to 4) obtained by the activated carbon membrane-passed solution was greater than the LRF (1 to 3) generally obtained by Protein A affinity chromatography.

In addition, the examination for the removal of parvovirus due to the activated carbon membrane using three types of antibody solutions (Mab B, Mab A, and Mab E) was performed.

Regarding the Mab B, Mab A, and Mab E, the antibody concentration was adjusted to 30 to 60 mg/mL, the pH was adjusted to 4.0 to 5.0, and the conductivity was 0 to 2 mS/cm. 5 vol% of mouse parvovirus Minute Virus of Mice (MVM) which is a model virus of the parvovirus was added to the respective antibody solutions, and 25 mL of each virus added solution was passed to an activated carbon membrane (manufactured by Pall Corp.: AKS1filter) of 22 $m^2$ at a flow rate of 1.5 mL/mL.

At this time, the treatment was performed under two conditions of the condition for passing the film once and recovering (one-time solution passage) and the condition for performing circulation solution passage and recovering (circulation solution passage). In addition, regarding the circulation solution passage, the recovering was performed after 4, 8, and 23 hours. At each time, the virus filter included in the recovered solution was estimated based on infectivity. The results are shown in Table 3.

[Table 3]

| Table 3 MVM removal rate (LRF) due to activated carbon membrane | | | | |
|---|---|---|---|---|
| Antibody type | | Mab B | Mab A | Mab E |
| One-time solution passage | Hold control | 0.31±0.36 | -0.31±0.29 | 0.06±0.33 |
| | Recovered solution | ≥ 3.18±0.41 | ≥ 5.08±0.41 | ≥ 5.85±0.25 |
| Circulation solution passage | Hold control | 0.56±0.42 | -0.06±0.36 | 0.25±0.32 |
| | Recovered solution after 4 hours | ≥ 5.45±0.92 | ≥ 5.14±0.91 | ≥ 4.94±0.27 |
| | Recovered solution after 8 hours | ≥ 5.00±0.30 | ≥ 4.69±0.27 | ≥ 5.07±0.68 |
| | Recovered solution after 23 hours | ≥ 5.64±0.46 | ≥ 5.66±0.27 | ≥ 6.07±0.67 |

As shown in Table 3, it was confirmed that the MVM was effectively removed by the activated carbon membrane-passed solution. In a case of comparing the one-time solution passage and the circulation solution passage, a significant difference was not observed between two. The Logarithm Reduction Factor (LRF) (3 to 6) obtained by the activated carbon membrane-passed solution was greater than the LRF (1 to 3) generally obtained by Protein A affinity chromatography.

Example 21 Effect of Removal of Impurities due to connection method of activated carbon membrane

The CHO cell culture broth containing monoclonal antibodies (Mab B) was adjusted to pH 4.5 with acid. After leaving the pH-adjusted CHO cell culture broth for 1 hour, the cell was removed using a cell separation depth membrane [manufactured by Merck millipore Corp.: Clarisolve (registered trademark)], and the pH-adjusted clarified solution was obtained with an SHC membrane (manufactured by Merck millipore Corp.).

The pH-adjusted clarified solution was concentrated with a UF membrane [manufactured by Merck millipore Corp.: Pellicon (registered trademark) 3], and diluted using the milli-Q water, and the conductivity of the concentrated dilute was 1 mS/cm or less. The antibody concentration of the concentrated dilute was adjusted to 15 mg/mL.

61.3 mL of the concentrated dilute was passed through four activated carbon membranes (manufactured by Pall Corp.: AKS1 filter) of 22 cm$^2$ connected in series once so that the load volume becomes 0.1 mg antibody/mg activated carbon, under the conditions of Table 4 for the contact time of 0.2 hours, and an activated carbon membrane-treated solution was obtained.

In addition, 61.3 mL of the concentrated dilute was passed through four activated carbon membranes (manufactured by Pall Corp.: AKS1 filter) of 22 cm$^2$ connected in parallel once so that the load volume becomes 0.1 mg antibody/mg activated carbon, under the conditions of Table 4 for the contact time of 0.2 hours, and an activated carbon membrane-treated solution was obtained. As a control, the solution not subjected to the activated carbon membrane treatment was used.

[Table 4]

| Table 4 Activated Carbon Membrane Treatment Conditions | | | | |
|---|---|---|---|---|
| Connection method | Membrane area (cm2) | Treatment solution amount (mL) | Solution passage flow rate (mL/min) | Load volume (mg antibody/mg activated carbon) |
| Four in series | 22 × 4 | 61.3 | 2.0 | 0.1 |
| Four in parallel | 22 × 4 | 61.3 (15.3/one membrane) | 2.0 (0.5/one membrane) | 0.1 |

FIG. 68 shows a recovery rate calculated by analyzing the antibody concentration of each of the obtained activated carbon membrane-treated solution and the control by protein A affinity HPLC. As shown in FIG. 68, a high recovery rate of approximately 80% was obtained, in both cases of the connection in series and in parallel.

FIG. 69 shows a result obtained by analyzing the content of polymer of each activated carbon membrane-treated solution by the gel filtration HPLC, and FIG. 70 shows the result obtained by analyzing the content of degradation product, respectively. As shown in FIG. 69, the content of polymer in the connection in series and in parallel was decreased from 0.7% to 0.6%. In addition, as shown in FIG. 70, the content of degradation product was further decreased in the connection in series (connection in series: 0.8%, connection in parallel: 1.3%).

FIG. 71 shows a result obtained by analyzing the content of the host cell protein per 1 mg of protein by the ELISA method. As shown in FIG. 71, the content of the host cell protein was more significantly decreased in the connection of activated carbon membranes in series, compared to the connection in parallel.

From the results described above, it was confirmed that, in the activated carbon membrane treatment, the impurities could be more effectively removed in the connection in series, compared to the connection in parallel.

**Claims**

1. A method for purifying a protein using an activated carbon, comprising:
   bringing an activated carbon pretreatment solution obtained by adjusting conductivity of a protein-containing aqueous solution into contact with an activated carbon; and

   separating the protein and impurities in a non-adsorption mode to obtain the protein of interest with a low content of impurities,
   wherein the conductivity of the activated carbon pretreatment solution is 0 to 2 mS/cm, and
   wherein the protein is an antibody and the concentration of the antibody is 5.5 to 23 mg/mL.

2. The purification method according to claim 1, wherein the conductivity of the activated carbon pretreatment solution is 0 to 1 mS/cm.

3. The purification method according to any one of claims 1 to 2, wherein the activated carbon pretreatment solution is obtained by adjusting the conductivity of the protein-containing aqueous solution by performing dilution, concentration dilution, or buffer exchange of the protein-containing aqueous solution.

4. The purification method according to any one of claims 1 to 3, wherein the activated carbon pretreatment solution is obtained by adjusting the conductivity of the protein-containing aqueous solution by adding sodium chloride to the protein-containing aqueous solution.

5. The purification method according to any one of claims 1 to 4, comprising:
   further adjusting load volume and a contact time for bringing the activated carbon pretreatment solution into contact with the activated carbon.

6. The purification method according to claim 5, wherein the load volume is 0.1 to 0.3 mg protein/mg activated carbon, and the contact time is 8 to 24 hours.

7. The purification method according to claim 5, wherein the load volume is 0.05 to 0.15 mg protein/mg activated carbon, and the contact time is 0.1 to 24 hours.

8. The purification method according to claim 5, wherein the load volume is 0.05 to 0.15 mg protein/mg activated carbon, and the contact time is 2 to 24 hours.

9. The purification method according to any one of claims 1 to 8, comprising:
   passing the activated carbon pretreatment solution through two or more membranes or cartridges including the activated carbon or columns filled with the activated carbon continuously.

10. The purification method according to any one of claims 1 to 9, comprising:
    circulating the activated carbon pretreatment solution through an activated carbon membrane or a column filled with the activated carbon to continuously contact with the activated carbon.

11. The purification method according to any one of claims 1 to 10, wherein the impurities are at least one selected from host cell proteins, protein-derived polymers, protein-derived degradation products, DNAs, and viruses.

12. A method for preparing a protein, comprising: the purification method according to any one of claims 1 to 11, wherein the protein is an antibody.

13. The preparation method according to claim 12, comprising the use of at least one selected from an anion exchange membrane, anion exchange chromatography, cation exchange chromatography, and multimodal chromatography.

**Patentansprüche**

1. Verfahren zur Aufreinigung eines Proteins unter Verwendung einer Aktivkohle ("activated carbon"), umfassend:

   das In-Kontakt-Bringen einer Aktivkohle-Vorbehandlungslösung ("activated carbon pretreatment solution"), erhalten durch Anpassen der Leitfähigkeit einer proteinhaltigen wässrigen Lösung, mit einer Aktivkohle; und
   Trennung des Proteins und Verunreinigungen in einem Nichtadsorptionsmodus ("non-adsorption mode"), um das Protein von Interesse mit einem geringen Gehalt an Verunreinigungen zu erhalten,
   wobei die Leitfähigkeit der Aktivkohle-Vorbehandlungslösung 0 bis 2 mS/cm beträgt, und
   wobei das Protein ein Antikörper ist und die Konzentration des Antikörpers 5,5 bis 23 mg/mL beträgt.

2. Aufreinigungsverfahren gemäß Anspruch 1, wobei die Leitfähigkeit der Aktivkohle-Vorbehandlungslösung 0 bis 1 mS/cm beträgt.

3. Aufreinigungsverfahren gemäß irgendeinem der Ansprüche 1 bis 2, wobei die Aktivkohle-Vorbehandlungslösung durch Anpassen der Leitfähigkeit der proteinhaltigen wässrigen Lösung erhalten wird, indem Verdünnung, Konzentrierungsverdünnung ("concentration dilution") oder Pufferaustausch der proteinhaltigen wässrigen Lösung durchgeführt wird.

4. Aufreinigungsverfahren gemäß irgendeinem der Ansprüche 1 bis 3, wobei die Aktivkohle-Vorbehandlungslösung durch Anpassen der Leitfähigkeit der proteinhaltigen wässrigen Lösung erhalten wird, indem Natriumchlorid zu der proteinhaltigen wässrigen Lösung hinzugefügt wird.

5. Aufreinigungsverfahren gemäß irgendeinem der Ansprüche 1 bis 4, umfassend:
   weiteres Anpassen des Beladungsvolumens ("loading volume") und einer Kontaktzeit, um die Aktivkohle-Vorbehandlungslösung mit der Aktivkohle in Kontakt zu bringen.

6. Aufreinigungsverfahren gemäß Anspruch 5, wobei das Beladungsvolumen 0,1 bis 0,3 mg Protein/mg Aktivkohle beträgt und die Kontaktzeit 8 bis 24 Stunden beträgt.

7. Aufreinigungsverfahren gemäß Anspruch 5, wobei das Beladungsvolumen 0,05 bis 0,15 mg Protein/mg Aktivkohle beträgt und die Kontaktzeit 0,1 bis 24 Stunden beträgt.

8. Aufreinigungsverfahren gemäß Anspruch 5, wobei das Beladungsvolumen 0,05 bis 0,15 mg Protein/mg Aktivkohle beträgt und die Kontaktzeit 2 bis 24 Stunden beträgt.

9. Aufreinigungsverfahren gemäß irgendeinem der Ansprüche 1 bis 8, umfassend:
   kontinuierliches Durchleiten der Aktivkohle-Vorbehandlungslösung durch zwei oder mehr Membrane oder durch Patronen, die die Aktivkohle enthalten, oder durch mit der Aktivkohle gefüllte Säulen.

10. Aufreinigungsverfahren gemäß irgendeinem der Ansprüche 1 bis 9, umfassend:
    Zirkulieren der Aktivkohle-Vorbehandlungslösung durch eine Aktivkohle-Membran oder durch eine mit der Aktivkohle gefüllte Säule, um kontinuierlich die Aktivkohle zu kontaktieren.

11. Aufreinigungsverfahren gemäß irgendeinem der Ansprüche 1 bis 10, wobei die Verunreinigungen mindestens eins ausgewählt von Wirtszellproteinen, von Proteinen stammenden Polymeren, von Proteinen stammenden Abbauprodukten, DNAs und Viren sind.

12. Verfahren zur Herstellung eines Proteins, umfassend: das Aufreinigungsverfahren gemäß irgendeinem der Ansprüche 1 bis 11, wobei das Protein ein Antikörper ist.

13. Herstellungsverfahren gemäß Anspruch 12, umfassend die Verwendung von mindestens einer ausgewählt von einer Anionenaustauschmembran, Anionenaustauschchromatographie, Kationenaustauschchromatographie und multimodaler Chromatographie.

**Revendications**

1. Procédé de purification d'une protéine utilisant un charbon actif, comprenant :

   la mise en contact d'une solution de prétraitement de charbon actif obtenue en ajustant la conductivité d'une solution aqueuse contenant une protéine avec un charbon actif ; et
   la séparation de la protéine et des impuretés dans un mode de non-adsorption pour obtenir la protéine d'intérêt avec une faible teneur en impuretés,
   dans lequel la conductivité de la solution de prétraitement de charbon actif est de 0 à 2 mS/cm, et
   dans lequel la protéine est un anticorps et la concentration de l'anticorps est de 5,5 à 23 mg/mL.

2. Procédé de purification selon la revendication 1, dans lequel la conductivité de la solution de prétraitement de charbon actif est de 0 à 1 mS/cm.

3. Procédé de purification selon l'une quelconque des revendications 1 à 2, dans lequel la solution de prétraitement de charbon actif est obtenue en ajustant la conductivité de la solution aqueuse contenant une protéine en effectuant une dilution, une dilution par concentration, ou un échange de tampon de la solution aqueuse contenant une protéine.

4. Procédé de purification selon l'une quelconque des revendications 1 à 3, dans lequel la solution de prétraitement de charbon actif est obtenue en ajustant la conductivité de la solution aqueuse contenant une protéine en ajoutant du chlorure de sodium à la solution aqueuse contenant une protéine.

5. Procédé de purification selon l'une quelconque des revendications 1 à 4, comprenant :
   l'ajustement en outre du volume de charge et d'un temps de contact pour mettre la solution de prétraitement de charbon actif en contact avec le charbon actif.

6. Procédé de purification selon la revendication 5, dans lequel le volume de charge est de 0,1 à 0,3 mg de protéine/mg de charbon actif, et le temps de contact est de 8 à 24 heures.

7. Procédé de purification selon la revendication 5, dans lequel le volume de charge est de 0,05 à 0,15 mg de protéine/mg de charbon actif, et le temps de contact est de 0,1 à 24 heures.

8. Procédé de purification selon la revendication 5, dans lequel le volume de charge est de 0,05 à 0,15 mg de protéine/mg de charbon actif, et le temps de contact est de 2 à 24 heures.

9. Procédé de purification selon l'une quelconque des revendications 1 à 8, comprenant :
   le fait de faire passer la solution de prétraitement de charbon actif à travers deux membranes ou cartouches ou plus incluant le charbon actif ou des colonnes remplies de charbon actif de façon continue.

10. Procédé de purification selon l'une quelconque des revendications 1 à 9, comprenant :
    le fait de faire circuler la solution de prétraitement de charbon actif à travers une membrane de charbon actif ou une colonne remplie de charbon actif pour un contact continu avec le charbon actif.

11. Procédé de purification selon l'une quelconque des revendications 1 à 10, dans lequel les impuretés sont au moins l'un choisi parmi des protéines de cellules hôtes, des polymères dérivés de protéines, des produits de dégradation dérivés de protéines, des ADN et des virus.

12. Procédé de préparation d'une protéine, comprenant : le procédé de purification selon l'une quelconque des revendications 1 à 11, dans lequel la protéine est un anticorps.

13. Procédé de préparation selon la revendication 12, comprenant l'utilisation d'au moins l'une choisie parmi une membrane échangeuse d'anions, une chromatographie échangeuse d'anions, une chromatographie échangeuse de cations, et une chromatographie multimodale.

## Fig. 1

## Fig. 2

Fig. 3

Fig. 4

Fig. 5

Fig. 6

Fig. 7

Fig. 8

Fig. 9

Fig. 10

## Fig. 11

## Fig. 12

Fig. 13

Fig. 14

Fig. 15

Fig. 16

Fig. 17

Fig. 18

## Fig. 19

## Fig. 20

Fig. 21

Fig. 22

Fig. 23

Fig. 24

Fig. 25

## Fig. 26

**Legend:**
- ■ 0.05mg mab/mg Activated Carbon
- ◆ 0.1mg mab/mg Activated Carbon
- ▲ 0.15mg mab/mg Activated Carbon
- ✕ 0.2mg mab/mg Activated Carbon
- ✳ 0.25mg mab/mg Activated Carbon
- ● 0.3mg mab/mg Activated Carbon
- ✛ 0.4mg mab/mg Activated Carbon
- ○ 0.8mg mab/mg Activated Carbon
- — 1.6mg mab/mg Activated Carbon
- ◇ 3.2mg mab/mg Activated Carbon

Before Activated Carbon Treatment: 12.66
Beyond Range after Activated Carbon Treatment: 0.8:6.12, 1.6:10.60, 3.2:11.72

## Fig. 27

Fig. 28

Fig. 29

Fig. 30

Fig. 31

Fig. 32

Fig. 33

Fig. 34

Fig. 35

Fig. 36

Fig. 37

Fig. 38

Fig. 39

Fig. 40

## Fig. 41

Fig. 42

Fig. 43

Fig. 44

Fig. 45

Fig. 46

Fig. 47

Legend:
- ■ Protein A Process
- □ Activated Carbon Process (1)
- ▨ Activated Carbon Process (2)

X-axis: HMWS (%)

Categories:
- Culture Broth
- Cell Separation
- Concentration/Dilution
- Protein A/Activated Carbon
- Virus Inactivation
- Anion Exchange
- Cation Exchange
- Concentration/Buffer Exchange
- Purified Product

Fig. 48

Fig. 49

Log$_{10}$[HCP(pg/mg-P)]

Protein A Process

Activated Carbon Process (1)

Activated Carbon Process (2)

Lower Than
※ Limit of Quantitation

Culture Broth

Cell Separation

Concentration/Dilution

Protein A/Activated Carbon

Virus Inactivation

Anion Exchange

Cation Exchange

Purified Product

EP 3 508 493 B1

Fig. 50

Fig. 51

Fig. 52

Fig. 53

Fig. 54

Fig. 55

Fig. 56

Fig. 57

Fig. 58

Fig. 59

Fig. 60

Activated Carbon Process (1)
Activated Carbon Process (2)
Activated Carbon Process (3)

Acid Precipitation
Cell Separation to Concentration/Dilution
Activated Carbon
Anion Exchange
Virus Inactivation
Cation Exchange
Virus-Removal Membrane
Concentration/Buffer Exchange
Purified Product

Yield (%)

0    50    100    150

Fig. 61

Fig. 62

Fig. 63

Fig. 64

Fig. 65

EPO (30000, pI 4.2~4.7)

Modified EPO (36000, pI 4.0~5.0)

Fig. 66

Fig. 67

Fig. 68

## Fig. 69

HMWs (%)

## Fig. 70

LMWs (%)

## Fig. 71

HCP(ng/mg-P)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2639239 A2
- JP HEI5504579 A
- WO 2014024514 A
- US 5725856 A
- WO 9630046 A
- US 5736137 A
- WO 9640201 A
- WO 9851793 A
- WO 2010008075 A
- WO 2008120801 A
- JP HEI3198792 A
- WO 2010018847 A
- WO 2007062245 A
- WO 2007114496 A

**Non-patent literature cited in the description**

- *Anticancer Res.,* 1993, vol. 13, 331
- *Cancer Immunol. Immunother.,* 1993, vol. 36, 260
- *Cancer Res.,* 1994, vol. 54, 1511
- *Proc. Natl. Acad. Sci. USA,* 1992, vol. 89, 4285
- *British J.Cancer,* 2000, vol. 83, 493
- *Molecular Immunol.,* 1999, vol. 36, 387
- *Cancer,* 2000, vol. 88, 2909
- *Proc. Natl. Acad. Sci. USA,* 1989, vol. 86, 9911
- *J. Biol. Chem.,* 1990, vol. 265, 16455
- *J. Neurosci. Res.,* 1995, vol. 40, 647
- *J. Urology,* 1998, vol. 160, 2396
- *Cancer Res.,* 1997, vol. 57, 4593
- *Oncogene,* 2000, vol. 19, 2138
- *Curr. Opin. Oncol.,* 1998, vol. 10, 548
- *Cancer Res.,* 1995, vol. 55 (23), 5935s-5945s
- *Immunol.Rev.,* 1992, vol. 127, 5
- *Molecular Immunol.,* 1994, vol. 31, 371
- *Cytokine,* 1991, vol. 3, 562
- *J.Immunol.Methods,* 1998, vol. 217, 41
- *Lymph.Cyto.Res.,* 1994, vol. 13, 183
- *Molecular Pharmacol.,* 2000, vol. 58, 237
- *Nature,* 1999, vol. 400, 776
- **PERI et al.** *J.Immunol.Meth.,* 1994, vol. 174, 249
- *J.Exp.Med.,* 1997, vol. 186, 1373
- *J. Immunol.,* 1994, vol. 152, 2968
- *Science,* 1991, vol. 253, 1129
- *J. BioL Chem.,* 1997, vol. 272, 17400
- *Circulation,* 2000, vol. 101, 1158
- *Structure,* 2000, vol. 8, 385
- *J. Rheumatology,* 1998, vol. 25, 2065
- *J. Clin. Microbiol.,* 1999, vol. 37, 396
- *The Journal of the American Medical Association,* 1967, vol. 199, 519
- *Science,* 1952, vol. 122, 501
- *Virology,* 1959, vol. 8, 396
- *Proceeding of the Society for the Biological Medicine,* 1950, vol. 73, 1
- *Proc. Natl. Acad. Sci. USA,* 1965, vol. 53, 288
- *J.Experimental Medicine,* 1978, vol. 147, 923